# EUROPEAN PATENT SPECIFICATION

(11) **EP 1 608 623 B1**
(45) Date of publication and mention of the grant of the patent: **19.01.2011**
(21) Application number: 04708284.7
(22) Date of filing: 05.02.2004
(51) Int. Cl.: A61P 35/00, A61P 29/00, A61P 9/00, A61K 31/4412, A61K 31/4427, C07D 213/53, C07D 409/14

(54) **METAL ION CHELATORS AND THERAPEUTIC USE THEREOF**
METALLIONENCHELATOREN UND IHRE THERAPEUTISCHE VERWENDUNG
CHELATEURS D'IONS METALLIQUES ET LEUR UTILISATION THERAPEUTIQUE

(30) Priority: 05.02.2003 AU 2003900495; 11.02.2003 AU 2003900922
(43) Date of publication of application: 28.12.2005
(73) Proprietor: Richardson, Desi Raymond, West Hoxton NSW 2171 (AU); Lovejoy, David Benn, Newtown NSW 2042 (AU)
(72) Inventor: RICHARDSON, Desi Raymond, West Hoxton, NSW 2171 (AU); LOVEJOY, David, Benn, Newtown, NSW 2042 (AU)
(74) Representative: Bridle, Andrew Barry
(86) International application number: PCT/AU2004/000132
(87) International publication number: WO 2004/069801

(56) References cited:
- EP-A1- 0 571 857
- DE-A- 4 207 401
- DE-A1- 4 207 400
- JP-A- 11 209 352
- F. CASE: "preparation and chromogenic properties of semicarbazones containing the ferroin group." J. CHEM. ENG. DATA, vol. 25, no. 4, 1980, pages 404-405, XP002436212 us
- HALL I H ET AL: "CYTOTOXICITY OF 2-ALDO- AND 2-KETOPYRIDINE-N(4)-SUBSTITUTED THIOSEMICARBAZONES AND MODE OF ACTION IN HUMAN TMOLT4 CELLS" PHARMAZIE, DIE, GOVI VERLAG, ESCHBORN, DE, vol. 56, no. 8, 2001, pages 648-653, XP001071296 ISSN: 0031-7144
- A. BACCHI ET AL.: "versatile ligand behaviour of phenyl 2-pyridyl ketone benzoylhydrazone in palladium(II) complexes." DALTON TRANSACTIONS., vol. 22, 1996, pages 4239-4244, XP002436213 GBRSC PUBLISHING, CAMBRIDGE.
- P. PELAGATTI ET AL.: "chemoselective homogenous hydrogenation of phenylacetylene using thiosemicarbazone and thiobenzoylhydrazone palladium(II) complexes" DALTON TRANSACTIONS., vol. 16, 1998, pages 2715-2722, XP002436214 GBRSC PUBLISHING, CAMBRIDGE.
- A.M. GARCIA RODRIGUEZ ET AL.: "simultaneous determination of iron,cobalt,nickel and copper by UV-visible spectrophotometry with multivariate calibration." TALANTA, vol. 47, no. 2, 1998, pages 463-470, XP002436215 engl
- M. CACELLI ET AL.: "synthetic routes to mono-and bicopper hydrazone complexes" INORGANIC CHIMICA ACTA, vol. 303, no. 2, 2000, pages 238-243, XP002436216 engl
- A. BACCHI ET AL.: "a new polydentate hydrazonic ligand and its dinuclear copper(II) complex with different coordination environments." DALTON TRANSACTIONS., vol. 5, 1993, pages 775-779, XP002436217 GBRSC PUBLISHING, CAMBRIDGE.
- E. BECKER ET AL.: "identification of the di-pyridyl ketone isonicotinoyl hydrazone (PKIH) analogues as potent iron chelators and anti-tumors agents." BRITISH JOURNAL OF PHARMACOLOGY, vol. 138, no. 5, March 2003 (2003-03), pages 819-830, XP002436218 engl
- LIBERTA A. AND WEST D.X.: 'Antifungal and antitumor activity of hetero cyclic thiosemicabazones and their metal complexes: current status.' BIOMETALS vol. 5, 1992, pages 121 - 126
- MILLER III M.C ET AL.: 'The cytotoxicity of copper (II) complexes of 2-acetyl-pyridyl-N-sustituded Thiosemicarbazones.' ANTICANCER RES. vol. 18, 1998, pages 4131 - 4140
- FRENCH F.A. AND BLANZ E.J.: 'The carcinostatic activity of thiosemicarbazones of formyl heteroaromatic compounds. III. Primary correlation.' J. MED. CHEM. vol. 9, 1966, F, pages 585 - 589

## Description

### Field of the invention

The present invention relates to compounds which are capable of chelating metal ions. In particular, the present invention relates to (thio)semicarbazone compounds and (thio)hydrazone compounds which are capable of chelating metal ions, including iron ions. The invention also relates to therapeutic use of such compounds and/or their metal ion complexes, in treating diseases associated with cell proliferation.

### Background of the invention

Iron (Fe) is fundamentally involved in many important cellular processes. For example, Fe-containing proteins catalyse key reactions involved in energy metabolism, respiration, and DNA synthesis. Cellular Fe-deprivation results in G₁/S arrest and apoptosis.^{1,2} Tumour cells are far more sensitive than norman cells to iron depletion possibly due to their increased rates of proliferation. The increased iron requirements of tumour cells is reflected by the increased expression of the Transferrin Receptor 1 which binds the serum Fe-transport protein, transferrin (Tf). Neoplastic cells also express higher levels of the Fe-containing enzyme, ribonucleotide reductase, which is the critical rate-limiting step of DNA synthesis. Cellular iron pools therefore represent an important therapeutic target and compounds which are capable of chelating iron may be useful therapeutic agents in the treatment of iron-associated diseases and disorders, as well as potential anti-tumour agents.

Desferrioxamine (DFO) is an iron chelator currently used for the treatment of iron overload disease. However, the use of DFO as an anti-cancer agent is limited by its modest anti-proliferative activity.

There is a need for therapeutically acceptable metal ion chelators, including iron chelators, which are capable of permeating cell membranes and chelating intracellular Fe. There is also a need for clinically useful iron chelators having anti-proliferative activity.

### Summary of the Invention

According to a first aspect of the invention there is provided a compound of Formula 3: 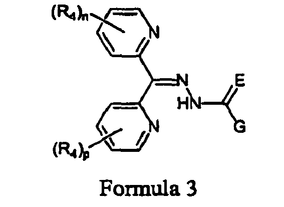 wherein
E is O or S;
n is 0, 1, 2, 3 or 4,
p is 0, 1, 2, 3 or 4, and
each R₄ is independently selected from halogen, alkyl, alkenyl, optionally substituted amino, hydroxyl, -O-alkyl, -S-alkyl, -C(O)-alkyl, -C(O)-alkenyl, -C(O)-O-alkyl, nitro and cyano, and
-G is NR₂R₃ or CR₂R₃R₅.
wherein the bond between C and R₅ may be a single bond, double bond, or triple bond; and wherein when the bond between C and R₅ is a double bond, one of R₂ and R₃ is absent, and wherein when the bond between C and R₅ is a triple bond, R₂ and R₃ are both absent;
R₂ and R₃ may be the same or different and are individually selected from hydrogen, halogen, optionally substituted alkyl, optionally substituted alkenyl, optionally substituted alkynyl, optionally substituted amino, optionally substituted heteroaromatic, an optionally substituted bicyclic group, and an optionally substituted aromatic group; and
R₅ is selected from the group consisting of hydrogen, halogen, optionally substituted alkyl, optionally substituted alkenyl, optionally substituted alkynyl, optionally substituted amino, optionally substituted heteroaromatic, an optionally substituted bicyclic group, and an optionally substituted aromatic group;
or -G is an optionally substituted heteroaromatic group, an optionally substituted bicyclic group, an optionally substituted aromatic group, an optionally substituted alkyl group; an optionally substituted cycloalkyl group, or an optionally substituted heterocycloalkyl group;
with the provisos that:
   (i) when E is O, n is 0 and p is 0, then
      -G is not -NH_{2:} -CH₂Cl; 2-furanyl; 2-thiophenyl; phenyl; 2-halophenyl, 3-halophenyl or 4halophenyl, where halo is F, Cl, Br or I; 2-aminophenyl; 4-aminophenyl; 2-hydroxyphenyl; 4-hydroxyphenyl; 2-pyridyl; 4-pyrldyl; 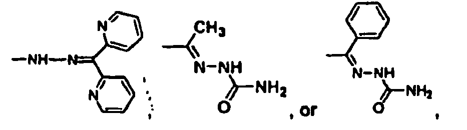 and
   (ii) when E is S, n is 0 and p is 0, then
      -G is not -NH₂; -NHCH₃; -NHPhenyl; N(benzyl)₂; -NH(2-pyidyl); N(methyl)₂; N(ethyl)₂; N(propyl)_{2;} N(butyl)₂; N(pentyl)₂; N(hexyl)₂; -N-pyrrolidinyl; -N-morpholinyl;
      2-thiophenyl; 2-furanyl; 2-hydroxyphenyl; 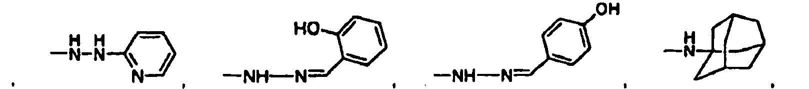 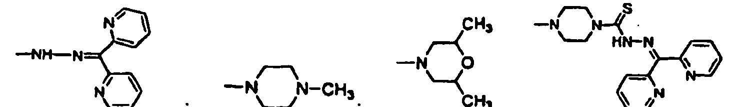 or 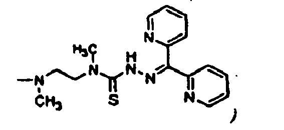 and
   (iii) when E is S, n is 0 and p is I, then
      a) when R₄ is 6-Br, G is not N(methyl)₂;
      b) when R₄ is 6-Me, G is not N(methyl)₂; and
      c) when R₄ is 6-F, G is not N-pyrrolidinyl.

According to a second aspect of the invention there is provided a compound of Formula 4: 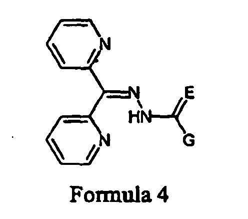 wherein
E is O or S; and
-G is as defined above for Formula 3 including provisos (i) and (ii).

In one embodiment of the invention, when E is O, the compound of Formula 4 is di-2-pyridylketone 4,4-diphenylsemicarbazone (abbreviated PK44pH). In another embodiment, when E is O the compound of Formula 4 is di-2-pyridylketone octanoic hydrazone (abbreviated PKoctH).

In a further embodiment of the invention, when E is S, the compound of Formula 4 is di-2-pyridylketone 4-ethyl-3-thiosemicarbazone (abbreviated Dp4eT). In another embodiment, when E is S the compound of Formula 4 is di-2-pyridylketone 4-allyl-3-thiosemicarbazone (abbreviated Dp4aT).

Compounds of Formula 3 or 4 may be capable of chelating metal ions. Accordingly, in another aspect of the invention there is provided a metal ion complex of a compound of Formula 3 or 4 as defined in any one of the first to fourth aspects of the invention, including or excluding provisos (i) and (ii).

In one embodiment, the metal ion complex of a compound of Formula 3 or 4 may be a transition metal ion complex, such as copper, iron, zinc, palladium, platinum, or gallium ion complex. In one embodiment, the metal ion is iron(II). In another embodiment, the metal ion is iron(III).

In one embodiment, compounds of Formula 3 or 4 may function as tridentate ligands. In one embodiment, two compounds of Formula 3 or 4 may form a hexacoordinate complex with a metal ion, for example, iron(II) or iron(III).

According to a third aspect of the invention there is provided a pharmaceutical composition comprising a pharmaceutically acceptable diluent, adjuvant or carrier together with at least one compound of Formula 3 or 4 as defined herein with or without provisos (i) and (ii).

In one embodiment, the pharmaceutical composition of the invention comprises at least one compound of Formula 3 or 4 according to any one of the first to fourth aspects of the invention, including provisos (i) and (ii).

In some embodiments of the invention, the pharmaceutical composition may comprise one or more metal ion complex of a compound of Formula 3 or 4. Suitable metal ions include transition metal ions. For example, the metal ion may be iron(II), iron(III), copper(II), zinc(II), palladium(II), platinum(II), or gallium(III).

Compounds of Formulae 3 and 4 as defined herein with or without provisos (i) and (ii), and compositions thereof, may be used for *in vitro* or *in vivo* metal ion chelation. In one embodiment, compounds of Formulae 3 and 4 as defined herein with or without provisos (i) and (ii), and compositions thereof, may be used for iron chelation. Accordingly, compounds of Formulae 3 and 4 as defined herein with or without provisos (i) and (ii), and compositions thereof, may be suitable for iron chelation therapy or treating iron-overload disorders. Compounds of Formulae 3 and 4 as defined herein with or without provisos (i) and (ii), and compositions thereof, may also be used for modifying cellular function by chelating cellular metal ions, including iron. For example, compounds of Formulae 3 and 4 as defined herein with or without provisos (i) and (ii), and compositions thereof, may be useful for inhibiting cellular proliferation and/or inducing apoptosis. Compounds of Formulae 3 and 4 as defined herein with or without provisos (i) and (ii), and compositions thereof may also be useful for modifying expression of genes which are sensitive to iron concentration. Examples of such genes include *WAF1, GADD45,* and *Ndrg1.*

Pharmaceutical compositions according to the invention may be formulated for subcutaneous or intravenous injection, oral administration, inhalation, transdermal application, or rectal administration. In one embodiment of the invention the composition is formulated for intravenous administration. In another embodiment the composition is formulated for oral administration.

According to a fourth aspect of the invention there is provided at least one compound of Formula 3 or 4 as defined herein with or without provisos (i) and (ii), or a pharmaceutical composition comprising at least one compound of Formula 3 or 4 with or without provisos (i) and (ii) together with a pharmaceutically acceptable diluent, adjuvant, or excipient for use in iron chelation therapy in a mammal.

According to a fifth aspect of the invention there is provided the use of at least one compound of Formula 3 or 4 as defined herein with or without provisos (i) and (ii), for the preparation of a medicament for iron chelation therapy.

According to a sixth aspect of the invention there is provided at least one compound of Formula 3 or 4 as defined herein with or without provisos (i) and (ii), or a pharmaceutical composition comprising at least one compound of Formula 1, 2, 3 or 4 with or without provisos (i) and (ii) together with a pharmaceutically acceptable diluent, adjuvant, or excipient, for treating an iron overload disorder is a mammal.

According to a seventh aspect of the invention there is provided the use of at least one compound of Formula 1, 2, 3 or 4 as defined herein with or without provisos (i) and (ii), for the manufacture of a medicament for treating an iron-overload disorder.

With reference to the sixth and seventh aspects of the invention, in one embodiment the iron overload disorder is β-thalassaemia.

According to a eigth aspect of the invention there is provided at least one compound of Formula 3 or 4 as defined herein with or without provisos (i) and (ii), or a metal ion complex thereof, or a pharmaceutical composition comprising at least one compound of Formula 1, 2, 3 or 4 with or without provisos (i) and (ii), or a metal ion complex thereof, together with a pharmaceutically acceptable diluent, adjuvant, or excipient, for inhibiting cellular proliferation.

With reference to the eigth aspect of the invention, in one embodiment, compounds of Formulae 3 and 4, metal ion complexes thereof, or compositions comprising said compound or metal ion complex may be used to inhibit *in vitro* cellular proliferation. In another embodiment, compounds of Formulae 3 and 4, metal ion complexes thereof, or compositions comprising said compound or metal ion complex may be used to inhibit in vivo cellular proliferation, for example, cellular proliferation in a mammal.

According to an ninth aspect of the invention there is provided at least one compound of Formula 3 or 4 as defined herein with or without provisos (i) and (ii), or a metal ion complex thereof, or a pharmaceutical composition comprising at least one compound of Formula 3 or 4 with or without provisos (i) and (ii), or a metal ion complex thereof together with a pharmaceutically acceptable diluent, adjuvant, or excipient for treating a proliferative disorder in a mammal.

According to a tenth aspect of the invention there is provided the use of at least one compound according to Formula 3 or 4 as defined herein with or without provisos (i) and (ii), or a metal ion complex thereof, for the manufacture of a medicament for inhibiting cellular proliferation.

According to an eleventh aspect of the invention there is provided the use of at least one compound of Formula 3 or 4 as defined herein with or without provisos (i) and (ii), or a metal ion complex thereof, for the manufacture of a medicament for treating a proliferative disorder.

With reference to the ninth or eleventh aspects of the invention, the proliferative disorder may be selected from angiogenesis-dependent diseases, cellular proliferative diseases (such as psoriasis, IBD, malignancies, restenosis), inflammatory disorders, auto-immune diseases, blood vessel diseases, thrombosis, cancer. The proliferative disease may be arthritis. The cancer may be a malignant tumour or benign tumour. The malignant tumour may be metastatic. The cancer may be a solid tumour or a non-solid tumour. For example, the cancer may be a leukaemia or lymphoma.

In one embodiment, when the compound or composition of the invention is administered as the sole active agent the resultant white blood cell count in the mammal is changed, and in particular is reduced, by no more than 1%, 2%, 3%, 4%, 5%, 10%, 15%, 20%, 25%, 30%, 35%, 40%, 45%, 50%, 55%, 60%, 65%, 70%, 75%, 80%, 85% following administration of the compound or composition. Similarly, in one embodiment when the compound or composition of the invention is administered as the sole active agent the resultant red blood cell count is changed, and in particular is reduced, by no more than 1%, 2%, 3%, 4%, 5%, 10%, 15%, 20%, 25%, 30%, 35%, 40%, 45%, 50%, 55%, 60%, 65%, 70%, 75%, 80%, 85% following administration of the compound or composition.

According to a twelfth aspect of the invention there is provided at least one compound of Formula 3 or 4 as defined herein with or without provisos (i) and (ii), or a metal ion complex thereof, or a pharmaceutical composition comprising at least one compound of Formula 3 or 4 with or without provisos (i) and (ii), or a metal ion complex thereof together with a pharmaceutically acceptable diluent, adjuvant, or excipient, for inducing apaptosis in a cell.

According to a thirteenth aspect of the invention there is provided at least one compound of Formula 3 or 4 as defined herein with or without provisos (i) and (ii), or a metal ion complex thereof, or a pharmaceutical composition comprising at least one compound of Formula 3 or 4 with or without provisos (i) and (ii), or a metal ion complex thereof together with a pharmaceutically acceptable diluent, adjuvant, or excipient, for inhibiting cellular proliferation and inducing apoptosis in a cell.

According to a fourteenth aspect of the invention there is provided at least one compound of Formula 3 or 4 as defined herein with or without provisos (i) and (ii), or a metal ion complex thereof, or a pharmaceutical composition comprising at least one compound of Formula 3 or 4 with or without provisos (i) and (ii), or a metal ion complex thereof together with a pharmaceutically acceptable diluent, adjuvant, or excipient, for inducing apoptosis in a mammal.

According to a fifteenth aspect of the invention there is provided at least one compound of Formula 3 or 4 as defined herein with or without provisos (i) and (ii), or a metal ion complex thereof, or a pharmaceutical composition comprising at least one compound of Formula 3 or 4 with or without provisos (i) and (ii), or a metal ion complex thereof together with a pharmaceutically acceptable diluent, adjuvant, or excipient, for inducing apoptosis and inhibiting cellular poliferation in a mammal.

According to a sixteenth aspect of the invention there is provided the use of at least one compound of Formula 3 or 4 as defined herein with or without provisos (i) and (ii), or a metal ion complex thereof, for the manufacture of a medicament for inducing apoptosis.

According to a seventeenth aspect of the invention there is provided the use of at least one compound of Formula 3 or 4 as defined herein with or without provisos (i) and (ii), or a metal ion complex thereof, for the manufacture of a medicament for inducing apoptosis and inhibiting cellular proliferation.

According to a eighteenth aspect of the invention there is provided at least one compound of Formula 3 or 4 as defined herein with or without provisos (i) and (ii), or a metal ion complex thereof, or a pharmaceutical composition comprising at least one compound of Formula 3 or 4 with or without provisos (i) and (ii), or a metal ion complex thereof together with a pharmaceutically acceptable diluent, adjuvant, or excipient, when used for any one or more of inhibiting cellular proliferation, treating a proliferative disorder, treating cancer, inducing apoptosis, iron chelation therapy, or treating an iron-overload disorder.

According to a nineteenth aspect of the invention there is provided at least one compound of Formula 3 or 4, with or without provisos (i) or (ii), or an iron complex thereof, or a composition comprising said compound or complex together with a pharmaceutically acceptable adjuvant, diluent or excipient, for treating or inhibiting a condition selected from the group consisting of cellular proliferative disorder, cancer, iron-overload disorder in a mammal, or inducing apoptosis in a mammal, or inducing apoptosis and inhibiting cellular proliferation in a mammal, wherein the resultant white and/or red blood cell count in the mammal is changed, and in particular is reduced, respectively by no more than 1%, 2%, 3%, 4%, 5%, 10%, 15%, 20%, 25%, 30%, 35%, 40%, 45%, 50%, 55%, 60%, 65%, 70%, 75%, 80%, or 85% following administration of the compound or composition to said mammal.

With reference to any one of the third and eighth to nineteenth aspects of the invention, the metal ion may be a transition metal ion. For example, the metal ion may be selected from iron, copper, zinc, palladium, platinum, and gallium ions. In one embodiment the metal ion is iron(II). In another embodiment, the metal ion is iron(III).

With reference to any one of the fourth, sixth, eigth, ninth, fourteenth fifteenth, and nineteenth aspects of the invention, the mammal may be human, non-human primate, murine, bovine, ovine, equine, caprine, leporine, avian, feline, porcine, or canine. In one embodiment the mammal is human.

A process for identifying an anti-proliferative compound is also disclosed said process comprising:
contacting a cell or cellular extract having a gene encoding one or more cell cycle inhibitors, with a compound capable of chelating iron,
determining the level of expression of said gene,
determining whether the level of expression of said gene differs in comparison to the expression of said gene in the absence of said compound;
and thereby determining whether said compound has anti-proliferative activity.

A process for screening a plurality of compounds to identify one or more anti-proliferative compounds is also disclosed said process comprising:
contacting a cell or cellular extract having gene encoding one or more cell cycle inhibitors, with a plurality of compounds capable of chelating iron,
determining whether any of said compounds modify the level of expression of said nucleic acid, and if so, separately determining the level of expression of said gene for each compound,
determining whether said level of expression differs comparison to the level of expression of said in the absence of each said compound;
and thereby determining whether each said compound has anti-proliferative activity.

Expression of the gene is up-regulated. In another embodiment, expression of the gene is down-regulated. In one embodiment the gene expressing cell cycle inhibitors may be *WAF1.* In another embodiment of the invention, the gene expressing cell cycle inhibitors may be *GADD45.* In a further embodiment, the gene is Ndrg1.

With reference to the twenteth and twenty-first aspects of the invention, expression of the gene (e.g., mRNA, protein) in the absence of said compound may be separately determined prior to, or after, determining expression of said gene in the presence of said compound.

With reference to the twenteth and twenty-first aspects of the invention, in one embodiment said compound or plurality of compounds is capable of chelating a metal ion. In another embodiment, said compound or plurality of compounds is capable of chelating iron. In a further embodiment, the compound is a compound of Formula 3 or 4 as defined herein, including or excluding provisos (i) and (ii).

With reference to any one of the first to twenty-first aspects of the invention, in one embodiment each said compound of Formula 3 or 4 is as defined in any one of the first or second aspects of the invention including provisos (i) and (ii).

The present invention includes within its scope all isomeric forms of compounds of Formulae 3 and 4, and metal ion complexes thereof. For example, as appropriate, the present invention includes all possible enantiomers, diasteriomers, racemates, cis isomers, trans isomers, (R), (S), (+), (-), Δ, and A isomers of the compounds and/or their metal complexes.

### Definitions

The following are some definitions that may be helpful in understanding the description of the present invention. These are intended as general definitions and are put forth for a better understanding of the following description.

Unless the context requires otherwise or specifically stated to the contrary, integers, steps, or elements of the invention recited herein as singular integers, steps or elements clearly encompass both singular and plural forms of the recited integers, steps or elements.

Throughout this specification, unless the context requires otherwise, the word "comprise", or variations such as "comprises" or "comprising", will be understood to imply the inclusion of a stated step or element or integer or group of steps or elements or integers, but not the exclusion of any other step or element or integer or group of elements or integers. Thus, in the context of this specification, the term "comprising" means "including principally, but not necessarily solely".

The invention also includes all of the steps, features, compositions and compounds referred to or indicated in the claims of this specification, individually or collectively, and any and all combinations or any two or more of said steps or features.

As used herein, the term "alkyl group" includes within its meaning straight chain or branched chain saturated aliphatic groups having from 1 to 10 carbon atoms, eg, 1, 2, 3, 4, 5, 6, 7, 8, 9 or 10 carbon atoms, or cyclic saturated aliphatic groups having from 3 to 8 carbon atoms, eg, 3, 4, 5, 6, 7, or 8 carbon atoms, or bicyclic saturated aliphatic groups having 7, 8, 9 or 10 carbon atoms. For example, the term alkyl includes, to, methyl, ethyl, 1-propyl, isopropyl, 1-butyl, 2-butyl, tert-butyl, amyl, 1,2-dimethylpropyl, 1,1-dimethylpropyl, pentyl, isopentyl, hexyl, 4-methylpentyl, 1-methylpentyl, 2-methylpentyl, 3-methylpentyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, 2-ethylpentyl, 3-ethylpentyl, heptyl, 1-methylhexyl, 2,2-dimethylpentyl, 3,3-dimethylpentyl, 4,4-dimethylpentyl, 1,2-dimethylpentyl, 1,3-dimethylpentyl, 1,4-dimethylpentyl, 1,2,3-trimethylbutyl, 1,1,2-trimethylbutyl, 1,1,3-trimethylbutyl, 5-methylheptyl, 1-methylheptyl, octyl, nonyl, decyl, cyclopropyl, 2-methylcyclopropyl, cyclobutyl, cyclopentyl, 2-methylcyclopentyl, 3-methylcyclopentyl, cyclohexyl.

The term "alkenyl group" includes within its meaning straight or branched chain unsaturated aliphatic hydrocarbon groups having from 2 to 8 carbon atoms, e.g., 2, 3, 4, 5, 6, 7, or 8 carbon atoms, or cyclic unsaturated aliphatic hydrocarbon groups having from 3 to 8 carbon atoms, e.g, 3, 4, 5, 6, 7 or 8 carbon atoms, and combinations thereof, having at least one double bond, of either E or Z stereochemistry where applicable. Examples of alkenyl groups include ethenyl, vinyl, allyl, 1-methylvinyl, 1-propenyl, 2-propenyl, 2-methyl-1-propenyl, 2-methyl-1-propenyl, 1-butenyl, 2-butenyl, 3-butentyl, 1,3-butadienyl, 1-pentenyl, 2-pententyl, 3-pentenyl, 4-pentenyl, 1,3-pentadienyl, 2,4-pentadienyl, 1,4-pentadienyl, 3-methyl-2-butenyl, 1-hexenyl, 2-hexenyl, 3-hexenyl, 1,3-hexadienyl, 1,4-hexadienyl, 2-methylpentenyl, 1-heptenyl, 2-heptentyl, 3-heptenyl, 1-octenyl, and the like.

The term "alkynyl group" as used herein includes within its meaning straight or branched chain unsaturated aliphatic hydrocarbon groups having from 2 to 8 carbon atoms, e.g., 2, 3, 4, 5, 6, 7, or 8 carbon atoms, and having at least one triple bond. Examples of alkynyl groups include ethynyl, 1-propynyl, 1-butynyl, 2-butynyl, 1-methyl-2-butynyl, 3-methyl-1-butynyl, 1-pentynyl, 1-hexynyl, methylpentynyl, 1-heptynyl, 2-heptynyl, 1-octynyl, 2-octynyl, and the like.

The term "heteroatom" as used herein refers to any atom other than carbon or hydrogen, including for example, oxygen, nitrogen and sulfur.

The term "halide" or "halo atom" as used herein refers to fluoro, chloro, bromo and iodo.

The term "amino group", or variants such as "amino" as used herein refers to groups of the form -NR₆R₇ wherein R₆ and R₇ are individually selected from the group including but not limited to hydrogen, optionally substituted alkyl, optionally substituted alkenyl, optionally substituted alkynyl, and optionally substituted aryl groups.

The term "aromatic group", or variants such as "aryl" as used herein refers to single, polynuclear, conjugated and fused residues of aromatic hydrocarbons having from 6 to 14 carbon atoms, e.g., 6, 7, 8, 9, 10, 11, 12, 13, or 14 carbon atoms. Examples of such groups include phenyl, tolyl, biphenyl, naphthyl, phenanthryl, and the like.

The term "heteroaromatic group" or variants such as "heteroaryl" as used herein includes within its meaning any 5-16-membered, eg., 5-, 6-, 7-, 8-, 9-, 10-, 11-, 12-, 13-, 14- ,15- or 16-membered monocyclic, bicyclic or tricyclic aromatic group having 1, 2, 3 or 4 heteroatoms. Examples include, pyrrolyl, pyridyl, phenanthryl, quinolyl, isoquinolyl, imidazolyl, thiophenyl, furanyl, purinyl, indolyl, isoindolyl, phenanthridinyl, pyrimidinyl, pyridazinyl, pyrazinyl, pyrazolyl, oxazolyl, isoxazolyl, thiazolyl, benzofuranyl, and the like.

The term "heterocycloalkyl group" as used herein includes within its meaning any 3-10-membered saturated or unsaturated monocyclic or bicyclic ring system, having 1, 2 or 3 heteroatoms. Examples include piperidinyl, pyrrolidinyl, tetrahydrofuranyl, tetrahydropyrrolyl, morpholinyl, tetrahydrothienyl, and the like.

The term "optionally substituted" as used herein means the group to which this term refers may be unsubstituted, or substituted with one or more groups independently selected from halogen, alkyl, alkenyl, alkynyl, cycloalkyl, cycloalkenyl, heterocycloalkyl, aryl, heteroaryl, cyano, cyanate, isocyanate, -O-alkyl, -S-alkyl, nitro, amino, -C(O)NH₂, -C(O)NH(alkyl), -C(O)N(alkyl)₂, and -C(O)-alkyl, -C(O)-alkenyl, -C(O)-O-alkyl.

The language "therapeutically effective amount" is intended to include within its meaning a non-toxic but sufficient amount of a compound or composition of the invention to provide the desired therapeutic effect. The exact therapeutically effective amount of the compound or composition will vary according to factors such as the type of disease of the animal, the age, sex, and weight of the animal, mode of administration, and the ability of the compound or composition to permeate cell membranes and chelate metal ions, such as iron, in cells of the animal. Dosage regima can be adjusted to provide the optimum therapeutic response. For example, several divided doses can be administered daily or the dose can be proportionally reduced as indicated by the exigencies of the therapeutic situation.

As used herein the term "treatment", refers to any and all uses which remedy a disease state or symptoms, prevent the establishment of disease, or otherwise prevent, hinder, retard, or reverse the progression of disease or other undesirable symptoms in any way whatsoever.

### Abbreviations

DFO - desferrioxamine
EDTA - ethylenediamine tetraacetic acid
MTT - [3-(4,5-dimethylthiazol-2-yl)-2,5-diphenyl tetrazolium]
Tf - transferrin
"311" - 2-hydroxy-1-naphthylaldehyde isonicotinoyl hydrazone
"311m" - 2-hydroxy-1-naphthylaldehyde nicotinoyl hydrazone
DpT - di-2-pyridylketone 3-thiosemicarbazone
Dp2mT - di-2-pyridylketone 2-methyl-3-thiosemicarbazone
Dp4mT - di-2-pyridylketone 4-methyl-3-thiosemicarbazone
Dp44mT - di-2-pyridylketone 4,4-dimethyl-3-thiosemicarbazone
Dp4eT - di-2-pyridylketone 4-ethyl-3-thiosemicarbazone
Dp4aT - di-2-pyridylketone 4-allyl-3-thiosemicarbazone
Dp4pT - di-2-pyridylketone 4-phenyl-3-thiosemicarbazone
PCBH - 2-pyridylketone benzoyl hydrazone
PCBBH - 2-pyridylketone 3-bromobenzoyl hydrazone
PCAH -2-pyridylketone 4-aminobenzoyl hydrazone
PCHH- 2-pyridylketone 4-hydroxybenzoyl hydrazone
PCIH - 2-pyridylketone isonicotinoyl hydrazone
PCTH - 2-pyridylketone 2-thiophenecarboxaldehyde hydrazone
PKIH - di-2-pyridylketone isonicotinoyl hydrazone
PKBH - di-2-pyridylketone benzoyl hydrazone
PKHH - di-2-pyridylketone 4-hydroxybenzoyl hydrazone
PKBBH - di-2-pyridylketone 3-bromobenzoyl hydrazone
PKAH - di-2-pyridylketone 4-aminobenzoyl hydrazone
PKTH - di-2-pyridylketone 2-thiophenecarboxaldehyde hydrazone
PK44pH - di-2-pyridylketone 4,4-diphenylcarboxaldehyde semicarbazone
PKoctH - di-2-pyridylketone octanoic hydrazone
QCIH - 2-quinolinecarboxaldehyde isonicotinoyl hydrazone
QCTH - 2-quinolinecarboxaldehyde 2-thiophenecarboxaldehyde hydrazone
QCBH - 2-quinolinecarboxaldehyde benzoyl hydrazone
QCBBH - 2-quinolinecarboxaldehyde 3-bromobenzoyl hydrazone
QCAH - 2-quinolinecarboxaldehyde 4-aminobenzoyl hydrazone
QCHH - 2-quinolinecarboxaldehyde 4-hydroxybenzoyl hydrazone

### Brief description of the drawings

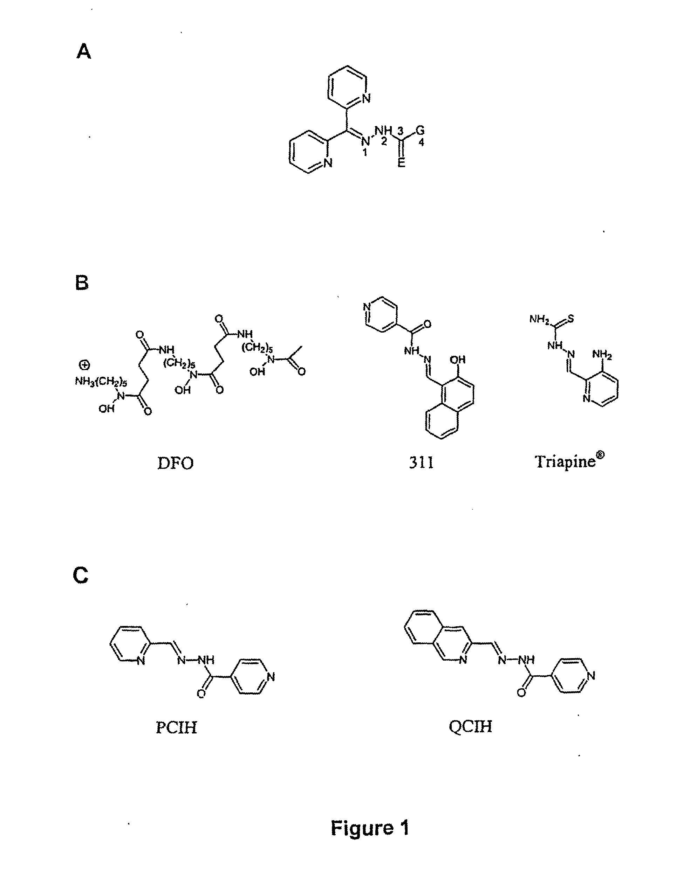

**Figure 1. (A)** General compound structure showing numbering scheme used. **(B)** Structures of DFO, 311 and Triapine®; **(C).** General structures of comparative PCIH and QCIH compounds.

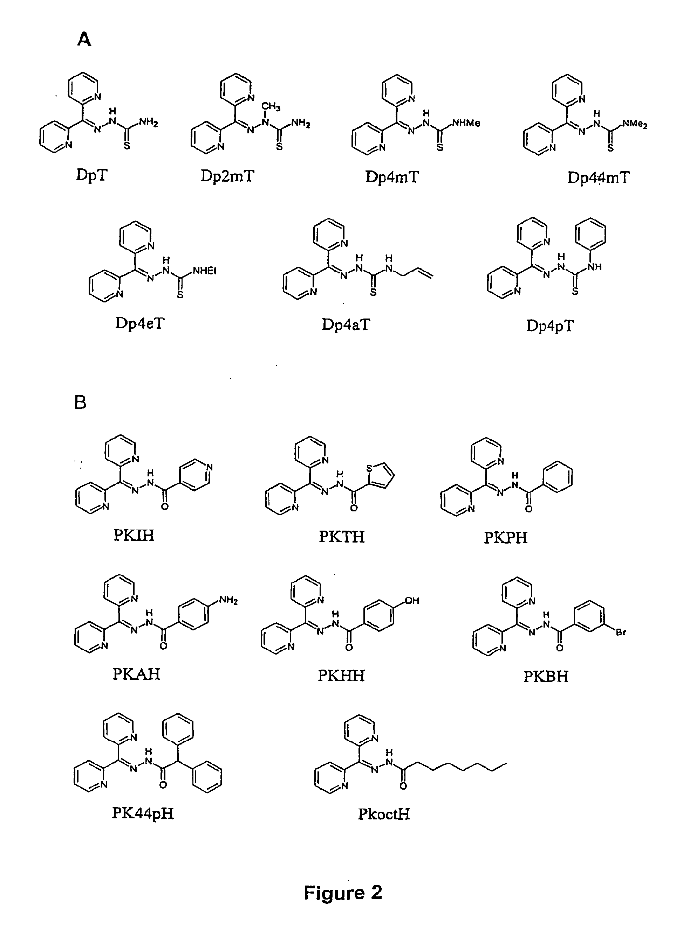

**Figure 2. (A).** Structures of representative DpT analogues, including: DpT, Dp2mT, Dp4mT, Dp44mT, Dp4eT, Dp4aT, and Dp4pT. **(B)** Structures of representative PKIH analogues, including PKIH, PKTH, PKPH, PKAH, PKHH, PKBH, PK44pH, and PKoctH.

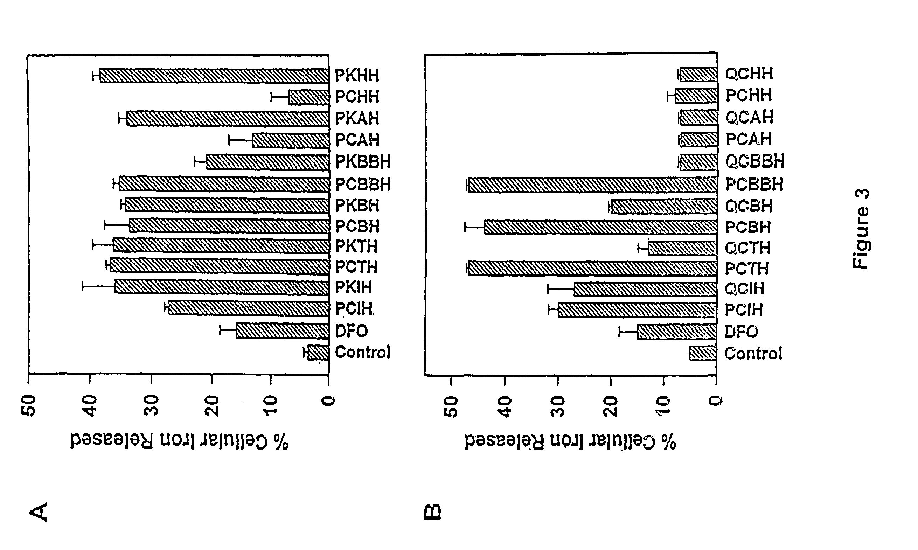

**Figure 3****.** Bar graphs of the effect of the chelators on ⁵⁹Fe mobilization from prelabelled SK-N-MC neuroepithelioma cells. **(A)** Comparison of the PKTH and PCIH analogues; **(B)** Comparison of the QCIH and PCIH analogues. Results are expressed as the mean ± SD of 3 replicates in a typical experiment of 2 experiments performed.

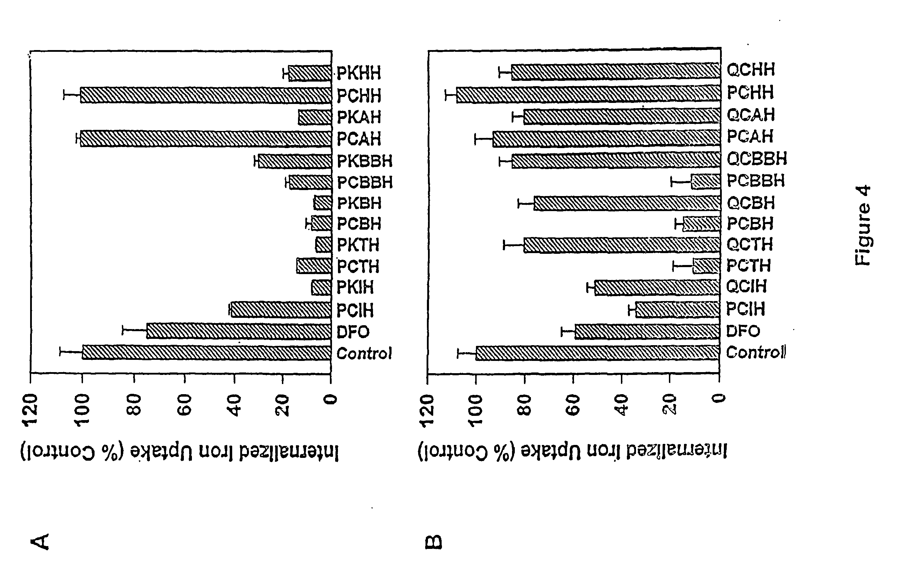

**Figure 4****.** Bar graphs of the effect of the chelators on ⁵⁹Fe uptake from ⁵⁹Fe-transferrin (⁵⁹Fe-Tf) by SK-N-MC neuroepithelioma cells. **(A)** Comparison of the PKIH and PCIH analogues; **(B)** Comparison of the QCIH and PCIH analogues. Results are expressed as the mean ± SD of 3 replicates in a typical experiment of 2 experiments performed.

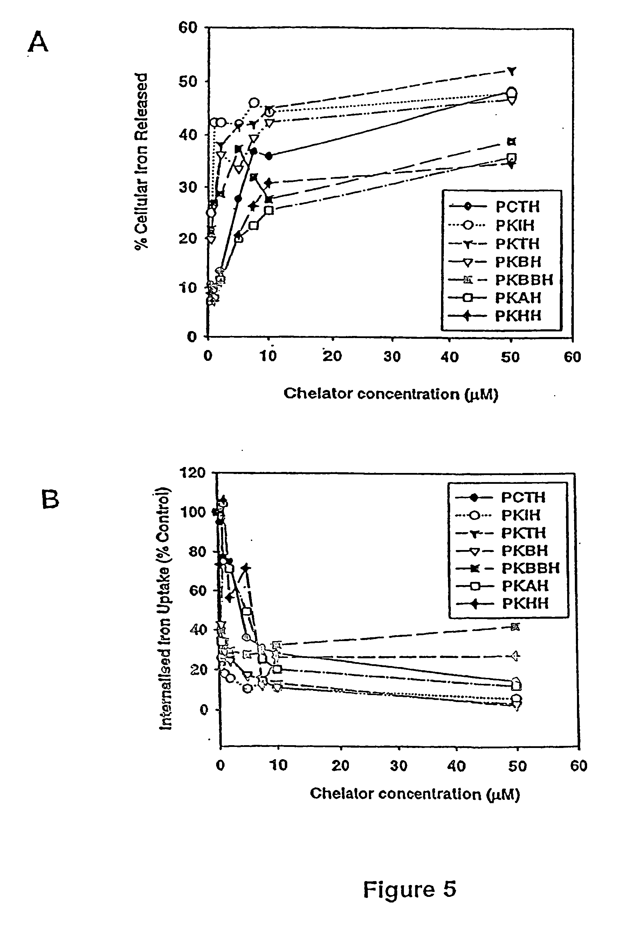

**Figure 5****.** Line graphs of the effect of concentration of the PKIH analogues compared to PCTH on: **(A)** cellular ⁵⁹Fe mobilization and **(B)** internalized ⁵⁹Fe uptake from ⁵⁹Fe-transferrin (⁵⁹Fe-Tf). Results are expressed as the mean ± SD of 3 replicates in a typical experiment of 2 experiments performed.

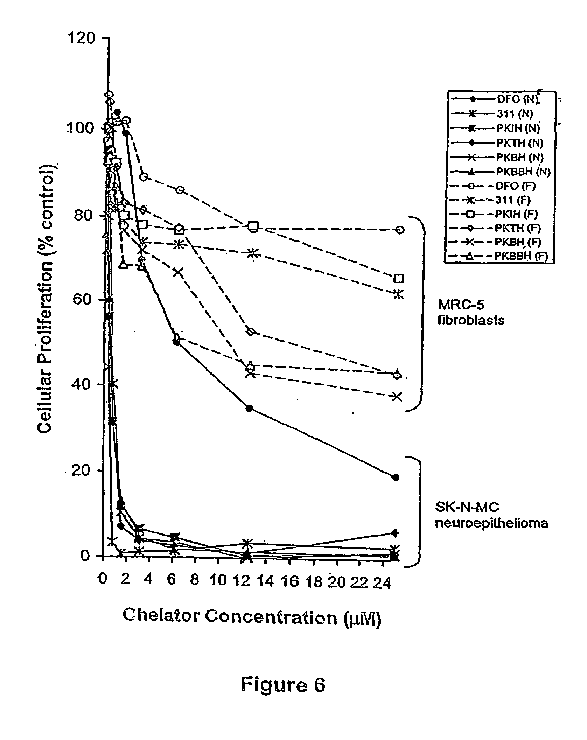

**Figure 6****.** Line graph of the influence of representative PKIH analogues, desferrioxamine (DFO), and 2-hydroxy-1-napthylaldehyde isonicotinoyl hydrazone (311) on the proliferation of SK-N-MC neuroepithelioma cells (N) compared to MRC-5 fibroblasts (F). Each data point represents the mean of 3 separate experiments with duplicate determinations in each experiment.

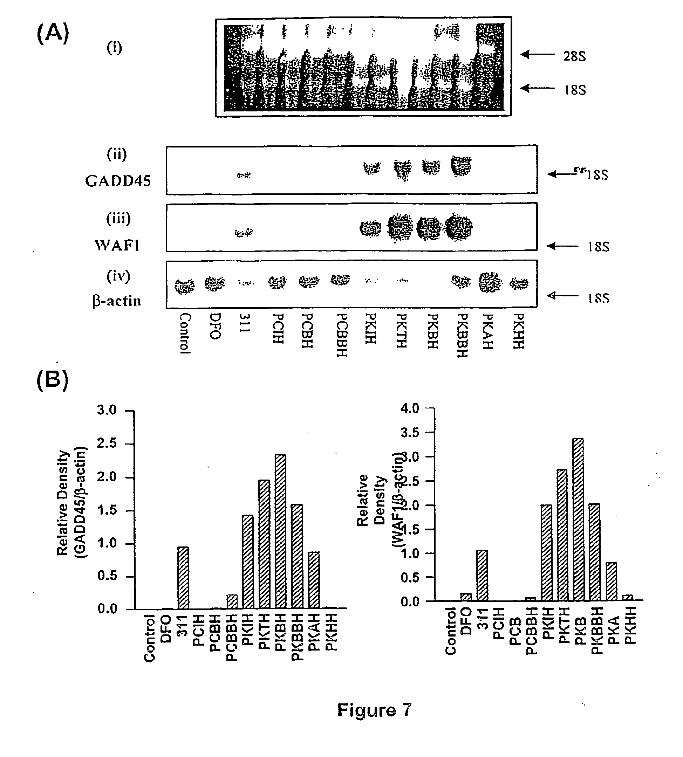

**Figure 7****.** Members of the PKIH series of Fe chelators markedly increase *WAF1* and *GADD45* mRNA expression in SK-N-MC neuroepithelioma cells. The effects of PKIH analogues are compared to DFO, 311 and some chelators of the PCIH series. **(A)** (i) Ethidium bromide staining of the agarose gel, (ii) *GADD45,* (iii) *WAF1*, and (iv) β-*actin* mRNA levels; **(B)** Densitometric analysis of the results in (A) normalised to the β*-actin* loading control. Total RNA was extracted from cells after a 20 h incubation at 37°C with medium alone (control) or medium containing DFO (100 µM) or the other chelators (25 µM). Northern blotting was performed using standard procedures (see Example 6). The result illustrated is a typical experiment from three experiments performed.

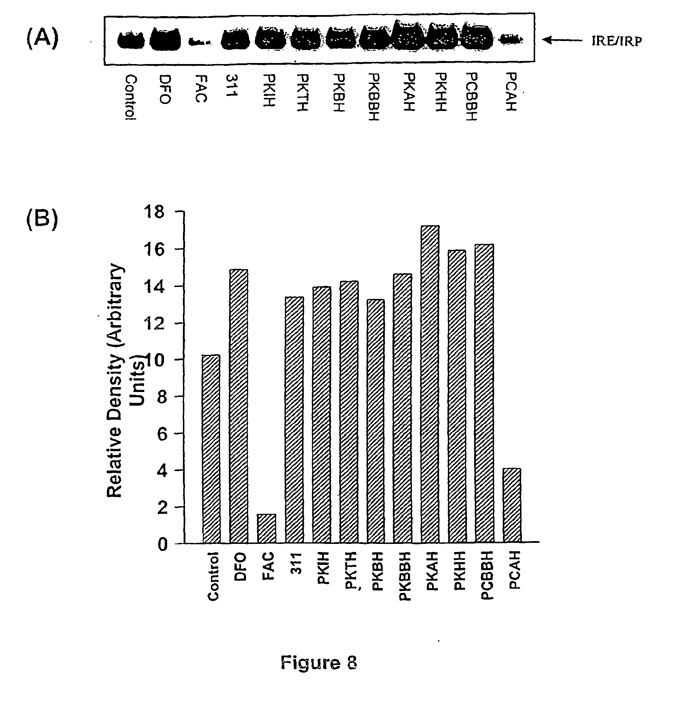

**Figure 8****.** PKIH analogues increase iron-regulatory protein (IRP)-RNA-binding activity in SK-N-MC neuroepithelioma cells. **(A)** Active IRP-RNA-binding activity; **(B)** Densitometric analysis of the results in (A). The effect of the PKIH analogues were compared to several chelators that acted as internal controls (ie., DFO, 311, PCBBH, and PCAH) and also the Fe donor, ferric ammonium citrate (FAC). Cells were incubated with DFO (100 µM), FAC (100 µg/ml) and the remaining chelators (25 µM) for 20 h at 37°C. The IRP-RNA-binding activity was then assessed by the gel-retardation assay using standard techniques. The result illustrated is a typical experiment from three experiments performed.

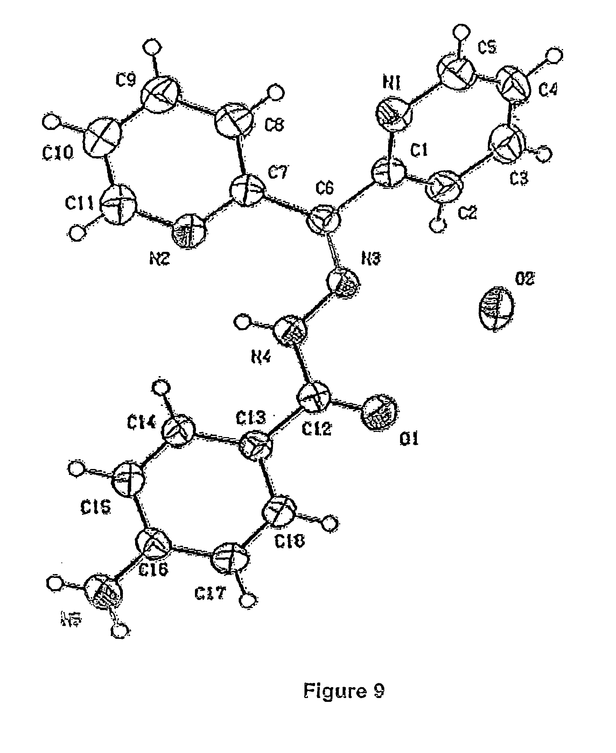

**Figure 9****.** Crystal structure of PKAH as determined by X-ray crystallography showing 30% thermal ellipsoids and water of solvation.

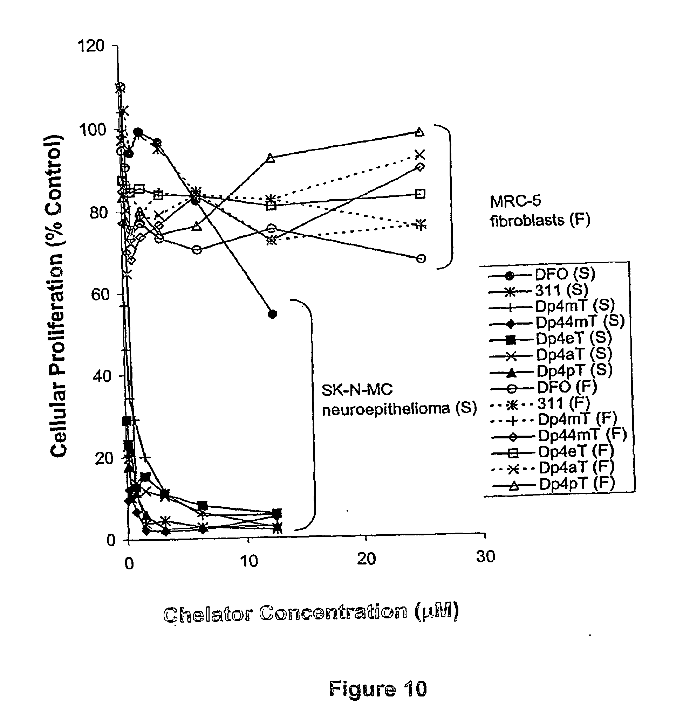

**Figure 10****.** DpT analogues and 311 (internal control) show selective anti-proliferative activity against immortal SK-N-MC neuroepithelioma cells (S) compared to mortal MRC-5 fibroblasts (F). Cells were incubated in the presence or absence of the compounds (0-25µM for 72 h at 37°C. After this incubation period, cellular density was measured using the MTT assay. Each data point represents the mean of 2 replicates in a typical experiment of at least 3-5 experiments performed.

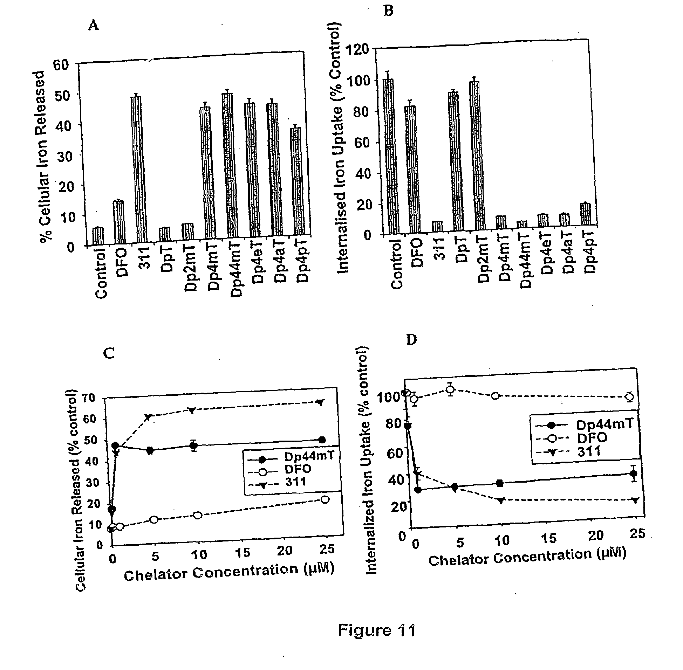

**Figure 11****.** The effect of the DpT analogues compared to DFO and 311 on ⁵⁹Fₑ mobilization and cellular ⁵⁹Fe uptake from ⁵⁹Fe-transferrin (⁵⁹Fe-Tf) in SK-N-M_{C} neuroepithelioma and M109 cells. **(A)** The effect of chelators on ⁵⁹Fe mobilization from prelabeled SK-N-MC neuroepithelioma cells. **(B)** The effect of the chelators at preventing ⁵⁹Fe uptake from ⁵⁹Fe-Tf by SK-N-MC cells. **(C)** The effect of the chelators on ⁵⁹Fe mobilization from prelabeled M109 cells as a function of chelator concentration. **(D)** The effect of the chelators at preventing ⁵⁹Fe uptake from ⁵⁹Fe-Tf by M109 cells as a function of chelator concentration. Results are expressed as the mean ± SD of 3 replicates in a typical experiment of three performed.

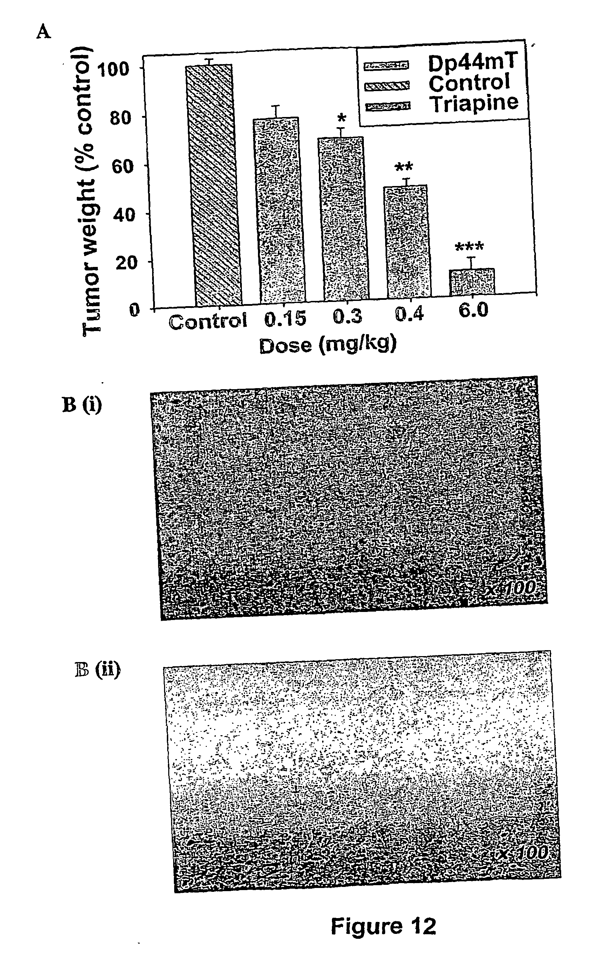

**Figure 12. (A)** Dp44mT and to a greater extent, Triapine^{®}, markedly decreased the growth of a M109 lung carcinoma in mice after a 5 day treatment regimen, and **(B)** the induction of apoptosis in tumors after injection of (i) vehicle control or (ii) Dp44mT as judged by the TUNEL assay.

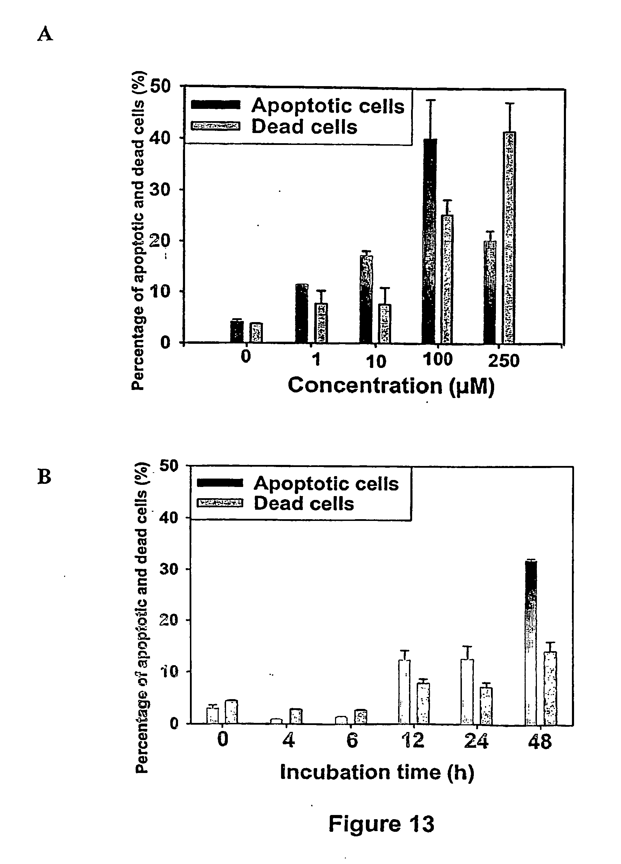

Figure 13. Effect of Dp44mT on M109 cellular apoptosis or necrosis/late stage apoptosis as a function of **(A)** chelator concentration and **(B)** incubation time. **(A)** M109 cells were incubated with Dp44mT at 0-250 µM for 24 h at 37°C. Cellular apoptosis or necrosis/late stage apoptosis were measured by Annexin V-FITC and PI staining, respectively (see *Materials and Methods* for details). **(B)** M109 cells were treated with Dp44mT (1 µM) for various incubation times (0-48 h). Cellular apoptosis and necrosis/late stage apoptosis were measured as described in **(A).** Data plotted are mean ± SEM of three separate experiments.

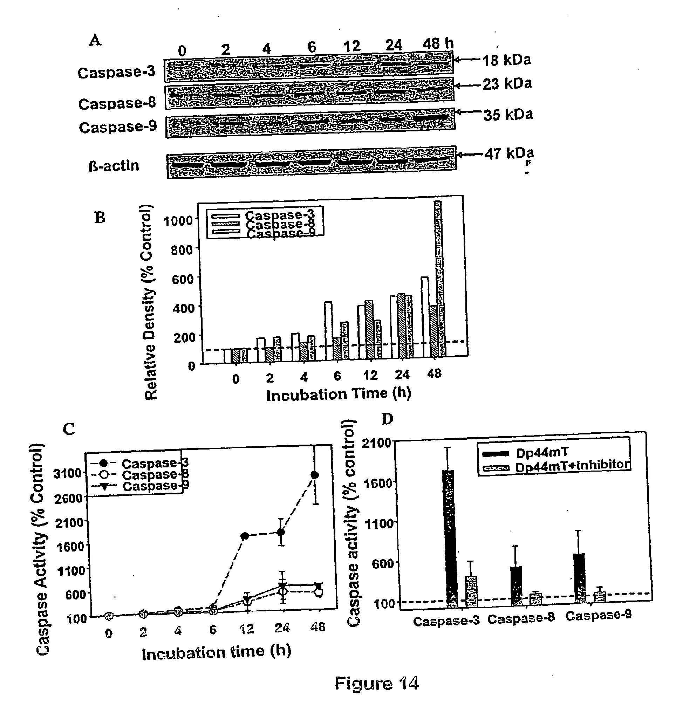

**Figure 14****.** Effect of Dp44mT (1 µM) on the **(A,B)** protein levels of active caspase-3, -8 and -9, and **(C,D)** the activity of caspase-3, -8 and -9 in cultured M109 cells in the absence **(C)** and presence **(D)** of cell-permeable caspase inhibitors. **(A)** Caspase-3, -8, and -9 levels after Dp44mT treatment at indicated times as detected by western blotting (upper panel). The anti-β-actin antibody was used to ensure equal protein loading (lower panel). **(B)** Densitometric analysis of the expression of caspase-3, -8, and -9 as a function of time normalized to β-actin (C) Caspase activity induced by Dp44mT (1 µM) at 0-48 h was expressed as a percentage of the 0 h time value. **(D)** Cell-permeable inhibitors of caspase-3, -8 or -9 at 1 µM prevented activation of these enzymes when incubated with Dp44mT (1 µM) for 48 h. Results are mean ± SEM of three separate experiments.

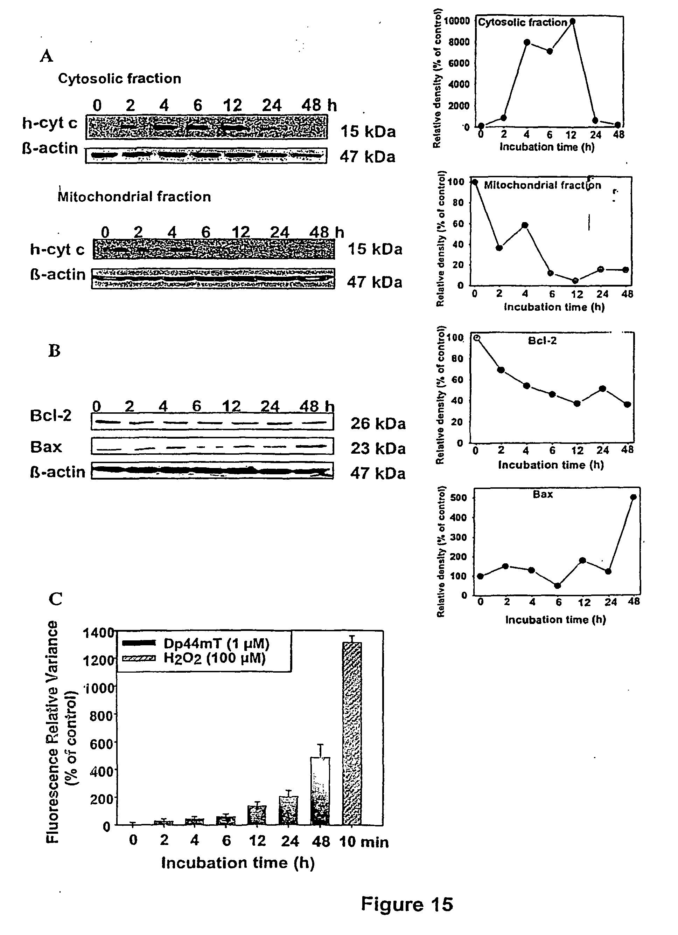

**Figure 15****.** Effect of Dp44mT (1 µM) in cultured M109 cells on: (A) the holo-cytochrome c (h-cytc) levels in cytosolic and stromal-mitochondrial membrane (SMM) fractions; (B) mitochondrial protein levels of Bcl-2 and Bax; (C) intracellular ROS production.

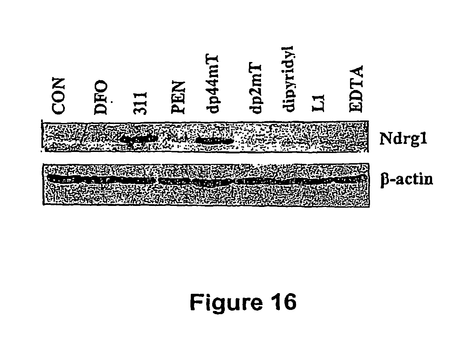

**Figure 16****.** Only the membrane-permeable chelators 311 and Dp44mT with high anti-proliferative activity and Fe chelation efficacy increase Ndrgl protein expression when examined at 25 µM. MCF-7 cells were incubated for 24 h at 37°C with 25 µM of DFO, 311, pencillamine, dp44mT, dp2mT, dipyridyl, L1 or EDTA. The expression of Ndrgl and β-actin protein levels were assessed by Western blotting. Results are a typical experiment from 3 performed.

### Detailed Description of Referred Embodiments of the Invention

The present invention relates to compounds which are capable of chelating metal ions. In particular, the present invention relates to (thio)semicarbazone and (thio)hydrazone compounds of Formulae 3 and 4: 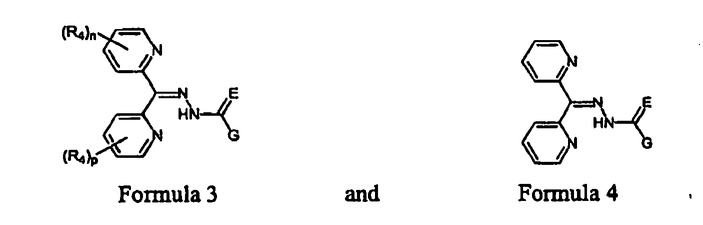 wherein E, n, p, and G are defined above, including provisos (i) and (ii).

The present invention also relates to the use of compounds of Formulae 1, 2, 3 and 4 as defined herein, including or excluding provisos (i) and (ii). Accordingly, the present invention encompasses the use of compounds of Formulae 3a and 4a below: 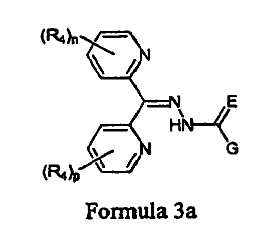 wherein
E is O or S;
n is 0, 1, 2, 3 or 4,
p is 0, 1, 2, 3 or 4, and
each R₄ is independently selected from halogen, alkyl, alkenyl, optionally substituted amino, hydroxyl, -O-alkyl, -S-alkyl, -C(O)-alkyl, -C(O)-alkenyl, -C(O)-O-alkyl, nitro and cyano,
and
   -G is NR₂R₃ or CR₂R₃R₅,
   wherein the bond between C and R₅ may be a single bond, double bond, or triple bond; and wherein when the bond between C and R₅ is a double bond, one of R₂ and R₃ is absent, and wherein when the bond between C and R₅ is a triple bond, R₂ and R₃ are both absent;
   R₅ is selected from the group consisting of hydrogen, halogen, optionally substituted alkyl, optionally substituted alkenyl, optionally substituted alkynyl, optionally substituted amino, optionally substituted heteroaromatic, an optionally substituted bicyclic group, and an optionally substituted aromatic group;
   R₂ and R₃ may be the same or different and are individually selected from hydrogen, halogen, optionally substituted alkyl, optionally substituted alkenyl, optionally substituted alkynyl, optionally substituted amino, optionally substituted heteroaromatic, an optionally substituted bicyclic group, and an optionally substituted aromatic group;
   or -G is an optionally substituted heteroaromatic group, an optionally substituted bicyclic group, an optionally substituted aromatic group, an optionally substituted alkyl group; an optionally substituted cycloalkyl group, or an optionally substituted heterocycloalkyl group;
   and 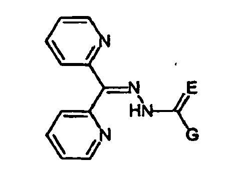 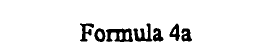
wherein
E is O or S; and
-G is as defined above for Formula 3a.

Accordingly, the expressions "a compound of formula 3 and/or 4 with or without provisos (i) and (ii)", and "a compound of formula 3 and/or 4 including or excluding provisos (i) and (ii)" should be understood to encompass compounds of Formulae 3 and 4, as well as compounds of Formulae 3a and 4a.

As used herein, the generic term "(thio)semicarbazone" includes within its scope semicarbazones, thiosemicarbazones, and derivatives or related analogues thereof. Similarly, as used herein, the generic term "(thio)hydrazone" includes within its scope hydrazones, thiohydrazones and derivatives or related analogues thereof.

With reference to Formulae 3, 3a, 4 and 4a, when E is O, the compounds are sometimes referred to herein as "PKIH analogues" or the "PKIH series"; when E is S, the compounds are sometimes referred to herein as "DpT analogues" or the "DpT series".

Compounds of Formulae 3, 3a, 4 and 4a may chelate metal ions to form metal ion complexes. Examples of metal ions capable of forming a metal ion complex with compounds of Formulae 3, 3a, 4 and 4a include transition metal ions, such as for example, iron, copper, zinc, palladium, platinum and gallium ions. In one embodiment the metal ion is iron(III). In another embodiment the metal ion is iron(II). In a further embodiment the metal ion is zinc(II).

The invention also encompasses pharmaceutical formulations comprising at least one compound of Formulae 3 and/or 4 including provisos (i) and (ii), and/or a metal ion complex of each said compound. The present invention also relates to therapeutic uses of compounds of Formulae 3 and 4 as defined herein including or excluding provisos (i) and (ii), or compositions comprising such compounds and/or metal ion complexes thereof.

Compounds of Formulae 3, 3a, 4 and 4a comprise a (thio)semicarbazone or (thio)hydrazone backbone. In one embodiment, compounds of Formulae 3 and 4 function as tridentate ligands. In one embodiment, the pyridyl nitrogen, the imino (C=N) nitrogen, and the carbonyl (C=O) or thiocarbonyl (C=S) groups may each serve as donor atoms to coordinate a suitable metal ion. Those skilled in the art will appreciate that when A and B are not identical, there may be cis/trans stereoisomerisation about the C=N double bond. Accordingly, in one embodiment, when A and B are non-identical optionally substituted heteroaromatic rings, a compound of Formula 3 may be capable of forming a metal ion complex through the heteroatom of ring A or ring B, depending on the cis/trans stereochemistry. This is illustrated schematically below, where M is a suitable metal ion:

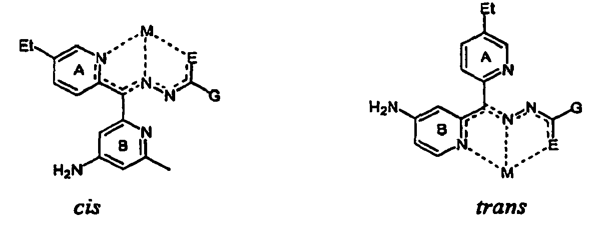

Compounds of Formulae 3, 3a, 4 and 4a may function as tridentate ligands and two compounds may form a hexacoordinate metal ion complex with a suitable metal ion, e.g., a metal ion capable of forming octahedral complexes.

Suitable methods for the synthesis of compounds of Formulae 3 and 4 are well known to those skilled in the art and some are described, for example, in J. March, Advanced Organic Chemistry, 4th Edition (John Wiley & Sons, New York, 1992); and Vogel's Textbook of Practical Organic Chemistry, 5th Edition (John Wiley & Sons, New York, 1989).

Compounds of Formulae 3 and 4 according to the present invention may be prepared by means of a Schiff base condensation reaction in which a ketone or aldehyde is condensed with either an acid hydrazide or acid thiosemicarbazide of choice to produce the corresponding (thio)semicarbazone or (thio)hydrazone compound of Formula 3 or 4 having a desired substitution pattern.

A suitable synthetic method of (thio)semicarbazone and (thio)hydrazone compounds has been described, for example, by Bacchi *et al.* (1996). A typical example of a general synthetic route for preparing PKIH analogues of Formulae 3 and 4 is represented in the following reaction scheme:

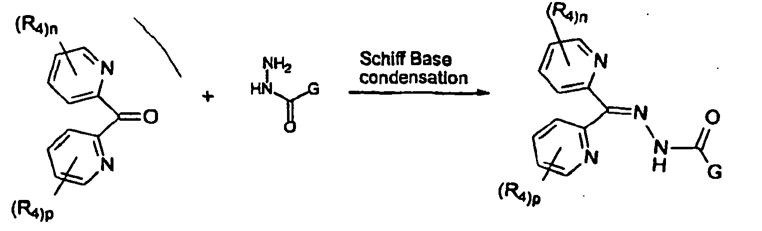

An example of a synthetic route for preparing PKIH analogues of Formula 4, is illustrated below for the compounds PK44pH and PKoctH:

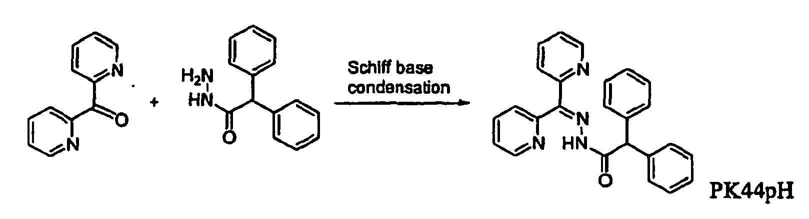

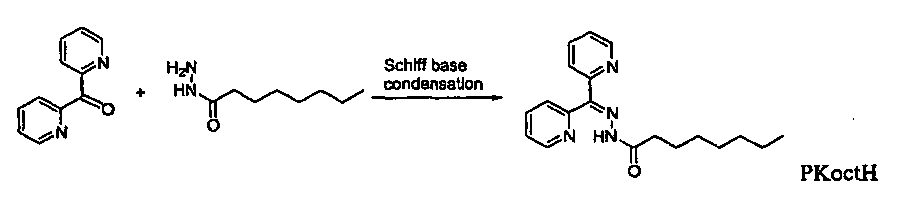

A suitable method for preparing DpT analogues has been described by Johnson *et al.* (1982). An example of a general synthetic route for preparing DpT analogues of Formulae 3 and 4 as defined herein, is represented in the following general reaction scheme:

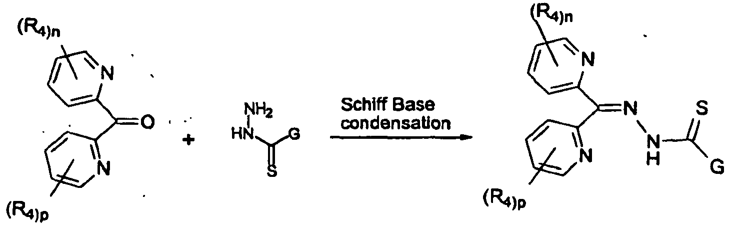

An example of a synthetic route for preparing DpT analogues of Formula 4 as defined herein, is illustrated below for the compounds Dp4eT and Dp4aT:

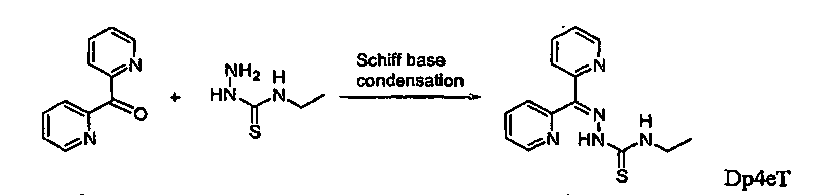

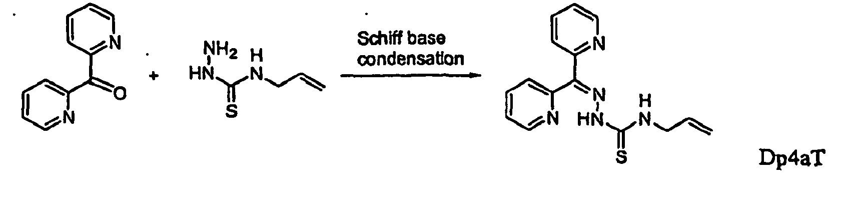

The condensation reactions represented above may be carried out under conditions known to those skilled in the art. For example, suitable solvent systems include ethanol, methanol, ethanol/water, methanol/water, or other common organic solvents such as acetone, benzene, toluene, etc. Compounds of Formulae 3 and 4 may be purified using standard techniques, including recrystallisation from a suitable solvent, and column chromatography.

In an alternative synthesis, thiosemicarbazones and thiohydrazones may be prepared from the corresponding semicarbazone and hydrazone compounds, respectfully, using methods known to those skilled in the art. For example, a C=O group may be converted into a C=S group using Lawesson's reagent under standard conditions. For example, a semicarbazone or hydrazone compound of Formula 3 or 4 may be heated at reflux with Lawesson's reagent in a suitable solvent, such as toluene or benzene, to produce the corresponding thio- compound. Preparation of compounds of Formula 4 are illustrated in the following general scheme:

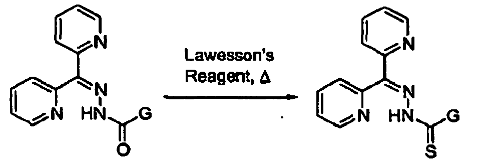

Those skilled in the art will understand that protecting groups can be employed as necessary or appropriate in the course of synthesising compounds in accordance with the invention. Suitable protecting groups are known to those skilled in the art. For example, common N-protecting groups include benzyl and carbamates, such as tert-butylcarbamate, benzylcarbamate. Protecting groups are described in Protective Groups in Organic Synthesis, Theodora W. Greene & Peter G. M. Wuts, Second Ed., John Wiley & Sons, Inc. (1991).

Examples of (thio)semicarbazone and (thio)hydrazone derivatives suitable for use in accordance with the present invention include the following: di-2-pyridylketone isonicotinoyl hydrazone ("PKIH"); di-2-pyridylketone benzoyl hydrazone ("PKBH"); di-2-pyridylketone 4-hydroxybenzoyl hydrazone ("PKHH"); di-2-pyridylketone 3-bromobenzoyl hydrazone ("PBBH"); di-2-pyridylketone 4-aminobenzoyl hydrazone ("PKAH"); di-2-pyridylketone 2-thiophenecarboxaldehyde hydrazone ("PKTH"); di-2-pyridylketone 4,4-diphenylcarboxaldehyde semicarbazone ("PK44pH"); di-2-pyridylketone octanoic hydrazone ("PKoctH"); di-2-pyridylketone 4-methyl-3-thiosemicarbazone ("Dp4mT"), di-2-pyridylketone 4,4-dimethyl-3-thiosemicarbazone ("Dp44mT"), di-2-pyridylketone 4-ethyl-3-thiosemicarbazone ("Dp4eT"); di-2-pyridylketone 4-allyl-3-thiosemicarbazone ("Dp4aT"); di-2-pyridylketone 4-phenyl-3-thiosemicarbazone ("Dp4pT").

Also disclosed herein are thiohydrazone analogues which correspond structurally to the PKIH analogues, but have a C=S instead of C=O. Such compounds include di-2-pyridylketone isonicotinoyl thiohydrazone ("PKITH"), di-2-pyridylketone benzoyl thiohydrazone ("PKBTH"), di-2-pyridylketone 4-hydroxybenzoyl thiohydrazone ("PKHTH"); di-2-pyridylketone 3-bromobenzoyl thiohydrazone ("PBBTH"); di-2-pyridylketone 4-aminobenzoyl thiohydrazone ("PKATH"), di-2-pyridylketone 2-thiophenecarboxaldehyde thiohydrazone ("PKTTH"); di-2-pyridylketone 4,4-diphenylcarboxaldehyde thiosemicarbazone ("PK44pTH"); di-2-pyridylketone octanoic thiohydrazone ("PKoctTH").

Iron complexes in accordance with the present invention include Fe[PKEH]₂, Fe[PKHH]₂, Fe[PKHH]₂, Fe[PBBH]₂, Fe[PKAH]₂, Fe[PKTH]₂, Fe[PK44pH]₂ and Fe[PKoctH]₂, Fe[Dp4mT]₂, Fe[Dp44mT]₂, Fe[Dp4eT]₂, Fe[Dp4aT]₂, and Fe[Dp4pT]₂. The iron ion may be Fe(II) or Fe(III).

Metal ion complexes of PKIH and DpT analogues of Formulae 3 and 4 in accordance with the present invention may be readily prepared using techniques and reagents well known to those skilled in the art. For example, suitable metal ion salts include, metal halides, nitrates, sulfates, perchlorates, acetates, and triflates. Suitable iron salts for forming iron complexes include, but are not limited to, FeCl₃, Fe(NO₃)₃, FeSO₄, Fe(OAC)₃, and Fe₂(ClO₄)₃.³

The presence of two organic groups (i.e, optionally substituted pyridyl groups A and B) at the 1-position of compounds of Formulae 3 and 4 generally increases the lipophilicity of the compounds in comparison to compounds without two such groups at that position. PKIH and DpT analogues of Formulae 3 and 4 may be capable of permeating cell membranes.

Structure-activity relationships of the PCIH, QCIH (Figure 1C), PKIH and DpT series of compounds, indicate that even though these classes of compound appear to have a similar metal-binding site, the lipophilicity, efficacy of metal ion chelation, and antiproliferative properties of the respective classes may differ substantially.

(Thio)semicarbazone and (thio)hydrazone compounds of Formulae 1, 2, 3 and 4 in accordance with the present invention may function as tridentate ligands capable of forming metal ion complexes. A general example of an iron complex is represented below for a compound of Formula 1. 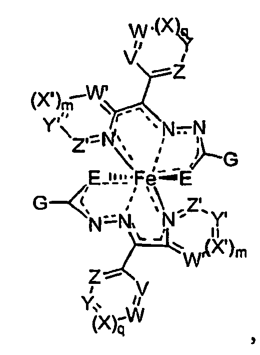 where E is O or S.

As a further example, an iron complex of the ligand PKAH, is illustrated below.

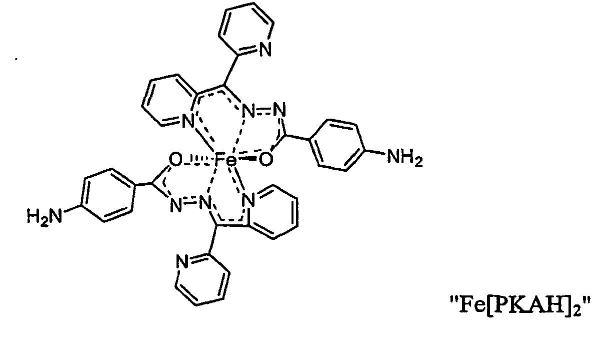

Also for example, an iron complex of the ligand Dp4mT, is illustrated below.

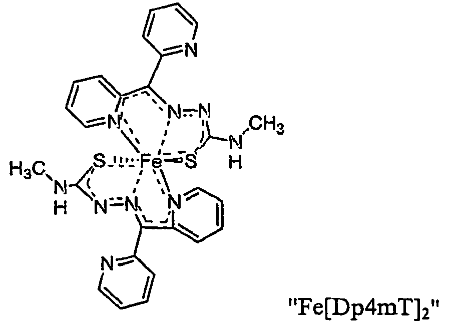

In accordance with the present invention, metal ion complexes of DpT or PKIH ligands may be neutral or charged.

Also disclosed herein is a process for identifying an anti-proliferative compound, wherein the process comprises exposing (eg, contacting, or incubating) a cell or cellular extract having a gene encoding one or more cell cycle inhibitors, with 4a compound capable of chelating iron, determining the level of expression of the gene, determining whether the level of expression of the gene differs in comparison to the expression of the gene in the absence of said compound; and thereby determining whether said compound has anti-proliferative activity.

A similar process may be used for assessing a plurality of compounds to identify one or more anti-proliferative compounds, the process comprising exposing (eg, contacting, incubating) a cell or cellular extract having gene encoding one or more cell cycle inhibitors, with a plurality of compounds capable of chelating iron, determining whether any of said compounds modify the level of expression of said gene, and if so, separately determining the level of expression of said gene for each compound, determining whether said level of expression differs in comparison to the level of expression of said gene in the absence of each compound; and thereby determining whether each compound has anti-proliferative activity.

The gene may be sensitive to cellular iron concentration. Expression of the gene may be up-regulated or down-regulated in response to Fe depletion. Examples of genes include *WAF1, GADD45,* and Ndrgl. Expression of the gene (e.g., mRNA, protein) in the absence of a compound being assessed may be separately determined prior to, or after, determining expression of the gene in the presence of the compound.

### Modification of Cellular Function

Compounds which are capable of chelating cellular iron may modify cellular function, eg, through enzyme inhibition, modification of gene expression (including upregulation and downregulation of genes), inhibition of translation (eg, of proteins, including p21), generation of Reactive Oxygen Species (ROS), etc, and may be therapeutically useful for inhibiting cellular proliferation and/or inducing apoptosis. For example, depletion of cellular iron may modify the activity of iron-dependent enzymes, such as ribonucleotide reductase. Iron deprivation can also induce G₁-S cell arrest and apoptosis.

The genes *WAF1* and *GADD45* encode cell cycle inhibitors essential for G₁-S arrest (including cyclin dependent kinase inhibitors such as p21^{CIP1/WAF1}).³⁰ Upregulation of these genes, e.g., through iron depletion, may induce cell cycle arrest. The gene *Ndrg1* (*N-myc down-regulated gene 1*) is sensitive to cellular iron concentration. Overexpression of *Ndrgl,* eg., in response to cellular iron deprivation may result in G₁-S cellular arrest Overexpression of Ndrgl has also been shown to slow tumour growth and inhibit tumour cell metastasis.⁴⁻⁷

Compounds of Formulae 3, 3a, 4 and 4a according to the present invention are capable of chelating cellular metal ions, including iron ions. Accordingly, compounds of Formulae 3, 3a, 4 and 4a may be capable of inhibiting DNA synthesis by inhibition of ribonucleotide reductase. Ribonucleotide reductase is the rate limiting step in DNA synthesis and is expressed in higher levels in neoplastic cells in comparison to normal cells. Compounds of Formulae 3, 3a, 4 and 4a may also result in overexpression of Fe-sensitive genes, such as *WAF1. GADD45* and *Ndrg1,* and therefore may be useful for inhibiting cellular proliferation. Overexpression of Fe-sensitive genes such as *WAF1, GADD45* and *Ndrg1* may occur at the mRNA level. Monitoring expression of *WAF1, GADD45* and *Ndrg1* may provide a useful indicator of the anti-proliferative potential of compounds which chelate metal ions, including Fe.

Apoptosis, or programmed cell death, plays an integral role in cellular turnover. Apoptosis can be induced in response to cellular iron depletion. Caspase enzymes are common executors of apoptosis.^{8,9} Two caspase-activating cascades that regulate apoptosis have been described, one of which is initiated via death receptors (eg., CD95), whereas the other is triggered by changes in mitochondrial integrity.^{8,10} In the former, engagement of death receptors by their ligands (eg., CD95L) leads to the formation of a death-inducing signalling complex.⁸ Activated death receptors recruit and activate caspase-8 which initiates "executioner" caspases, including caspase-3. In mitochondrial-dependent apoptosis, release of holo-cytochrome c (h-cytc) into the cytosol results in the formation of an "apoptosome" containing h-cytc, Apaf-1 and pro-caspase-9.^{8,9} In the apoptosome, pro-caspase-9 becomes activated and triggers executioner caspases that initiate terminal events overlapping with those induced by the death receptor pathway. ^{8,9} Major modulators of the mitochondrial pathway of apoptosis include the Bcl-2/Bax gene family.^{8,11,12} The Bcl-2 group of proteins are found in the outer mitochondrial membrane and play a role in its stability.^{8,11} In contrast, the Bax proteins are cytosolic molecules that upon receipt of an apoptotic signal migrate to the mitochondrion where they bind to the permeability transition pore.^{8,12} Imbalance of Bcl-2 and Bax induces the loss of selective ion permeability resulting in h-cytc release that initiates the caspase cascade responsible for apoptosis.^{8,12} Chelation of cellular metal ions, such as iron and_possibly zinc by compounds of Formulae 3 and 4 may initiate the caspase cascade and induce apoptosis.

Cellular metal ion depletion, including depletion of cellular iron, may lead to apoptosis. Apoptosis may also be induced in response to oxidative stress which can lead to release of h-cytc from the mitochondrion and an imbalance of Bcl-2/Bax expression. Metal ion complexes, including Fe-complexes of compounds of Formulae 3, 3a, 4 and 4a may be capable of inducing apoptosis through the generation of reactive oxygen species. Accordingly, compounds of Formulae 3, 3a, 4 and 4a, as well as metal ion complexes of compounds of Formulae 3, 3a, 4 and 4a (including iron complexes), may be therapeutically useful, e.g., as anti-tumour agents.

### Treatment and/or Prevention of Disease

DpT and PKIH compounds of Formulae 3, 3a, 4 and 4a disclosed herein may be useful in the treatment of various disorders and diseases of vertebrates. Examples of disorders and diseases may be grouped into broad categories, including the following: angiogenesis-dependent diseases, cellular proliferative diseases (e.g. psoriasis, IBD, malignancies, restenosis), cancer.

The cancer may be selected from the group including, but not limited to, carcinogenic tumours, tumours of epithelial origin, such as colo-rectal cancer, breast cancer, lung cancer, head and neck tumours, hepatic cancer, pancreatic cancer, ovarian cancer, gastric cancer, brain cancer, bladder cancer, prostate cancer and urinary/genital tract cancer; mesenchymal tumours, such as sarcoma; and haemopoietic tumours such as B cell lymphoma. For example, the cancer may be a haematological tumour, a solid tumour, a non-solid tumour, (e.g., leukaemia, lymphoma).

DpT and PKIH analogues of Formulae 3, 3a, 4 and 4a disclosed herein may be used in combination with other known treatments, such as surgery and/or therapeutic agents, including chemotherapeutic or radiotherapeutics. For example, when used in the treatment of solid tumours, compounds of the present invention may be administered with chemotherapeutic agents such as: adriamycin, taxol, fluorouricil, melphalan, cisplatin, alpha interferon, COMP (cyclophosphamide, vincristine, methotrexate and prednisone), etoposide, mBACOD (methotrexate, bleomycin, doxorubicin, cyclophosphamide, vincristine and dexamethasone), PROMACE/MOPP (prednisone, methotrexate (w/leucovin rescue), doxorubicin, cyclophosphamide, taxol, etoposide/mechlorethamine, vincristine, prednisone and procarbazine), vincristine, vinblastine, angioinhibins, TNP-470, pentosan polysulfate, platelet factor 4, angiostatin, LM-609, SU-101, CM-101, Techgalan, thalidomide, SP-PG and the like. Other chemotherapeutic agents include alkylating agents such as nitrogen mustards including mechloethamine, melphan, chlorambucil, cyclophosphamide and ifosfamide; nitrosoureas including carmustine, lomustine, semustine and streptozocin; alkyl sulfonates including busulfan; triazines including dacarbazine; ethyenimines including thiotepa and hexamethylmelamine; folic acid analogues including methotrexate; pyrimidine analogues including 5-fluorouracil, cytosine arabinoside; purine analogues including 6-mercaptopurine and 6-thioguanine; antitumour antibiotics including actinomycin D; the anthracyclines including doxorubicin, bleomycin, mitomycin C and methramycin; hormones and hormone antagonists including tamoxifen and cortiosteroids and miscellaneous agents including cisplatin and brequinar.

The physiological system to be treated in accordance with the present invention (e.g, the hepatic system, pancreatic system) may be isolated by or during surgery prior to administration of one or more compounds of Formulae 3, 3a, 4 and 4a.

### Pharmaceutical and/or Therapeutic Formulations

In accordance with the present invention, when used for the treatment of disease, compounds of Formulae 3, 3a, 4 and 4a may be administered alone. Alternatively, the compounds may be administered as a pharmaceutical formulation which comprises at least one compound of Formulae 3, 3a, 4 and 4a, and/or a metal ion complex thereof. Compounds of Formulae 3, 3a, 4 and 4a may also be present as pharmaceutically acceptable salts.

By pharmaceutically acceptable salt it is meant those salts which, within the scope of sound medical judgement, are suitable for use in contact with the tissues of humans and lower animals without undue toxicity, irritation, allergic response and the like, and are commensurate with a reasonable benefit/risk ratio. Pharmaceutically acceptable salts are well known in the art.

For instance, suitable pharmaceutically acceptable salts of PKIH and DpT analogues in accordance with the present invention may be prepared by mixing a pharmaceutically acceptable acid such as hydrochloric acid, sulfuric acid, methanesulfonic acid, succinic acid, fumaric acid, maleic acid, benzoic acid, phosphoric acid, acetic acid, oxalic acid, carbonic acid, tartaric acid, or citric acid with the compounds of the invention. Suitable pharmaceutically acceptable salts of the compounds of the present invention therefore include acid addition salts.

For example, S. M. Berge et al. describe pharmaceutically acceptable salts in detail in J. Pharmaceutical Sciences, 1977, 66:1-19*.* The salts can be prepared *in situ* during the final isolation and purification of the compounds of the invention, or separately by reacting the free base function with a suitable organic acid. Representative acid addition salts include acetate, adipate, alginate, ascorbate, asparate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalencsulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

Convenient modes of administration include injection (subcutaneous, intravenous, etc.), oral administration, inhalation, transdermal application, or rectal administration. Depending on the route of administration, the analogue may be coated with a material to protect the analogue from the action of enzymes, acids and other natural conditions which may inactivate the therapeutic activity of the analogue. The compound may also be administered parenterally or intraperitoneally.

Dispersions of compounds of Formulae 3, 3a, 4 and 4a may also be prepared in glycerol, liquid polyethylene glycols, and mixtures thereof and in oils. Under ordinary conditions of storage and use, pharmaceutical preparations may contain a preservative to prevent the growth of microorganisms.

Pharmaceutical compositions suitable for injection include sterile aqueous solutions (where water soluble) or dispersions and sterile powders for the extemporaneous preparation of sterile injectable solutions or dispersions. Ideally, the composition is stable under the conditions of manufacture and storage and may include a preservative to stabilise the composition against the contaminating action of microorganisms such as bacteria and fungi.

In a preferred embodiment, the compound is administered orally, for example, with an inert diluent or an assimilable edible carrier. The compound and other ingredients can also be enclosed in a hard or soft shell gelatin capsule, compressed into tablets, or incorporated directly into an individual's diet. For oral therapeutic administration, the analogue can be incorporated with excipients and used in the form of ingestible tablets, buccal tablets, troches, capsules, elixirs, suspensions, syrups, wafers, and the like. Suitably, such compositions and preparations may contain at least 1% by weight of active compound. The percentage of the PKIH or DpT analogue of Formulae 1, 1a, 2, 2a, 3, 3a, 4 and 4a in pharmaceutical compositions and preparations can, of course, be varied and, for example, can conveniently range from about 2% to about 90%, about 5% to about 80%, about 10% to about 75%, about 15% to about 65%; about 20% to about 60%, about 25% to about 50%, about 30% to about 45%, or about 35% to about 45%, of the weight of the dosage unit. The amount of analogue in therapeutically useful compositions is such that a suitable dosage will be obtained.

The language "pharmaceutically acceptable carrier" is intended to include solvents, dispersion media, coatings, anti-bacterial and anti-fungal agents, isotonic and absorption delaying agents, and the like. The use of such media and agents for pharmaceutically active substances is well known in the art. Except insofar as any conventional media or agent is incompatible with the analogue, use thereof in the therapeutic compositions and methods of treatment is contemplated. Supplementary active compounds can also be incorporated into the compositions according to the present invention. It is especially advantageous to formulate parenteral compositions in dosage unit form for ease of administration and uniformity of dosage. Dosage unit form as used herein refers to physically discrete units suited as unitary dosages for the individual to be treated; each unit containing a predetermined quantity of analogue is calculated to produce the desired therapeutic effect in association with the required pharmaceutical carrier. The specification for the novel dosage unit forms of the invention are dictated by and directly dependent on (a) the unique characteristics of the analogue and the particular therapeutic effect to be achieved, and (b) the limitations inherent in the art of compounding such an analogue for the treatment of proliferative diseases, iron-related conditions, or iron overload disorders in individuals. The principal analogue is compounded for convenient and effective administration in effective amounts with a suitable pharmaceutically acceptable carrier in an acceptable dosage unit. In the case of compositions containing supplementary active ingredients, the dosages are determined by reference to the usual dose and manner of administration of the said ingredients.

In one embodiment, the carrier is an orally administrable carrier. In another embodiment, the carrier is suitable for intravenous administration.

One suitable form of a pharmaceutical composition is a dosage form formulated as enterically coated granules, tablets or capsules suitable for oral administration.

In the case of injectable solutions, the carrier can be a solvent or dispersion medium containing, for example, water, ethanol, polyol (for example, glycerol, propylene glycol, and liquid polyetheylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by including various anti-bacterial and/or anti-fungal agents. Suitable agents are well known to those skilled in the art and include, for example, parabens, chlorobutanol, phenol, benzyl alcohol, ascorbic acid, thimerosal, and the like. In many cases, it may be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminium monostearate and gelatin.

Sterile injectable solutions can be prepared by incorporating the analogue in the required amount in an appropriate solvent with one or a combination of ingredients enumerated above, as required, followed by filtered sterilisation. Generally, dispersions are prepared by incorporating the analogue into a sterile vehicle which contains a basic dispersion medium and the required other ingredients from those enumerated above.

Tablets, troches, pills, capsules and the like can also contain the following: a binder such as gum gragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin or a flavouring agent such as peppermint, oil of wintergreen, or cherry flavouring. When the dosage unit form is a capsule, it can contain, in addition to materials of the above type, a liquid carrier. Various other materials can be present as coatings or to otherwise modify the physical form of the dosage unit. For instance, tablets, pills, or capsules can be coated with shellac, sugar or both. A syrup or elixir can contain the analogue, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, compounds of Formulae 3 and 4 may be incorporated into sustained-release preparations and formulations.

A pharmaceutical composition may further include a suitable buffer to minimise acid hydrolysis. Suitable buffer agent agents are well known to those skilled in the art and include, but are not limited to, phosphates, citrates, carbonates and mixtures thereof.

Single or multiple administrations of a pharmaceutical compositions according to the invention may be carried out One skilled in the art would be able, by routine experimentation, to determine effective, non-toxic dosage levels of the compound and/or composition of the invention and an administration pattern which would be suitable for treating the disorders or diseases to which the compounds and compositions are applicable.

Further, it will be apparent to one of ordinary skill in the art that the optimal course of treatment, such as the number of doses of the compound or composition of the invention given per day for a defined number of days, can be ascertained using conventional course of treatment determination tests.

Generally, an effective dosage per 24 hours may be in the range of about 0.0001 mg to about 1000 mg per kg body weight; suitably, about 0.001 mg to about 750 mg per kg body weight; about 0.01 mg to about 500 mg per kg body weight; about 0.1 mg to about 500 mg per kg body weight; about 0.1 mg to about 250 mg per kg body weight; or about 1.0 mg to about 250 mg per kg body weight. Still suitably, an effective dosage per 24 hours may be in the range of about 1.0 mg to about 200 mg per kg body weight; about 1.0 mg to about 100 mg per kg body weight; about 1.0 mg to about 50 mg per kg body weight; about 1.0 mg to about 25 mg per kg body weight; about 5.0 mg to about 50 mg per kg body weight; about 5.0 mg to about 20 mg per kg body weight; or about 5.0 mg to about 15 mg per kg body weight.

Alternatively, an effective dosage may be up to about 500 mg/m². Generally, an effective dosage may be in the range of about 25 to about 500 mg/m², about 25 to about 350 mg/m², about 25 to about 300 mg/m², about 25 to about 250 mg/m², about 50 to about 250 mg/m², or about 75 to about 150 mg/m².

By way of example, suitable dosage forms in accordance with the present invention include the following:

### Tablet

| | |
|---|---|
| Compound of Formula 3 and 4 | 0.01 to 20 mg, generally 0.1 to 10 mg |
| Starch | 10 to 20 mg |
| Lactose | 100 to 250 mg |
| Gelatin | 0 to 5 mg |
| Magnesium Stearate | 0 to 5 mg |

### Injectable Solution

| | |
|---|---|
| Compound of Formula 3 and 4 | 0.01 to 20 mg, generally 0.1 to 10 mg |
| Sodium Chloride | 8.5 mg |
| Potassium Chloride | 3 mg |
| Calcium Chloride | 4.8 mg |
| Water for injection, q.s. to | 10 ml |

The invention will now be described in greater detail by reference to specific examples.

### EXAMPLE 1 - Synthesis of Compounds

Comparative 2-pyridyl- and quinolyl-aroylhydrazone analogues, referred to herein as "PCIH" and "QCIH" analogues, were synthesised, respectively, by Schiff base condensation of 2-pyridylcarboxaldehyde or 2-quinolinecarboxaldehyde with an appropriate acid hydrazide.

The comparative compounds pyridoxal isonicotinoyl hydrazone (PIH) and 2-hydroxy-1-naphthylaldehyde isonicotinoyl hydrazone (311), were synthesised by the method of Richardson & Bernhardt (1999).³

Representative structures of the PCIH, QCIH aroylhydrazone series of compounds and "311" are provided in Figure 1B and 1C.

### Example 1a - Synthesis of PKIH Analogues

Representative PKIH analogues were synthesised by condensing 2-di-pyridyl ketone with an appropriate acid hydrazide according to the method of Bacchi *et al*., (1996).¹³ Suitable solvents include ethanol, methanol, ethanol/water, methanol/water, acetone, benzene, toluene. Structures of representative FKIH analogues are shown in Figure 2B.

An X-ray crystal structure of PKAH is provided in Figure 9. The X-ray crystallographic data is provided below.

### Crystal data collection:

*CAD*-4 software (Enraf-Nonius, 1989); cell refinement: *SET*4 in CAD-4 software; data reduction: *Xtal* (Hall et al., 1992); programs used to solve structure: *SHELX86* (Sheldrick, 1990); Program used to refine structure: *SHELXL97* (Sheldrick, 1997); molecular graphic: *PLATON* (Spek, 1990; software used to generate crystallographic data: *SHELXL 97.*

Data collection was obtained under same general conditions as described by D. R. Richardson, E. Becker and P. V. Bernhardt. (1999).

### Crystal Data and Details of the Structure Determination for: PKAH (C18 H16 N5 O) P-1 (2) Z = 2

### Crystal Data

| | | | | | |
|---|---|---|---|---|---|
| Empirical Formula: | C18 H16 N5 O, O | | | | |
| Formula Weight | | 334.36 | | | |
| Crystal System | | Triclinic | | | |
| Space group | P-1 | | (No. 2) | | |
| a, b, c [Angstrom] | 8.667(2) | | 9.977(2) | 11.197(2) | |
| alpha, beta, gamma [deg] | 91.010(10) | | 109.20(2) | | 109.63(2) |
| V [Ang**3] | | | 852.2(3) | | |
| Z | | 2 | | | |
| D(calc) [g/cm**3] | | | 1.303 | | |
| F(000) | | | 350 | | |
| Mu(MoKa) [/mm] | | | 0.1 | | |

Final Structure Factor Calculation for PKAH (C18 H16 N5 O) P-1 (2) Z = 2
Total number of 1.s. parameters = 240 Maximum vector length = 511 Memory required = 2380 / 22995
wR2 = 0.1767 before cycle 21 for 2985 data and 0 / 240 parameters
GooF = S = 0.987; Restrained GooF = 0.987 for 0 restraints
Weight = 1 / [sigma^2(Fo^2) + ( 0.1000 * P )^2 + 0.00 * P ] where
P=(Max(Fo^2,0)+2*Fc^2)/3
R1 = 0.0503 for 1497 Fo > 4sig(Fo) and 0.1265 for all 2985 data
wR2 = 0.1767, GooF = S = 0.987, Restrained GooF = 0.987 for all data
Occupancy sum of asymmetric unit = 25.00 for non-hydrogen and 16.00 for hydrogen atoms

### Bond lengths and angles

| C1 - | Distance | | Angles | |
|---|---|---|---|---|
| N1 | 1.3378 | (0.0036) | | |
| C2. | 1.3775 | (0.0042) | 122.49 (0.27) | |
| C6 | 1.4967 | (0.0038) | 116.20 (0.26) | 121.26 (0.27) |
| | C1 - | N1 | C2 | |
| | | | | |

| C2 - | Distance | | Angles | |
|---|---|---|---|---|
| C3 | 1.3738 | (0.0042) | | |
| C1 | 1.3775 | (0.0041) | 119.20(0.31) | |
| | C2 - | C3 | | |
| | | | | |

| C3 - | Distance | | Angles | |
|---|---|---|---|---|
| C4 | 1.3623 | (0.0049) | | |
| C2 | 1.3738 | (0.0042) | 118.91 (0.33) | |
| | C3 - | C4 | | |
| | | | | |

| C4 - | Distance | | Angles | |
|---|---|---|---|---|
| C3 | 1.3623 | (0.0049) | | |
| C5 | 1.3668 | (0.0047) | 118.81 (0.31) | |
| | C4 - | C3 | | |

| C5 - | Distance | | Angles | |
|---|---|---|---|---|
| N1 | 1.3420 | (0.0038) | | |
| C4 | 1.3668 | (0.0047) | 123.65 (0.32) | |
| | C5 - | N1 | | |
| | | | | |

| C6 - | Distance | | Angles | |
|---|---|---|---|---|
| N3 | 1.2972 | (0.0033) | | |
| C7 | 1.4777 | (0.0039) | 128.71 (0.25) | |
| C1 | 1.4967 | (0.0038) | 111.31 (0.24) | 119.98 (0.24) |
| | C6 - | N3 | C7 | |
| | | | | |

| C7 - | Distance | | Angles | |
|---|---|---|---|---|
| N2 | 1.3265 | (0.0037) | | |
| C8 | 1.3871 | (0.0040) | 121.37 (0.28) | |
| C6 | 1.4777 | (0.0039) | 117.66 (0.25) | 120.96 (0.27) |
| | C7- | N2 | C8 | |
| | | | | |

| C8 - | Distance | | Angles | |
|---|---|---|---|---|
| C9 | 1.3698 | (0.0044) | | |
| C7 | | 1.3871 (0.0040) | 119.8 (0.31) | |
| | C8 - | C9 | | |
| | | | | |

| C9 - | Distance | | Angles | |
|---|---|---|---|---|
| C10 | 1.3489 | (0.0050) | | |
| C8 | 1.3698 | (0.0044) | 119.27 (0.33) | |
| | C9 - | C10 | | |
| | | | | |

| C10 - | Distance | | Angles | |
|---|---|---|---|---|
| C9 | 1.3489 | (0.0050) | | |
| C11 | 1.3641 | (0.0052) | 117.97 (0.35) | |
| | C10 - | C9 | | |
| | | | | |

| C11 - | Distance | | Angles | |
|---|---|---|---|---|
| N2 | 1.3423 | (0.0041) | | |
| C10 | 1.3641 | (0.0052) | 124.54 (0.36) | |
| | C11 - | N2 | | |
| | | | | |

| C12 - | Distance | | Angles | |
|---|---|---|---|---|
| O1 | 1.2202 | (0.0032) | | |
| N4 | 1.3690 | (0.0034) | 120.54 (0.27) | |
| C13 | 1.4649 | (0.0039) | 122.80 (0.26) | 116.56 (0.25) |
| | C12 - | O1 | N4 | |
| | | | | |

| C13 - | Distance | | Angles | |
|---|---|---|---|---|
| C14 | 1.3913 | (0.0038) | | |
| C18 | 1.3927 | (0.0038) | 116.65 (0.26) | |
| C12 | 1.4649 | (0.0039) | 124.91 (0.25) | 118.42 (0.26) |
| | C13 - | C14 | C18 | |
| | | | | |

| C14 - | Distance | | Angles | |
|---|---|---|---|---|
| C15 | 1.3794 | (0.0039) | | |
| C13 | 1.3913 | (0.0038) | 121.52 (0.26) | |
| | C14 - | C15 | | |
| | | | | |

| C15 - | Distance | | Angles | |
|---|---|---|---|---|
| C14 | 1.3794 | (0.0039) | | |
| C16 | 1.3878 | (0.0040) | 120.80 (0.28) | |
| | C15 - | C14 | | |

| C16 - | Distance | | Angles | |
|---|---|---|---|---|
| N5 | 1.3677 | (0.0035) | | |
| C15 | 1.3878 | (0.0040) | 121.49 (0.28) | |
| C17 | 1.3939 | (0.0040) | 120.60 (0.27) | 117.90 (0.27) |
| | C16 - | N5 | C15 | |
| | | | | |

| C17 - | Distance | | Angles | |
|---|---|---|---|---|
| C18 | 1.3592 | (0.0040) | | |
| C16 | 1.3939 | (0.0040) | 120.71 (0.27) | |
| | C17 - | C18 | | |
| | | | | |

| C18 - | Distance | | Angles | |
|---|---|---|---|---|
| C17 | 1.3592 | (0.0040) | | |
| C13 | 1.3927 | (0.0038) | 122.40 (0.27) | |
| | C18 - | C17 | | |
| | | | | |

| N1 - | Distance | | Angles | |
|---|---|---|---|---|
| C1 | 1.3378 | (0.0036) | | |
| C5 | 1.3420 | (0.0038) | 116.87 (0.28) | |
| | N1 - | C1 | | |
| | | | | |

| N2 - | Distance | | Angles | |
|---|---|---|---|---|
| C7 | 1.3265 | (0.0037) | | |
| C11 | 1.3423 | (0.0042) | 117.02 (0.28) | |
| | N2 - | C7 | | |
| | | | | |

| N3 - | Distance | | Angles | |
|---|---|---|---|---|
| C6 | 1.2972 | (0.0033) | | |
| N4 | 1.3639 | (0.0030) | 119.64 (0.23) | |
| | N3 - | C6 | | |
| | | | | |

| N4 - | Distance | | Angles | |
|---|---|---|---|---|
| N3 | 1.3639 | (0.0030) | | |
| C12 | 1.3690 | (0.0034) | 118.10 (0.23) | |
| | N4- | N3 | | |
| | | | | |

| N5 - | Distance | | Angles | |
|---|---|---|---|---|
| C16 | 1.3677 | (0.0035) | | |
| | N5 - | | | |
| | | | | |

| O1 - | Distance | | Angles | |
|---|---|---|---|---|
| C12 | 1.2202 | (0.0032) | | |
| | O1- | | | |

### Final Coordinates and Equivalent Isotropic Displacement Parameters of the non-Hydrogen atoms for: PKAH (C18 H16 N5 O) P-1 (2) Z = 2

| Atom | x | y | z | U(eq) [Ang^2] |
|---|---|---|---|---|
| O1 | 1.04785 | 0.75127 | 0.33347 | 0.0701 |
| N1 | 0.49528 | 0.32443 | -0.06396 | 0.0535 |
| N2 | 0.58397 | 0.30148 | 0.32830 | 0.0725 |
| N3 | 0.80911 | 0.49927 | 0.21355 | 0.0479 |
| N4 | 0.84496 | 0.54222 | 0.33916 | 0.0501 |
| N5 | 1.03285 | 0.90464 | 0.88111 | 0.0752 |
| C1 | 0.65900 | 0.35466 | 0.01770 | 0.0462 |
| C2 | 0.79735 | 0.37032 | -0.02257 | 0.0553 |
| C3 | 0.76880 | 0.36059 | -0.15113 | 0.0668 |
| C4 | 0.60365 | 0.33400 | -0.23528 | 0.0672 |
| C5 | 0.47153 | 0.31568 | -0.18890 | 0.0628 |
| C6 | 0.68602 | 0.37702 | 0.15670 | 0.0462 |
| C7 | 0.57670 | 0.26672 | 0.21116 | 0.0511 |
| C8 | 0.47317 | 0.13076 | 0.14286 | 0.0639 |
| C9 | 0.37451 | 0.02995 | 0.19607 | 0.0772 |
| C10 | 0.38249 | 0.06372 | 0.31568 | 0.0877 |
| C11 | 0.48770 | 0.19900 | 0.37791 | 0.0969 |
| C12 | 0.96466 | 0.67714 | 0.39312 | 0.0484 |
| C13 | 0.97835 | 0.72880 | 0.52083 | 0.0444 |
| C14 | 0.88621 | 0.64856 | 0.59243 | 0.0544 |
| C15 | 0.90329 | 0.70590 | 0.71113 | 0.0582 |
| C16 | 1.01258 | 0.84689 | 0.76276 | 0.0541 |
| C17 | 1.10477 | 0.92790 | 0.69123 | 0.0615 |
| C18 | 1.08799 | 0.86970 | 0.57489 | 0.0577 |
| O2 | 0.85377 | 0.76504 | 0.05842 | 0.0756 |

| | | | | |
|---|---|---|---|---|
| U(eq) = 1/3 of the trace of the orthogonalized U Tensor | | | | |

### Example 1b - Preparation of DpT Analogues

DpT analogues were synthesised by Schiff base condensation of 2-di-pyridylketone and the respective thiosemicarbazides or acid hydrazides using standard procedures (Johnson *et al*., 1982).¹⁴ Suitable solvents for carrying out the reaction include ethanol, methanol, ethanol/water, methanol/water, acetone, benzene, toluene.

Structures of representative DpT analogues are shown in Figure 2A.

### Example 1c - Preparation of Iron Complexes

Iron complexes of PKIH analogues were prepared using techniques described by Richardson & Bernhardt, 1999.³ Generally, 1 mole equivalent of an Fe(III) salt was refluxed in hot ethanol with 2 mole equivalents of a PKIH analogue for 1 hour. A dark green/black complex solid formed and was isolated from the mother liquor by filtration under reduced pressure. The complex was then washed with ethanol and air dried.

Iron complexes of DpT analogues were prepared in an analogous manner.

### EXAMPLE 2 - Comparison of Lipophilicity and Activity of PCTH, QCIH and PKIH Analogues

Lipophilicity was measured as the average log P values (*n*-octaliol-water partition coefficients) for all chelators. Log P values were estimated (Table 1) according to Broto's method (Broto *et al*., 1984),¹⁵ Crippen's fragmentation procedure (Ghose & Crippen, 1987),¹⁶ or Viswanadhan's fragmentation procedure (Viswanadhan *et al*., 1987)¹⁷ using Chem Draw Pro. (v. 4.5, Cambridge Software, 1997). The means of these values (average calculated log P values; Table 1) were then plotted against either the IC₅₀ values, or the ability of chelators to induce ⁵⁹Fe mobilisation or prevent ⁵⁹Fe uptake. These plots demonstrate no strong correlation between lipophilicity and anti-proliferative activity or Fe chelation efficacy of the compounds tested (all correlations: r < 0.52) (data not shown).

**Table 1: Calculated n-octanol partition coefficients (log P_{calc})**

| | Crippens' Fragmentation | Viswanadhans' Fragmentation | Brotos' Method | Average log P_{calc} |
|---|---|---|---|---|
| PIH | 0.43 | 0.63 | -0.16 | 0.30 |
| 311 | 2.63 | 2.83 | 2.17 | 2.54 |
| | | | | |
| PCIH | 1.11 | 1.20 | 0.56 | 0.96 |
| PCTH | 2.43 | 2.54 | * | ** |
| PCBH | 2.45 | 2.51 | 1.68 | 2.21 |
| PCBBH | 3.28 | 3.30 | 2.56 | 3.05 |
| PCAH | 1.65 | 1.73 | 0.86 | 1.41 |
| PCHH | 2.06 | 2.23 | 1.29 | 1.86 |
| | | | | |
| PKIH | 1.66 | 1.83 | 0.93 | 1.47 |
| PKTH | 2.98 | 3.17 | * | ** |
| PKBH | 3.00 | 3.15 | 2.05 | 2.73 |
| PKBBH | 3.83 | 3.94 | 2.93 | 3.57 |
| PKAH | 2.20 | 2.36 | 1.23 | 1.93 |
| PKHH | 2.61 | 2.86 | 1.66 | 2.38 |
| | | | | |
| QCIH | 2.11 | 2.20 | 1.68 | 2.00 |
| QCTH | 3.43 | 3.54 | * | ** |
| QCBH | 3.45 | 3.52 | 2.80 | 3.26 |
| QCBBH | 4.28 | 4.31 | 3.68 | 4.09 |
| QCAH | 2.65 | 2.73 | 1.98 | 2.45 |
| QCHH | 3.06 | 3.23 | 2.41 | 2.90 |

| | | | | |
|---|---|---|---|---|
| * Unable to be calculated by Broto's method ** Unable to calculate Log P_{calc} values were calculated using the program Chem Draw Pro V 4.5. | | | | |

The comparative QCIH analogues were the most lipophilic of the compounds studied due to the presence of the single quinoline ring (Table 1). However, the iron chelation efficacy and anti-proliferative activity of the QCIH series is very low (see Examples 3 a, 3b and 4a).

### In Vitro and In Vivo STUDIES

### General Methodology for Examples 3-10

DFO was purchased from Novartis (Basel, Switzerland).

311 was prepared according to standard methods³.

Triapine® (3-aminopyridine-2-carboxaldehyde thiosemicarbazone; Figure 1B) was a gift from Vion Pharmaceuticals, NJ, USA.

### Presentation of Compounds

All compounds were dissolved in dimethyl sulfoxide (DMSO) as 10 mM stock solutions immediately prior to an experiment and then diluted in 10% fetal calf serum (FCS; Commonwealth Serum Laboratories, Melbourne, Australia) so that the final DMSO concentration was equal to or less than 0.5 % (v/v).

### Cell Culture Preparation

The human SK-N-MC neuroepithelioma cell line, SK-Mel-28 melanoma cells, and MCF-7 breast cancer cells were purchased from the American Type Culture Collection (ATCC), Rockville, MD, USA. The normal cell types Mac-5 fibroblasts and L8 rat skeletal muscle cells were also obtained from the American Type Culture Collection (ATCC), Rockville, MD, USA. The cells were grown in Eagle's modified minimum essential medium (MEM; Gibco BRL, Sydney, Australia) containing 10% FCS (Commonwealth Serum Laboratories, Melbourne, Australia), 1% (v/v) non-essential amino acids (Gibco), 2 mM L-glutamine (Sigma Chemical Co., St. Louis, MO, USA), 100 µg/ml of streptomycin (Gibco), 100 U/ml penicillin (Gibco), and 0.28 µg/ml of fungizone (Squibb Pharmaceuticals, Montreal, Canada). This growth medium is subsequently referred to as "complete medium". Cells were grown in an incubator (Forma Scientific, OH, USA) at 37°C in a humidified atmosphere of 5% CO₂/95% air and subcultured. Cellular growth and viability were carefully assessed using phase-contrast microscopy and trypan blue staining.

### MTT cellular proliferation assay

Cellular proliferation was examined using the MTT (3-(4,5-dimethylthiazol-2-yl)-2,5-diphenyl tetrazolium) assay by essentially the same method as described previously.¹⁸ Cells were seeded in 96-well microtitre plates at 15,000 cells/well in 0.1 mL of MEM medium containing all supplements as described in section 2.2 above (complete medium) and also 1.25 µM human diferric Tf. This seeding density resulted in exponential growth of the cells for the duration of the assay. The cells were grown overnight and the compounds to be tested were then added in 0.1 mL of complete medium containing 1.25 µM diferric transferrin. The final concentration of the compounds was 0.39-50 µM. Control samples contained complete medium and 1.25 µM diferric Tf. Cells were incubated with the compounds for 90 h at 37°C in a humidified atmosphere containing 95% air and 5% CO₂. After this incubation, 0.01 mL of MTT was added to each well and the plates incubated for 2 h at 37°C. The cells were then solubilised by adding 0.1 mL of 10% SDS-50% isobutanol in 0.01 M HCl and the plates then read at 570 nm on a scanning multiwell spectrophotometer (Titertek Multiscan; Beckman Instruments Inc, California). MTT colour formation was directly proportional to the number of viable cells. The results of the MTT assays are expressed as a percentage of the control value.

### Statistics

Experimental data were compared using Student's paired *t*-test. Results were considered statistically significant when p < 0.05. Results are presented as the mean or mean ± standard deviation (SD) of 2-5 separate experiments.

### EXAMPLE 3 - Iron Mobilisation/Uptake Experiments

### Labelling of Transferrin with ⁵⁹Fe

Apotransferrin (Sigma Chemical Co., St. Louis, USA) was prepared and labelled with ⁵⁹Fe (as ferric chloride in 0.1 M HCl, Dupont NEN, MA, USA) to produce ⁵⁹Fetransferrin (⁵⁹Fe-Tf) using standard procedures described by Richardson & Baker, 1992.

### Example 3a- Comparison of the Effect of PCIH, QCIH and PKIH Analogues on Iron Mobilization from Prelabelled SK-N-MC Neuroepithelioma Cells

### Methodology: Iron Mobilization Assay

The effect of chelators on ⁵⁹Fe release from prelabelled cells was studied using standard procedures.¹⁸ Cells were prelabelled with ⁵⁹Fe-transferrin (0.75 µM) for 3 h at 37°C. The cells were then placed on ice and washed 4 times with ice-cold BSS, and then reincubated for 3 h at 37°C in the presence of medium alone (control) or medium containing DFO (100 µM) or the other compounds (50 µM). The overlying medium was then removed and placed into γ-counting tubes. The cells were removed from the petri dishes in 1 ml of BSS using a plastic spatula and transferred to a separate set of γ-counting tubes.

The Fe chelation efficacy of the QCIH and PKIH analogues was examined by comparing their activity to DFO and the corresponding PCIH analogue. The ability of the compounds to mobilise ⁵⁹Fe from SK-N-MC neuroepithelioma cells was assessed after a 3 h labelling period with ⁵⁹Fe-Tf (0.75 µM) at 37°C. The cells were then washed and reincubated for 3 h at 37°C in the presence and absence of the internal standard DFO (100 µM) or the other chelators at 50 µM (Fig. 3 and Fig. 4). DFO was used at a concentration of 100 µM due to the low efficacy of this chelator at mobilising ⁵⁹Fe from cells and preventing ⁵⁹Fe uptake from ⁵⁹Fe-Tf.

In general, the PKIH series showed similar or greater ability to mobilise ⁵⁹Fe from prelabelled cells, than the corresponding PCIH control compound (Fig. 3A). The greatest difference in activity between the PKIH and PCIH groups was found when comparing the more hydrophilic compounds from these series (see Table 1), i.e., PCAH and PKAH or PCHH and PKHH.

In general, the comparative QCIH analogues did not behave in a similar manner to the PCIH or PKIH analogues and showed little activity in terms of inducing ⁵⁹Fe mobilization (Fig. 3B). The most effective chelator of the QCIH series was the parent compound QCIH, which exhibited comparable cellular ⁵⁹Fe mobilisation to PCIH (Fig. 3B).

Analysis of the ⁵⁹Fe mobilisation data for these three groups of compounds suggested that the dipyridyl substituent of the PKIH series appears to confer on the chelators high Fe chelation activity irrespective of the hydrazide substitution (Fig. 3A). In contrast, the presence of a single quinoline ring in the comparative QCIH series of compounds appears to result in low Fe chelation activity (Fig. 3B).

### Example 3b - Comparison of the Effect of the PCIH, QCIH and PKIH Chelators on Cellular Iron Uptake from ⁵⁹Fe-labelled Transferrin

### Methodology: Iron Uptake Assay

The ability of PKIH analogues to inhibit cellular uptake of ⁵⁹Fe uptake from ⁵⁹Fe-Tf was studied using standard procedures.¹⁸ SK-N-MC Cells were incubated for 3 h at 37°C in media containing ⁵⁹Fe-Tf (0.75 µM) and either DFO (100 µM) or a compound of the of the PCIH, QCIH or PKIH series (50 µM). The cells were then placed on ice and washed four times with ice-cold Hank's balanced salt solution (BSS) to remove non-specifically bound ⁵⁹Fe-Tf. Cells were then incubated with the general protease, pronase (1 mg/ml), for 30 min at 4°C to remove membrane-bound ⁵⁹Fe and Tf.

All PKIH analogues were very effective at inhibiting ⁵⁹Fe uptake from ⁵⁹Fe-Tf (Fig. 4A). In particular, the PKAH and PKHH ligands showed marked activity while the corresponding control PCIH analogues, PCAH and PCHH, showed little effect (Fig. 4A). In general, the QCIH ligands were less effective than the PCIH and PKIH analogues at preventing ⁵⁹Fe uptake from ⁵⁹Fe-Tf (Fig. 4B).

### Example 3c - Effect of PKIH Analogue Concentration on Cellular Iron Mobilization and Iron Uptake from Transferrin

### Methodology: Iron Mobilisation Assay

The efficacy of PKIH analogues on ⁵⁹Fe mobilisation was assessed as a function of compound concentration (Fig. 5A). Cells were prelabelled with ⁵⁹Fe-Tf (0.75 µM) for 3 h at 37°C, washed, and then reincubated for 3 h at 37°C with a PKIH compound at a range of concentrations (0.5-50 µM).

Similarly, the effect of chelator concentration on ⁵⁹Fe uptake from ⁵⁹Fe-Tf was assessed by incubating the PKIH analogues (0.5-50 µM) with ⁵⁹Fe-Tf (0.75 µM) for 3 h at 37°C and the cells then washed (Fig 5B). PCTH was used as an internal control.

PKIH, PKTH, and PKBH, were more effective than PCTH at mobilising cellular ⁵⁹Fe at concentrations less than 10 µM (Fig. 5A). For instance, at a concentration of 1 µM, PKTH released 26% of cellular ⁵⁹Fe, whereas PCTH only released 10% (Fig. 5A). Similarly, PKIH, PKBH and PKTH, had higher efficacy than the control PCTH at preventing ⁵⁹Fe uptake from ⁵⁹Fe-Tf (Fig. 5B). The more hydrophilic ligands, PKAH and PKHH (Table 1), exhibited lower efficacy in the ⁵⁹Fe uptake and ⁵⁹Fe mobilisation assays.

### Example 3d - The Effect of DpT Analogues on Iron Mobilisation from Prelabelled SK-N-MC Neuroepithelioma Cells

### Methodology: Iron Mobilisation Assay

Apotransferrin (Sigma) was labelled with ⁵⁹Fe (Dupont-NEN, MA, USA) to produce ⁵⁹Fetransferrin (⁵⁹Fe-Tf) using standard procedures. ¹⁸ Cells were prelabelled for 3 h at 37°C with ⁵⁹Fe-Tf (0.75 µM), washed, and then reincubated for 3 h at 37°C with the chelators (25 µM; Fig. 11A). DFO and 311 were used as internal standards.

The ability of the DpT analogues to directly mobilise ⁵⁹Fe from SK-N-MC cells was examined (Fig. 11A). DpT and Dp2mT resulted in the efflux of 6-7% of total cellular ⁵⁹Fe, while control cells reincubated with media alone released 5% of ⁵⁹Fe (Fig. 11A). The compound Dp2mT acts as a negative control as the presence of the methyl group at the 2-position of the thiosemicarbazone hinders iron chelation. DFO only resulted in the mobilisation of 11% of cellular ⁵⁹Fe (Fig. 11A). The other DpT compounds studied were significantly (p < 0.0001) more effective than DFO at mobilising ⁵⁹Fe, and resulted in the release of 37-47% of cellular ⁵⁹Fe (Fig. 11). Dp44mT was the most efficient, resulting in the release of 47% of ⁵⁹Fe (Fig. 11). The efficacy of the DpT analogues at inducing ⁵⁹Fe efflux correlated well to their anti-tumour activity (Example 4d, Table 3).

### Example 3e - The Effect of DpT Analogues on Cellular Iron Uptake from ⁵⁹Fe-Labelled Transferrin by SK-N-MC Neuroepithelioma Cells

### Methodology: Iron Uptake Assay

The ability of DpT analogues to inhibit cellular uptake of ⁵⁹Fe from ⁵⁹Fe-Tf was studied using standard procedures.¹⁸ The amount of ⁵⁹Fe internalised by the cells was measured by incubation with the general protease pronase (1 mg/ml) for 30 min at 4°C to remove membrane-bound ⁵⁹Fe and Tf.

The ability of DpT analogues to inhibit ⁵⁹Fe uptake from ⁵⁹Fe-Tf (0.75 µM) by SK-N-MC cells was examined in the absence or presence of a range of DpT analogues (25 µM) for 3 h at 37°C (Fig. 11B). In agreement with their low ability to mobilise ⁵⁹Fe, DpT and D2mT, had little effect at preventing ⁵⁹Fe uptake. The most efficient compounds examined, including 311 and Dp44mT, limited ⁵⁹Fe uptake to 5-9% of the control, their activity being significantly greater (p < 0.0001) than DFO (Fig. 11B). These results agree with the ability of these ligands to mobilise cellular ⁵⁹Fe (Fig. 11A). All DpT analogues tested (except DpT and Dp2mT) showed much greater efficacy than DFO in preventing ⁵⁹Fe uptake from ⁵⁹Fe-Tf by cells (Fig. 11B).

The ability of DpT analogues to inhibit tumour cell growth (Table 3) was shown to correlated with their efficacy to induce ⁵⁹Fe release (Fig. 11A) and prevent ⁵⁹Fe uptake (Fig. 11B). The Fe chelation activity of Dp44mT, Dp4mT, Dp4eT, Dp4aT and Dp4pT was much greater than that mediated by DFO.

### Example 3f - The Effects of Dp44mT on Iron Uptake from Tf and Iron Mobilisation from M109 Cells

The ability of the compound Dp44mT to inhibit the growth of M109 lung carcinoma cells in mice was assessed. Studies were performed using M109 carcinoma cells in culture to determine the effect of Dp44mT on mobilising ⁵⁹Fe from these cells (Fig. 11C) and inhibiting ⁵⁹Fe uptake from ⁵⁹Fe-Tf (Fig 11D). As internal controls, cells were incubated with DFO and 311. The effect of compound concentration on increasing ⁵⁹Fe mobilisation from M109 cells was examined by labelling cells with ⁵⁹Fe-Tf (0.75 µM) for 3 h at 37°C followed by washing and reincubation with the respective compounds (0.2-25 µM) for 3 h at 37°C (Fig. 11C). DFO was the least effective compound examined, and at 25 µM released only 12 ± 1% of ⁵⁹Fe from cells, whereas control medium released 8 ± 1% (Fig. 11C). In contrast, 311 increased cellular ⁵⁹Fe release from 8 ± 1% (control medium) to 60 ± 1% at 5 µM (Fig 11C). For Dp44mT ⁵⁹Fe release plateaued at 2 µM, resulting in the efflux of 48 ± 2 % of ⁵⁹Fe (Fig 11C).

The compound 311 was the most active compound studied at inhibiting cellular ⁵⁹Fe uptake from ⁵⁹Fe-Tf (0.75 µM) by M109 cells (Fig. 11D). At 1 µM, 311 reduced ⁵⁹Fe uptake to 40 ± 4% of the control. In contrast, DFO had little effect. Dp44mT had similar activity to 311 up to a concentration of 5 µM, where it reduced ⁵⁹Fe uptake to 30 ± 1% of the control (Fig. 11D).

### EXAMPLE 4 - Inhibition of Cellular Proliferation In Vitro

### Example 4a - Effect of PKIH Analogues on the Proliferation of SK-N-MC Neuroepithelioma Cells

### Methodology: Cellular Proliferation Assay

The effect of compounds of the PCIH, QCIH and PKIH series on the proliferation of the SK-N-MC neuroepithelioma cell line (Table 2) was studied. The effect of the compounds on the proliferation of SK-N-MC neuroepithelioma cells was examined using the MTT assay (described above). MTT colour formation was directly proportional to the number of viable cells measured by Trypan blue staining. In all experiments, the compounds 311 and DFO were used as relevant internal controls.

**Table 2: The effect of compounds of the PCIH, QCIH and PKIH series of Fe chelators on the proliferation of SK-N-MC neuroepithelioma cells using the MTT assay.**

| **IC₅₀ Values of Chelators** (µ**M**) | | | | | |
|---|---|---|---|---|---|
| **PCIH Analogues** | | **QCIH Analogues** | | **PKIH Analogues** | |
| DFO | >50 | DFO | >50 | DFO | >50 |
| 311 | 3±2 | 311 | 3±2 | 311 | 3±2 |
| PCTH | >50 | QCIH | 49±3 | PKIH | 2±1 |
| PCTH | 48±4 | QCTH | >50 | PKTH | 3±1 |
| PCBH | 46±7 | QCBH | 50±1 | PKBH | 3±1 |
| PCBBH | 40±12 | QCBBH | 49±3 | PKBBH | 1±1 |
| PCAH | >50 | QCAH | >50 | PKAH | 42±9 |
| PCHH | >50 | QCHH | >50 | PKHH | 38±10 |

| | | | | | |
|---|---|---|---|---|---|
| Data is expressed as Mean ± SD from 4 experiments. | | | | | |

Cytotoxicity of the PKIH analogues was apparent from the morphological appearance of the cells and the marked decrease in cell numbers. The PKIH analogues showed very high anti-proliferative activity even at very low chelator concentrations (Table 2). The compounds, PKIH, PKTH, PKBH and PKBBH demonstrated very similar anti-proliferative activity to 311 (Table 2).

In contrast to the PKIH analogues, the comparative PCIH and QCIH analogues had little effect on cellular proliferation (Table 2). All compounds of the PCIH and QCIH series had IC₅₀ values of 40 µM to greater than 50 µM. These results were in strong agreement with examination of cell morphology by phase contrast microscopy and Trypan blue staining which demonstrated the PCIH and QCIH series of compounds showed little cytotoxic activity.

### Example 4b - Effect of Fe(III) Complexes of PKIH Analogues on the Proliferation of SK-N-MC Neuroepithelioma Cells

Experiments were performed to determine whether the PKIH analogues were effective at inhibiting SK-N-MC cellular proliferation when complexed with Fe(III). At a chelator to Fe molar ratio of 1:1 and 2:1, all of the PKIH-Fe complexes showed a similar level of anti-proliferative activity as that found for the free ligands (data not shown). As a relevant control in the same studies, the 1:1 and 2:1 compound to Fe complexes of 311 were also prepared and assessed. The Fe complexes of 311 had no significant effect on proliferation at concentrations up to 50 µM (data not shown).

### Example 4c - Comparison of the Effect of PKIH Analogues on Proliferation of Fibroblasts and SK-N-MC Neuroepithelioma Cells

The effect of PKIH, PKTH, PKBH and PKBBH on the growth of SK-N-MC neuroepithelioma cells and MRC-5 fibroblasts was studied (Fig. 6). The MRC-5 cell type is a normal human lung fibroblast cell strain that is mortal and senesces after a number of passages *in vitro.* The SK-N-MC neuroepithelioma cell type is immortal. The effect of the PKIH analogues at inhibiting proliferation was increased in SK-N-MC cells than fibroblasts. For instance, while the PKIH analogues had IC₅₀ values of 1-3 µM in SK-N-MC cells (Table 2), the IC₅₀ values in fibroblasts for PKBBH, PKBH, PKTH and PKIH were 8, 11, 16 and >25 µM respectively.

### Example 4d - Effect of DpT Analogues on Proliferation of SK-N-MC Neuroepithelioma Cells

The ability of DpT analogues (Fig. 2A) to inhibit proliferation of SK-N-MC neuroepithelioma cells was assessed (Fig 10). Compounds (0-25 µM) were incubated with cells for 72 h and at the end of this incubation period, cell density was determined by the MTT assay, as described above. Dp2mT served as a negative control. Data obtained using the MTT assay directly correlated with cell numbers estimated via light microscopy.

Dp44mT, Dp4eT, Dp4aT and Dp4pT showed very high anti-proliferative activity (IC₅₀ = 0.03-0.06 µM; Table 3). These DpT analogues were significantly (p < 0.0001) more active than DFO (IC₅₀ = 5 µM) and have greater efficacy than other Fe chelators such as 311 (IC₅₀ = 0.3 µM; Table 3). Compared to the anti-proliferative activity of the established cytotoxic agent, doxorubicin, in SK-N-MC cells (IC₅₀ = 0.02 µM; Table 3), Dp44mT, Dp4eT, Dp4aT and Dp4pT have similar efficacy. The iron chelator, Triapine^{®} (Fig. 1B) had less activity (IC₅₀ = 0.26 µM; Table 3) than the above DpT analogues. Dp4mT also demonstrated appreciable activity against SK-N-MC cells and was comparable to Triapine^{®}, having an IC₅₀ value of 0.19 µM (Table 3).

**Table 3: The effect of Fe-chelators on cellular proliferation of neoplastic and normal cells**

| **IC₅₀ (**µ**M)** | | | | |
|---|---|---|---|---|
| **Neoplastic Cells** | | | | **Normal Cells** |
| | SK-N-MC neuroepithelioma | SK-Mel-28 melanoma | MCF-7 breast cancer | MRC-5 fibroblasts |
| DFO | 5 ± 2 | 15 ± 7 | 14 ± 9 | >25 |
| 311 | 0.30 ± 0.2 | 0.9 ± 0.5 | * | >25 |
| 311m | 0.80 ± 0.2 | 1.4 ± 0.4 | 0.9 ± 0.4 | >25 |
| | | | | |
| Dp311 | 0.6 ± 0.04 | 1.1 ± 0.5 | 1.6 ± 0.1 | >25 |
| DpT | >25 | >25 | >25 | >25 |
| Dp2mT | >25 | >25 | >25 | >25 |
| Dp4mT | 0.19 ± 0.1 | 0.6 ± 0.5 | 0.3 ± 0.2 | >25 |
| Dp44mT | 0.03 ± 0.01 | 0.06 ± 0.03 | 0.06 ± 0.01 | >25 |
| Dp4eT | 0.06 ± 0.01 | 0.09 ± 0.06 | 0.08 ± 0.01 | >25 |
| Dp4aT | 0.06 ± 0.01 | 0.10 ± 0.06 | 0.07 ± 0.01 | >25 |
| Dp4pT | 0.05 ± 0.006 | 0.09 ± 0.05 | 0.07 ± 0.01 | >25 |
| | | | | |
| Triapine | 0.26 ± 0.01 | 2.6 ± 0.6 | 3.0 ± 1.5 | * |
| Dox | 0.02 ± 0.01 | 0.35 ± 0.09 | 0.6 ± 0.2 | * |

| | | | | |
|---|---|---|---|---|
| Results are mean ± SD (at least 5 experiments). * Data not available | | | | |

DpT analogues were also assessed against the SK-Mel-28 melanoma and MCF-7 breast cancer cell lines (Table 3). Dp2mT served as a negative control as the methyl group at the 2-position hinders Fe-chelation. The compounds Dp4mT, Dp44mT, Dp4eT, Dp4aT and Dp4pT were each more active (p < 0.0001) than DFO at inhibiting proliferation of SK-Mel-28 melanoma and MCF-7 breast cancer cells (Table 3). Against these cell lines, Dp44mT, Dp4eT, Dp4aT and Dp4pT were the most active (IC₅₀ = 0.06-0.10 µM; Table 3), Dp44mT being the most effective anti-proliferative agent when assessing all neoplastic cell types. Overall, the efficacy of the active DpT analogues was greater than that of 311 and Triapine^{®}, and better than that found for doxorubicin (Table 3). Significantly, in contrast to the marked anti-proliferative activity of Dp44mT, Dp4eT, Dp4aT and Dp4pT against immortal neoplastic cells, those compounds showed surprisingly little effect on the proliferation of mortal MRC-5 fibroblasts (IC₅₀ values >25 µM) (Fig. 10 and Table 3).

### Example 4e - Comparison of Inhibition of Proliferation for a Range of Cell Types

The compounds 311, DFO, DOX, PKIH, PKTH, Dp44mT, Dp4eT and Dp4pT (0-50 µM) were incubated with cells for 72 h. At the end of the incubation period, cell density was determined by the MTT assay (described above). Results are shown in Table 4.

**Table 4 - The effect of Fe-chelators on cellular proliferation of a range of neoplastic cells, including, H69AR lung cancer, IMR-32 Neuroblastoma, HEP G2 Hepatoma, DU 145 prostate cancer, and NCI H2452 mesothelioma cell lines.**

| **IC₅₀ (**µ**M)** | | | | | |
|---|---|---|---|---|---|
| | H69AR^ | IMR-32^ | HEP G2^ | DU 145* | NCI H2452^{#} |
| | Lung Cancer | Neuroblastoma | Hepatoma | Prostate Cancer | Mesothelioma |
| 311 | 0.97 | 0.30 | 1.18 | 1.94 | 0.47 |
| DFO | >50 | 9.38 | 18.7 | 15.75 | 32.3 |
| DOX | 0.39 | 0.01 | 2.61 | 0.09 | 0.17 |
| PKTH | -- | 0.38 | 1.41 | 0.45 | 0.86 |
| PKTH | 0.78 | 0.30 | 1.17 | 1.43 | 0.73 |
| Dp44mT | 0.05 | 0.01 | 1.36 | 0.06 | 0.01 |
| Dp4eT | -- | 0.01 | 0.02 | -- | 1.17 |
| Dp4pT | 0.97 | 0.01 | 0.08 | 0.53 | 0.01 |

| | | | | | |
|---|---|---|---|---|---|
| ^ 1 experiment; * mean ± SD of 2 experiments; # mean ± SD of 3 experiments | | | | | |

The anti-proliferative activity of the more effective compounds on a range of tumour cell lines derived from a number of very aggressive cancers was assessed. In these experiments, DFO and 311 were used as appropriate standards. In addition, these studies included the cytotoxic agent, doxorubicin (DOX), which is one of the most effective anti-tumour agents in current clinical use. The least effective chelator examined was the internal standard, DFO, which is the current drug in clinical use for the treatment of iron overload disease. The most effective compounds examined included the 3 DpT analogues, namely, Dp44mT, Dp4eT and Dp4pT. Notably, the activity of these DpT analogues in some cancer cell lines was more pronounced than that of DOX. For instance, in the hepatoma cell line, HepG2, the IC₅₀ of Dp4eT was 0.02 µM which is over 10-fold less than that found for DOX (2.61 µM). The activity of the PKIH analogues, PKIH and PKTH, was also impressive, being similar to 311 and slightly less effective than DOX. Importantly, the DpT and PKIH analogues work by a very different mechanism of action to DOX, and may possibly be useful in cancer cell types resistant to DOX.

### EXAMPLE 5 - In Vivo Dose-Dependent Inhibition of the Growth of the M109 Lung Carcinoma

### General Methodology

### Determination of the Maximum Tolerated Dose in CD2F1 Hybrid Mice.

BALB/c and DBA/2 mice were obtained from Animal Resources Centre (University of New South Wales (UNSW), Sydney, Australia) and crossed to produce CD2F1 animals (all procedures used were approved by the Animal Ethics Committee, UNSW). Groups of eight female CD2F1 mice (8-10 weeks old) were fed and watered *adlibitum,* and injected via the tail vein with compounds being studied in 30% propylene glycol in 0.9% sterile saline, twice a day, 6 h apart, 5 days/week for 2 weeks. The maximum tolerated dose (MTD) was defined as the dose at which 30% of a cohort either died or lost weight in excess of 10%.¹⁹

### Inhibition of M109 Lung Cancer Cell Growth by Iron Chelators In Vino

Female CD2F1 mice aged 8-10 weeks old were sc implanted with 1 x 10⁵ M109 cells. The tumour cells were passaged in CD2F1 mice by preparing a brei from the tumour and injecting it sc.²⁰ Dp44mT was dissolved in 30 % (v/v) propylene glycol in saline and injected iv 4 days after tumour engraftment, twice daily for 5 days followed by a "rest period" of 2 days when the animal was not injected. On the twelfth day after tumour implantation, mice were sacrificed using methoxyfluorane and cardiac puncture performed. Blood counts and erythrocyte indices were measured using a Sysmex Blood Counter (Prince of Wales Hospital, Sydney, Australia). Mouse body weight changes during the treatment period were recorded. The tumours were weighed and fixed for histological examination. The experimental groups consisted of eight mice for the control and each treatment (Table 5).

Figure 12A shows the effects of 0.15, 0.3, and 0.4 mg/kg Dp44mT (MTD = 0.4 mg/kg) on tumour growth after iv injection twice daily for 5 consecutive days from day 4. As a relevant positive control, animals were administered Triapine^{®} at its MTD (6 mg/kg). The tumour weight was measured on the twelfth day after the tumour cell implantation.

**Table 5. Mouse weight loss and haematological indices from animals treated with vehicle alone (control), Dp44mT (0.15,0.3 and 0.4 mg/kg), or Triapine® (6 mg/kg).**

| | Control | °Dp44mT (mg/kg) | | | Triapine® (6 mg/kg) |
|---|---|---|---|---|---|
| | | 0.15 | 0.3 | 0.4 | |
| Body weight loss (g) | 0.83 ± 0.3 | 1.4 ± 0.2 | 1.3 ± 0.3 | 1.1 ± 0.2 | 5.3 ± 0.4 |
| WBC (10⁹/L) | 4.4 ± 0.4 | 4.2 ± 0.8 | 4.2 ± 0.4 | 3.9 ± 0.6 | 1.4 ± 0.3 |
| RBC (10¹²/L) | 9.1 ± 0.2 | 9.4 ± 0.1 | 8.9 ± 0.4 | 8.0 ± 0.6 | 6.1 ± 0.4 |
| Haemoglobin (g/dL) | 13.7 ± 0.2 | 14.0 ± 0.2 | 13.3 ± 0.5 | 11.8 ± 0.8 | 8.6 ± 0.5 |
| Haematocrit | 0.46 ± 0.01 | 0.46 ± 0.01 | 0.40 ± 0.03 | 0.40 ± 0.01 | 0.28 ± 0.01 |
| Platelets (10⁹/L) | 788 ± 47 | 1047 ± 42 | 1019 ± 87 | 636 ± 88 | 976 ± 171 |

| | | | | | |
|---|---|---|---|---|---|
| Results are Mean ± SEM (3 separate experiments). The experimental groups consisted of eight mice for the control and each treatment. | | | | | |

Administration of Dp44mT inhibited tumour growth in a dose-dependent manner (Fig. 12A) and at 0.4 mg/kg reduced tumour weight to 47 % (p < 0.01) of control values. There was no significant difference in mouse body weight loss or leukocyte cell count in the control group compared to animals treated with Dp44mT (Table 5). However, compared to the control, a slight (p < 0.05) decrease in haemoglobin, hematocrit, erythrocyte and platelet counts were observed in animals treated with Dp44mT at its MTD (0.4 mg/kg) but not at lower doses (Table 5). Administration of Dp44mT at 0.15 and 0.3 mg/kg increased platelet counts (Table 5). Triapine^{®} was more effective than Dp44mT, significantly (p < 0.001) reducing tumour weight to 10% of the control (Fig. 12A). However, in contrast to Dp44mT, Triapine^{®} at its MTD (6 mg/kg) markedly and significantly (p < 0.0001) decreased animal weight, haemoglobin concentration, hematocrit, erythrocyte and leukocyte cell counts (Table 5), suggesting that under these conditions Triapine® was systemically toxic.

### EXAMPLE 6 - Effect of PKIH Analogues on ³H-Thymidine, ³H-Leucine, and ³H-Uridine Incorporation

### Methodology: ³H-Thymidine, ³H-Leucine, and ³H-Uridine Incorporation Assay

After a 20 h incubation of the SK-N-MC cells with a selection of analogues, ³H-thymidine, ³H-leucine, or ³H-uridine (1 µCi/ml) was added for 2 h at 37°C. The cells were detached from the 96 well plate using 1 mM EDTA in Ca(II)/Mg(II)-free PBS and collected and washed using a cell harvester (Tomtec Harvester 96, Linbrook, Queensland, Australia). Radioactivity was measured on a β-scintillation counter (LKB Wallace, Turku, Finland).

### Example 6a - Effect of Compounds of the PCIH, QCIH and PKIH Series on ³H-Thymidine Incorporation

The effect of compounds of the PKIH series on ³H-thymidine incorporation was compared to the corresponding PCIH and QCIH analogues and also to DFO and 311 as relevant controls (Table 6).

**Table 6: The effect of compounds of the PCIH, QCIH and PKIH series on ³H-thymidine incorporation by SK-N-MC neuroepithelioma cells.**

| **³H-Thymidine Incorporation (% Control)** | | | | | |
|---|---|---|---|---|---|
| **PCIH Analogues** | | **QCIH Analogues** | | **PITH Analogues** | |
| | | | | | |
| Control | 100 ± 7 | Control | 100 ± 7 | Control | 100 ± 7 |
| DFO | 29 ± 13 | DFO | 29 ± 13 | DFO | 29 ± 13 |
| 311 | 0.1 ± 0.1 | 311 | 0.1 ± 0.1 | 311 | 0.1 ± 0.1 |
| PCIH | 43 ± 1 | QCIH | 77 ± 9.5 | PKIH | 0.06 ± 0.02 |
| PCTH | 64 ± 14 | QCTH | 83 ± 6 | PKTH | 0.04 ± 0.01 |
| PCBH | 72 ± 16 | QCBH | 64 ± 5 | PKBH | 0.03 ± 0.01 |
| PCBBH | 33 ± 6 | QCBBH | 81 ± 10 | PKBBH | 0.02 ± 0.01 |
| PCAH | 12 ± 1 | QCAH | 43 ± 2 | PKAH | 23 ± 10 |
| PCHH | 11 ± 2 | QCHH | 41 ± 2 | PKHH | 25 ± 4 |

| | | | | | |
|---|---|---|---|---|---|
| Data is expressed as Mean t SD from 4 experiments. | | | | | |

PKIH, PKTH, PKBH, and PKBBH, were the most effective compounds examined in terms of their ability to inhibit ³H-thymidine incorporation, showing similar or slightly greater activity than the ribonucleotide reductase inhibitor, 311 (Table 6). In fact, there was almost undetectable levels of ³H-thymidine in cells treated with the PKIH analogues. For instance, PKBBH reduced ³H-thymidine incorporation to 0.02 % of the control (Table 6). Several of the more hydrophilic PKIH analogues, namely PKAH and PKHH (Table 1), showed significantly (p < 0.0001) less activity, inhibiting ³H-thymidine incorporation to 23 % and 25 % of the control respectively (Table 6). However, the relatively low efficacy of these latter compounds at inhibiting ³H-thymidine incorporation cannot solely be attributed to their hydrophilicity, as the most hydrophilic analogue of this series (PKIH; Table 1) had very high activity (Table 6).

In comparison to 311 and the PKIH analogues, the QCIH analogues showed considerably less activity at inhibiting ³H-thymidine incorporation, reducing incorporation to 41-83% of the control (Table 6).

### Example 6b - Comparison of Compounds of the PCIH and PKIH Series on ³H-Leucine and ³H-Uridine Incorporation

Considering the marked effects of the PKIH group of analogues on ³H-thymidine incorporation, the effect of these compounds on ³H-leucine (Table 7) and ³H-uridine (Table 8) incorporation was also assessed. The results obtained were compared to the PCIH series of analogues which show high Fe chelation activity but low anti-proliferative effects.

**Table 7: The effect of the PCIH, and PKIH ligands on ³H-leucine incorporation by SK-N-MC neuroepithelioma cells.**

| **³H-Leucine Incorporation (% Control)** | | | |
|---|---|---|---|
| **PCIH Analogues** | | **PKIH Analogues** | |
| Control | 100 ± 7 | Control | 100 ± 7 |
| DFO | 9 ± 1 | DFO | 9 ± 1 |
| 311 | 2 ± 1 | 311 | 2 ± 1 |
| PCIH | 74 ± 7 | PKIH | 26 ± 1 |
| PCTH | 21 ± 6 | PKTH | 19 ± 8 |
| PCBH | 30 ± 13 | PKBH | 21 ± 2 |
| PCBBH | 16 ± 5 | PKBBH | 8 ± 1 |
| PCAH | 76 ± 2 | PKAH | 25 ± 2 |
| PCHH | 80 ± 10 | PKHH | 22 ± 0.5 |

| | | | |
|---|---|---|---|
| Data is expressed as Mean ± SD from 4 experiments. | | | |

**Table 8: The effect of the PCIH, and PKIH ligands on ³H-uridine incorporation by SK-N-MC neuroepithelioma cells.**

| **³H-Uridine Incorporation (% Control)** | | | |
|---|---|---|---|
| **PCIH Analogues** | | **PKIH Analogues** | |
| Control | 100 ± 2 | Control | 100 ± 2 |
| DFO | 34 ± 5 | DFO | 34 ± 5 |
| 311 | 5 ± 2 | 311 | 5 ± 2 |
| PCIH | 50 ± 8 | PKIH | 19 ± 1 |
| PCTH | 43 ± 2 | PKTH | 5 ± 1 |
| PCBH | 31 ± 9 | PKBH | 5 ± 1 |
| PCBBH | 47 ± 2 | PKBBH | 3 ± 1 |
| PCAH | 40 ± 9 | PKAH | 28 ± 1.5 |
| PCHH | 41 ± 11 | PKHH | 21 ± 1.5 |

| | | | |
|---|---|---|---|
| Data is expressed as Mean ± SD from 3 experiments. | | | |

The most active of the PKIH analogues in terms of inhibiting ³H-thymidine incorporation, namely PKIH, PKTH, PKBH and PKBBH, were significantly (p < 0.0001) less effective at preventing the incorporation of ³H-leucine and ³H-uridine into protein and RNA respectively (Tables 7 and 8). Further, these PKIH analogues had significantly (p < 0.001) less effect than 311 on ³H-leucine incorporation (Table 7). In general, the PKIH analogues were more effective than the PCTH analogues at preventing ³H-leucine or ³H-uridine incorporation (Tables 7 and 8). PKBBH showed the greatest activity at inhibiting ³H-leucine or ³H-uridine incorporation.

### EXAMPLE 7 - Effect of the PKIH Analogues on the Expression of WAF1 and GADD45 mRNA

### Methodology: Northern Blot Analysis

Northern blot analysis was performed by isolating total RNA using the Total RNA Isolation Reagent (Advanced Biotechnologies Ltd, Surrey, United Kingdom). The membranes were hybridised with probes specific for human WAF1, GADD45, and βactin. The *WAF1* probe consisted of a 1 kb fragment from pSXV (ATCC; Cat. No. 79928). The *GADD45* probe consisted of a 760 bp fragment from human GADD45 cDNA cloned into pHu145B2 (kindly supplied by Dr. Albert Fornace, National Cancer Institute, NIH, Maryland). The β-actin probe consisted of a 1.4 kb fragment from human β-actin cDNA cloned into pBluescript SK- (ATCC; Cat. No. 37997).

The effect of PKIH analogues on the expression of the genes *WAF1* and *GADD45* which encode cell cycle inhibitors essential for G₁-S arrest was assessed. The internal controls DFO, PCBBH, and 311, caused up-regulation of *GADD45* and *WAF1* mRNA expression, while PCBH and PCIH showed little effect (Fig. 7). In good correlation with their marked anti-proliferative activity, PKIH, PKTH, PKBH and PKBBH had a pronounced effect, significantly (p < 0.0001) increasing *GADD45* and *WAF1* mRNA levels when compared to the control. The PKIH analogues were significantly (p < 0.01) more potent at inducing *WAF1* and *GADD45* mRNA expression than 311 (Fig. 7).

### EXAMPLE 8 - Induction of Ndrg1 mRNA Expression

MCF-7 cells were incubated for 24 h with a range of metal ion chelators. The cell membrane impermeable chelator, EDTA (250 µM) does not show anti-proliferative effects (IC₅₀ > 250 µM), and was compared to more permeable metal chelating compounds which show low and high anti-proliferative activity, respectively. The compounds with low anti-proliferative activity in MCF-7 cells, include DFO (IC₅₀ = 20 µM), L1 (deferiprone; IC₅₀ = 180 µM) and dipyridyl (IC₅₀ >250 µM) and these were assessed at 25 and 250 µM. The compounds 311 and dp44mT demonstrate high anti-tumour efficacy against MCF-7 cells (IC₅₀ = 0.6 µM and 0.1 µM, respectively), and were assessed at 25 µM (Fig. 16) Penicillamine is a well known copper chelator and was assessed for comparison. Dp2mT was used as a negative control.

At a concentration of 25 µM, DFO, penicillamine (PEN), dp2mT, EDTA, dipyridyl and L1 had no effect at up-regulating *Ndrg1* (Fig. 16). In contrast 311 and dp44mT markedly increased *Ndrg1* levels (Fig. 16).

### EXAMPLE 9 - The Effect of PKIH Analogues on Iron Regulatory Protein RNA-Binding Activity

### Methodology: Gel-retardation Assay

The gel-retardation assay was used to measure the interaction between the iron regulatory proteins (IRPs) and iron-responsive element (IRE) using established techniques.²¹ Cells were incubated with DFO (100 µM), FAC (100 µg/ml) and PKIH analogues (25 µM) for 20 h at 37°C and cell lysates prepared. The IRP-RNA-binding activity was then assessed by the gel-retardation assay. Aliquots of cell lysate containing 2 µg of protein were incubated with 0.1 ng (approximately 1 x 10⁵ cpm) of ³²P-Iabelled riboprobe for 30 min at room temperature. Using this protocol the level of RNA added was saturating. Unprotected probe was degraded by incubation with 1 U of RNAse T1 for 10 min at room temperature. Heparin (5 mg/ml) was then added for another 10 min to exclude non-specific binding. The IRE-protein complexes were analyzed on 6 % non-denaturing polyacrylamide gels. In parallel experiments, samples were treated with ferricyanide (FeCN, 5 mM) and 0.5% β-mercaptoethanol (β-ME), prior to addition of the IRE probe. This procedure results in the conversion of IRP1, whether spontaneously active or not, into an active IRE-binding form.²²

The IRP-IRE mechanism is a major regulator of cellular Fe homeostasis that responds to Fe chelation. Accordingly, the effect of PKIH analogues on IRP-RNA-binding activity was determined (Fig. 8). SK-N-MC cells were incubated with the chelators (25 µM) for 20 h at 37°C. The Fe chelators DFO (100 µM), 311 (25 µM), or the Fe donor ferric ammonium citrate (FAC; 100 µg/ml), were used as relevant controls in all experiments.^{3,23} PCAH was used as a further control as it was known to reduce IRP-RNA-binding activity.²⁴

A significant (p < 0.005) increase in IRP-RNA binding occurred in cells incubated with DFO and 311, while FAC and PCAH significantly (p < 0.001) decreased RNA-binding (Fig. 8). Compared to the control, there was a significant (p < 0.01) increase in IRP-RNA binding activity after treatment of cells with the PKIH series (Fig. 8), reflecting their ability to bind cellular Fe pools. Addition of β-mercaptoethanol and FeCN to the cell lysates demonstrated no change occurred in the total amount of IRP-RNA binding activity after incubation with all the chelators (data not shown).

### EXAMPLE 10 - INDUCTION OF APOPTOSIS

### Example 10a - Increased apoptotic cells in Dp44mT-treated tumour samples from mice and the examination of apoptosis in cultured M109 cells after incubation with Dp44mT

### Apoptosis Assay Using Flow Cytometry

Apoptotic cells were detected by a flow cytometer (FACSCalibur, Becton Dickinson, CA, USA) using fluorescein isothiocyanate (FITC) labelled Annexin V binding to translocated plasma membrane phosphatidylserine (PS).²⁵ Propidium iodide (PI) was added to identify loss of cell membrane integrity, which is indicative of necrotic cells.

DNA cleavage was assessed by the terminal deoxyribonucelotidyl transferase-mediated dUTP nick end-labelling (TUNEL) reaction.²⁶ Using the *in situ* TUNEL assay, tumour samples from animals treated with Dp44mT (0.4 mg/kg) (Fig. 12B(ii)) showed clear evidence of increased apoptotic cell death compared to vehicle-treated controls (Fig. 12B(i)). To further investigate the apoptotic pathways induced by Dp44mT, cultured M109 cells were incubated with Dp44mT for 24 h and analysed for apoptosis and death using Annexin V-FITC and PI staining, respectively. There was a dose-dependent increase in the percentage of apoptotic and dead cells after Dp44mT treatment (Fig. 13A). Exposure of cells to 1 µM Dp44mT caused a significant (p < 0.05) increase of apoptotic and dead cells. This increase peaked at a Dp44mT concentration of 100 µM resulting in a population consisting of 40 ± 7% apoptotic cells and 27 ± 3% dead cells (Fig. 13A). After incubation with Dp44mT at 250 µM, apoptotic cell numbers decreased to 20 ± 2%, and the dead cell population increased to 42 ± 6 %, suggesting dose-dependent cytotoxicity.

Induction of apoptosis by Dp44mT (1 µM) was also time-dependent (Fig 13B). After incubation with 1 µM Dp44mT for 12 h, a significant (p < 0.001) increase in apoptotic cells (12 ± 2 %) was detected. Thus, at 1 µM Dp44mT markedly promoted ⁵⁹Fe release (Fig. 11A,C), inhibited ⁵⁹Fe uptake (Fig. 11B,D), and induced apoptotic cell death (Fig. 13B).

### Example 10b - Protein levels and activities of caspase-3, 8, and 9 in M109 cells after incubation with Dp44mT.

### General Methodology

### Antibodies

Rabbit anti-active caspase-3 antibody, rabbit anti-active caspase-8 antibody and Annexin V fluorescein isothiocyanate (FITC) were from BD PharMingen (San Diego, CA, USA). Anti-h-cytc MoAb was from R&D Systems (Minneapolis, MN, USA). Rabbit anti-active caspase-9 antibody, mouse anti-Bcl-2 and mouse anti-Bax antibodies were obtained from Santa Cruz (Santa Cruz, CA, USA). Mouse anti-mouse β-actin antibody, horseradish peroxidase-conjugated antirabbit, and anti-mouse IgG were from Sigma Chemical Co. (St. Louis, MO, USA).

### Caspase Activation and Activity Assay

Cultured M109 cells were treated with or without Dp44mT (1 µM) for 0-48 h at 37°C. Cells were lysed²⁷ and caspase activities were determined using the BD Apolert^{™} Caspase Assay Plate (BD Biosciences, San Diego, CA, USA). Cell-permeable pancaspase-3, -8, and -9 inhibitors (BD Biosciences) were included as inhibitory controls.

To determine the role of caspase activation in Dp44mT-induced apoptosis in M109 cells, protein levels of active caspase-3, -8 and -9 (Fig. 14A,B) and their enzymatic activity (Fig. 14C,D) were examined. Short incubations of 2 h with Dp44mT (1 µM) increased the protein levels of caspase-3 and -9 to 168 and 171% of the control, respectively, while caspase-8 was not increased.

Incubation of M109 cells with Dp44mT (1 µM) for 6 h resulted in a pronounced increase of caspase-3 to 400% of the control, while the increase in caspase-9 and caspase-8 were less marked, being elevated to 280% and 150% of the control, respectively (Fig.14A,B). An incubation of 12 h with Dp44mT resulted in caspase-8 protein levels increasing to 400% of the control, while that of caspase-3 and -9 remained at approximately the same level as that found at 6 h (i.e., at 380% and 280% of the control, respectively). An incubation of 24 h with Dp44mT increased the protein level of all caspases to about 4-fold that of control cells, while after 48 h, caspase-3, -8 and -9 levels were 5.5-, 3.4- and greater than 10-fold that of control cells (Fig. 14A,B).

Caspase-3 activity increased most markedly after 6 h, and this occurred in a time-dependent manner to 29-fold of that found for the control after 48 h (Fig. 14C). The activities of caspase-8 and -9 increased to a far lesser extent to 299% and 354% of the control after a 12 h incubation with Dp44mT (Fig. 14C). The activity of these latter enzymes plateaued at approximately 400-600% of the control after 24 h (Fig. 14C). Membrane-permeable inhibitors of each caspase reversed their activation when incubated with Dp44mT (1 µM), demonstrating the specificity of the observed activity (Fig. 14D.

These results show that Dp44mT increased caspase-3 and -9 protein levels within 2 h, while the protein levels of caspase-8 were only slightly increased even after a 4 h or 6 h (Fig. 14A,B). The most striking increase in enzymatic activity was observed for caspase-3, which increased 300-fold compared to the control after 48 h incubation with Dp44mT (Fig. 14C).

### Example 10c - Release of holo-cytochrome c (h-cytc) from mitochondria into the cytosol of M109 cells after incubation with Dp44mT

Due to the importance of h-cytc release from the mitochondrion on apoptosis induction,⁸ further studies investigated the effect of Dp44mT on this process (Fig. 15A). Preparation of cytosolic and mitochondrial fractions and western blotting were carried out using standard techniques.²⁷ There was very little h-cytc expression in the cytosolic fractions of control M109 cells (i.e., at 0 h), with the greatest amount of h-cytc being found in the mitochondrion at this time (Fig. 15A). However, after a 2 h exposure to Dp44mT (1 µM), h-cytc was found in the cytosolic fraction of M109 cells, while there was a concomitant decrease in the mitochondrial level of h-cytc (Fig. 15A). The release of h-cytc into the cytosol plateaued after a 4 h incubation with Dp44mT and then decreased to that found in control cells after 24 and 48 h. This increase in cytosolic h-cytc correlated with the loss from the mitochondrion (Fig. 15A). The near complete disappearance of h-cytc from the cytosol at 24 and 48 h may be due to its release from cells or its degradation²⁸ (Fig. 15A).

Incubation with Dp44mT for only 2 h resulted in h-cytc release from the mitochondrion (Fig. 15A) and this occurred at a similar time or before activation of caspases-3, -8 and -9 (Fig. 14A-C). While note intending to be limited to a particular mechanism of action, this observation is consistent with Dp44mT inducing the mitochondrial pathway of apoptosis and accounts, at least in part, for the marked activation of caspases.

### Example 10d - Expression of Bcl-2 and Bax in M109 cells after incubation with Dp44mT.

Considering the roles of Bcl-2 and Bax in the mitochondrial pathway of apoptosis and their role in h-cytc release,^{8,11,12} the effect of Dp44mT (1 µM) on the protein levels of these molecules was assessed (Fig. 15B). After incubation with Dp44mT (1 µM), the anti-apoptotic molecule Bcl-2 gradually decreased as a function of time, decreasing to 69% and 37% of the 0 h time point after a 2 and 48 h incubation, respectively (Fig. 15B). Conversely, Bax expression only increased markedly after a 48 h incubation with Dp44mT, reaching 500% of that found at the 0 h time point (Fig. 15B).

The release of h-cytc observed within 2 h (Fig. 15A) coincided with the decrease in the mitochondrial Bcl-2 expression (Fig. 15B), which is involved in preventing h-cytc release from mitochondria.⁸ However, a marked increase in Bax expression which plays a role in h-cytc release and caspase activation⁸ was only observed after 48 h (Fig. 15B).⁸ The imbalance of Bcl-2 and Bax expression may have resulted in mitochondrial h-cytc release, as found under other conditions.⁸ Considering h-cytc efflux from the mitochondrion, previous studies have demonstrated that oxidant stress is a potent inducer of its release as well as the down-regulation of Bcl-2, the upregulation of pro-apoptotic proteins (eg., Bax), and the activation of caspases.²⁷

### Example 10e - Increase of Intracellular ROS Generation after Incubation with Dp44mT Intracellular Reactive Oxygen Species (ROS) Assay

Intracellular ROS generation was assessed using the cell-permeable dye 2',7'-dichlorofluorescein-diacetate (DCF-DA) (Sigma). DCF-DA readily diffuses into cells and becomes highly fluorescent when oxidised by O₂^{·-}, H₂O₂, or OH⁻.²⁹ Cellular fluorescence intensity is directly proportional to the level of intracellular ROS.²⁹ M109 cells (1 x 10⁵ cells/well) were incubated with or without 1 µM Dp44mT for 0-48 h or 50 µM H₂O₂ (positive control) for 10 min at 37°C. The cells were then incubated with DCF-DA (5 µM) for 20 min at 37°C, washed twice, and detached from the culture plates. The fluorescence of oxidised DCF in cells was measured using flow cytometry.²⁹⁾

To test whether induction of h-cytc release from the mitochondrion (Fig 15A) and the imbalance of Bcl-2Bax expression (Fig 15B) was due to the presence of ROS, intracellular ROS were measured by flow cytometry using the DCFH-DA probe which becomes fluorescent when oxidised by O₂^{·-}, H₂O₂, or OH⁻.²⁹ Incubation of M109 cells with Dp44mT (1 µM) increased the levels of oxidised probe as a function of time. After 2 h, a 25 ± 10% increase in oxidised DCFH-DA fluorescence was found compared to the 0 h time point, and this increased to 486 ± 96% after 48 h (Fig. 15C). For comparison, a 10 min incubation with the positive control, H₂O₂ (50 µM), increased fluorescence to 1312 ± 46% of the 0 h time point (Fig. 15C)

In this study, an increase in ROS was detected after a 2 h incubation with Dp44mT (Fig. 15C) and this coincided with h-cytc release (Fig. 15A). These observations are consistent with a role of ROS in inducing h-cytc release, however other mechanisms cannot be excluded.

### References

1. Blatt J, Stitely S. Cancer Res. 1987;47:1749-1750.
2. Chaston TB, Lovejoy DB, Watts RN, Richardson DR. Clin Cancer Res. 2003;9:402-414.
3. Richardson, D.R. & Bernhardt, P. (1999) *J. Biol. Inorg. Chem.,* **4**, 266-273.
4. Salnikow, K., Su, W., Blagosklonny, M.V. and Costa, M. (2000). Cancer Res. 60, 3375-3378.
5. Kurdistani, S.K., Arizti, P., Reimer, C.L., Sugrue, M.M., Aaronson, S.A., and Lee, S.W. (1998). Cancer Res. 58, 4439-4444.
6. Bandyopadhyay S, Pai SK, Gross SC, Hirota S, Hosobe S, Miura K, Saito K, Commes T, Hayashi S, Watabe M, Watabe K. Cancer Res.2003;63:1731-6.
7. Shimono, A., Okuda, T., Kondho H. (1999). Mech Devel. 83, 39-52.
8. Lim MLR, Lum M-G, Hansen TM, Roucou X, Nagley P. J Biomed Sci. 2002;9:488-506.
9. Cohen GM. Biochem J. 1997;326:1-16
10. Budihardjo I, Oliver H, Lutter M, Luo X, Wang X. Annu Rev Cell Dev Biol. 1999;15:269-290.
11. Kluck RM, Bossy-Wetzel E, Green DR, Newmeyer DD. Science. 1997;275:1132-1136.
12. Jurgensmeier JM, Xie Z, Deveraux Q, Ellerby L, Bredesen D, Reed JC. Proc Natl Acad Sci USA. 1998;95:4997-5002.
13. Bacchi, A., Carcelli, M., Costa, M., Pelagatti, P., Pelizzi, C. & Pelizzi, G. (1996). *J. Chem. Soc. Dalton Trans.,* **22**, 4239-4244.
14. Johnson, D.K, Murphy, T.B, Rose NJ, Goodwin WH, Pickart L. Inorg Chim Acta. 1982;67:159-162.
15. Broto, P., Moreau, G. & Vandycke, C. (1984). *Eur. J. Med. Chem. Chim. Theor.,* **19**, 71-78
16. Ghose, A.K. & Crippen, G.M. (1987). *J*. *Chem. Inf: Comput. Sci.,* **27**, 21-35.
17. Viswanadhan, V.N., Chose, A.K., Revankar, G.R. & Robins, R.K. (1987) *J*. *Chem. Inf. Comput. Sci.,* **29**, 163-172.
18. Richardson, D.R., Tran, E. & Ponka, P. (1995). *Blood,* **86**,4295-4306
19. Selig RA, White L, Gramacho C, Sterlinglevis K, Fraser IW, Naidoo D. Cancer Res. 1998;58:473-478
20. Finch RA, Liu M-C, Grill SP, et al. Biochem Pharmacol. 2000;59:983-991
21. Müllner, E.W., Neupert, B. & Kühn, L.C. (1989). *Cell,* **58**, 373-382.
22. Abboud, S. & Haile, D.J. (2000). *J. Biol. Chem.* **275**, 19906-19912.
23. Darnell, G. & Richardson, D.R. (1999). *Blood,* **94**, 781-792.
24. Becker, E. & Richardson, D.R. (1999). *J. Lab. Clin. Med.,* **134**, 510-521
25. Vermes I, Haanen C, Steffens-Nakken H, Reutelingsperger C. J Immunol Methods. 1995;184:39-51.
26. Gavrieli Y, Sherman Y, Ben-Sasson SA. J Cell Biol. 1992;119:493-501.
27. Yuan J, Murrell GA, Trickett A, Wang MX. Biochim Biophys Acta. 2003;1641:35-41.
28. Jemmerson R, LaPlante B, Treeful A. Cell Death Differ. 2002;9:538-548
29. Wang M, Wei AQ, Yuan J, Trickett A, Knoops B, Murrell GAC. FEBS Lett. 2002;532:359-362.
30. El-Deiry, W.S., Tokino, Tl, Velculescu, V.E., Levy, D.B., Parsons, R., trent, J.M., Lin, D., Mercer, W.E., Kinzler, K.W., & vogelstein, B. (1993). Cell, 75, 817-825.

## Claims

1. A compound of Formula 3 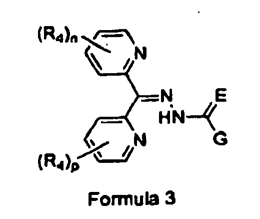 wherein
E is o or S;
n is 0, 1, 2, 3 or 4,
p is 0, 1, 2, 3 or 4, and
each R₄ is independently selected from halogen, alkyl, alkenyl, optionally substituted amino, hydroxyl, -O-alkyl, -S-alkyl, -C(O)-alkyl, -C(O)-alkenyl, -C(O)-O-alkyl, nitro and cyano,
wherein, the term "alkyl, group" includes within its meaning straight chain or branched chain saturated aliphatic groups having from 1 to 10 carbon atoms, eg, 1, 2, 3, 4, 5, 6, 7, 8, 9 or 10 carbon atoms, or cyclic saturated aliphatic groups having from 3 to 8 carbon atoms, eg, 3, 4, 5, 6, 7, or 8 carbon atoms, or bicyclic saturated aliphatic groups having 7, 8, 9 or 10 carbon atoms
and the term "alkenyl group" includes within its meaning straight or branched chain unsaturated aliphatic hydrocarbon groups having from 2 to 8 carbon atoms, e.g., 2, 3, 4, 5, 6, 7, or 8 carbon atoms, or cyclic unsaturated aliphatic hydrocarbon groups having from 3 to 8 carbon atoms, e.g, 3, 4, 5, 6, 7or 8 carbon atoms, and combinations thereof, having at least one double bond, of either E or Z stereochemistry where applicable;
-G is NR₂R₃ or CR₂R₃R₅,
wherein the bond between C and R₅ may be a single bond, double bond, or triple bond; and wherein when the bond between C and R₅ is a double bond, one of R₂ and R₃ is absent, and wherein when the bond between C and R₅ is a triple bond, R₂ and R₃ are both absent;
R₂ and R₃ may be the same or different and are individually selected from hydrogen, halogen, optionally substituted alkyl, optionally substituted alkenyl, optionally substituted alkynyl, optionally substituted amino, optionally substituted heteroaromatic, an optionally substituted bicyclic group, and an optionally substituted aromatic group; and
R₅ is selected from the group consisting of hydrogen, halogen, optionally substituted alkyl, optionally substituted alkenyl, optionally substituted alkynyl optionally substituted amino, optionally substituted heteroaromatic, an optionally substituted bicyclic group, and an optionally substituted aromatic group;
or -G is an optionally substituted heteroaromatic group, an optionally substituted bicyclic group, an optionally substituted aromatic group, an optionally substituted alkyl group; an optionally substituted cycloalkyl group, or an optionally substituted heterocycloalkyl group;
with the provisos that:
(i) when E is O, n is 0 and p is 0, then
-G is not -NH_{2;} -CH₂Cl; 2-furanyl; 2-thiophenyl; phenyl; 2-halophenyl, 3-halophenyl or 4-halophenyl, where halo is F, Cl, Br or I; 2-aminophanyl; 4-aminophenyl; 2-hydroxyphenyl; 4-hydroxyphenyl; 2-pyridyl; 4-pyridyl; 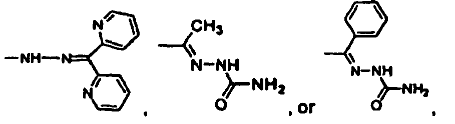 and
(ii) when E is S, n is 0 and p is 0, then;
-G is not -NH₂; -NHCH₃; -NHPhenyl; N(benzyl)₂; -NH(2-pyridyl); N(methyl)₂; N(ethyl)₂; N(propyl)_{2;} N(butyl)₂; N(pentyl)₂; N(hexyl)₂; -N-pyrrolidinyl: -N-morpholinyl;
2-thiophenyl; 2-furanyl; 2-hydroxyphenyl; 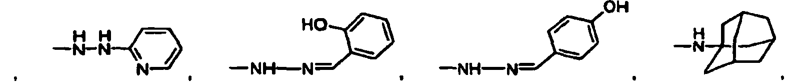 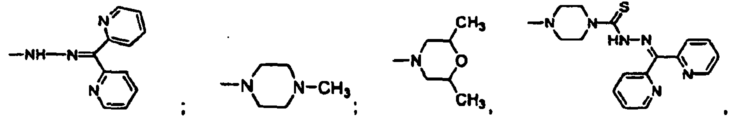 or 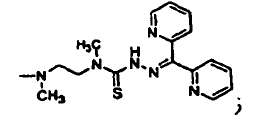 and
(iii) when E is S, n is O and P is 1, then
a) when R₄ is 6-Br, G is not N(methyl)₂;
b) when R₄ is 6-Me, G is not N(Methyl)₂; and
c) when R₄ is 6-F, G is not N-pyrrolidinyl.

2. A compound according to claim 1, wherein E is S; and -G is selected from an optionally substituted C₁₋₈ alkyl group, C₂₋₈ alkenyl, aryl, heteroaryl. -CH₂(aryl)₂, and CH(aryl)₂.

3. A compound according to claim 1, wherein E is O: and G is selected from an optionally substituted C₁₋₅ alkyl group, C₂₋₄ alkenyl, aryl, heteroaryl, -CH₂(aryl)₂ and CH(aryl)₂.

4. A compound according to any one of claims 1 to 3, wherein each of said optional substituents is independently selected from alkyl, alkenyl, alkynyl, C₈₋₁₀ aryl, halo, amino, hydroxyl, O-alkyl, S-alkyl, nitro, cyano, C(O)-alkyl, C(O)-O-( alkyl), C(O)NH(alkyl), and C(O)N(alkyl)₂.

5. A compound according to claim 1, wherein said compound is a compound of Formula 4: 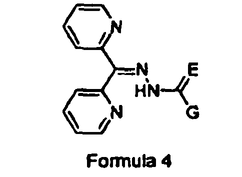 wherein
E is O ar S; and
G is as defined in claim 1, including provisos (i) and (ii).

6. A compound selected from

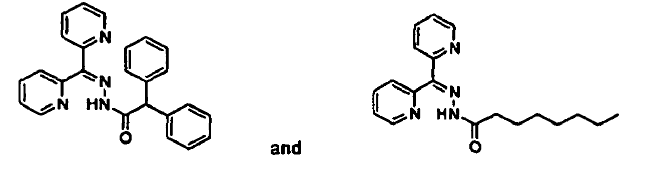

7. A compound selected from

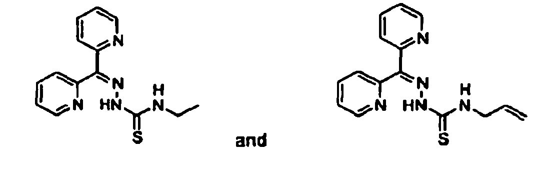

8. A metal ion complex of a compound according to any one of claims 1 to 7 with or without provisos (i) and (ii), wherein the metal ion is selected from iron (II), iron (III), copper, zinc, palladium, platinum and gallium.

9. A pharmaceutical composition comprising a pharmaceutically acceptable diluent or carrier and at least one compound according to any one of claims 1 to 7 or at least one metal ion complex according to claim 8.

10. A least one compound according to any one of claims 1 to 7 with or without provisos (i) and (ii), or a pharmaceutical composition comprising at least one compound according to any one of claims 1 to 7 with or without provisos (i) and (ii), together with a pharmaceutically acceptable diluent, adjuvant, or excipient, for use in iron chelation therapy.

11. A compound according to any one of claims 1 to 7 with or without provisos (i) and (ii), or a metal ion complex according to claim 8, or a pharmaceutical composition according to claim 9 with the exclusion of the compound bis(2-pyridyl)ketone thiosemicarbazone for use in therapy for inhibiting cellular proliferation or for treating a proliferative disorder.

12. A compound, complex or composition for use according to claim 11, wherein the proliferative disorder is selected from angiogenesis-dependent diseases, cellular proliferative diseases, Inflammatory disorders, auto-immune diseases, blood vessel diseases, thrombosis, cancer, a malignant tumour, a benign tumour, a solid tumour and a non-solid tumour.

13. In vitro use of a compound according to any one of claims 1 to 7 with or without provisos (i) and (ii), or a metal ion complex according to claim 8, or a pharmaceutical composition according to claim 9 for inducing apoptosis in a cell, or for inhibiting cellular proliferation and inducing apoptosis in a cell.

14. A compound according to any one of claims 1 to 7 with or without provisos (i) and (ii), or a metal ion complex according to claim 8, or a pharmaceutical composition according to claim 9 for use in therapy for inducing apoptosis in a mammal or for inducing apoptosis and inhibiting cellular proliferation in a mammal.

15. A metal ion complex for use according to any one of claims 11 to 14, wherein the metal ion complex is an iron(ii) complex or an iron (III) complex.

16. A compound for use according to any one of claims 11 to 14, wherein the compound is selected from:
di-2-pyridylketone 4-methyl-3-thiosemicarbazone;
di-2-pyridylketone 4,4-dimethyl-3-thiosemicarbazone;
di-2-pyridylketone 4-ethyl-3-thiosemicarbazone;
di-2-pyridylketone 4-allyl-3-thiosemicarbazone;
di-2-pyridylketone 4-phenyl-3-thiosemicarbazone:
di-2-pyridylketone isonicotinoyl hydrazone;
di-2-pyridylketone benzoyl hydrazone;
di-2-pyridylketone 4-hydroxybenzoyl hydrazone;
di-2-pyridylketone 3-bromobenzoyl hydrazone;
di-2-pyridylketone 4-aminobenzoyl hydrazone;
di-2-pyridylketone 2-thiophenecarboxaldehyde hydrazone;
di-2-pyridylketone 4,4-dlphenylcarboxaldehyde semicarbazone; and
di-2-pyridylketone octanoic hydrazone.

17. A compound for use according to any one of claims 11 to 14, wherein the compound, is di-2-pyridytketone d,4-dimethyl-3-thiosemlcarbazone.

18. A compound for use according to any one of claims 11 to 14, wherein the compound is a metal ion complex selected from Fe[PKIH]₂, Fe[PKBH]₂, Fe[PKHH]₂, Fe[PBBH]₂. Fe[PKAH]₂, Fe[PKTH]₂. Fe[PK44pH]₂ and Fe[PKoctH]₂, Fe[Dp4mT]₂, Fe[Dp44mT]₂, Fe[Dp4eT]₂, Fe[Dp4aT]₂, and Fe[Dp4pT]₂.

19. A compound for use according to any one of claims 11 to 14, wherein the compound is selected from di-2-pyridylketane isonicotinoyl thiohydrazone; di-2-pyridylketone benzoyl thiohydrazone; di-2-pyridylketane 4-hydroxybenzoyl thiohydrazone; di-2-pyridylketone 3-bromobenzoyl thiohydrazone; di-2-pyrtdylketone 4-aminobenzoyl thiohydrazone; di-2-pyridylketone 2-thiophenecarboxaldehyde thiohydrazone; di-2-pyridylketone 4,4-diphenylcarboxaldehyde thlosemicarbazone; and di-2-pyridylketone octanoic thiohydrazone.

## Patentansprüche

1. Verbindung nach Formel 3 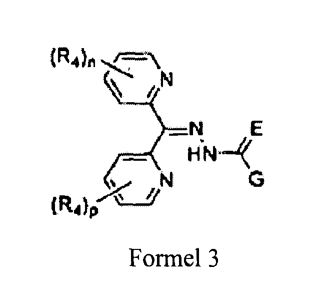 wobei
E O oder S ist;
n 0, 1, 2, 3 oder 4 ist,
p 0, 1, 2, 3 oder 4 ist und
jedes R₄ unabhängig aus Folgendem ausgewählt wird: Halogen, Alkyl, Alkenyl, optional substituiertes Amino, Hydroxyl, -O-Alkyl, -S-Alkyl, -C(O)-Alkyl, -C(O)-Alkenyl, -C(O)-O-Alkyl, Nitro und Cyano,
wobei der Begriff "Alkylgruppe" geradkettige oder verzweigtkettige gesättigte aliphatische Gruppen umfasst, die 1 bis 10 Kohlenstoffatome, z.B. 1, 2, 3, 4, 5, 6, 7, 8, 9 oder 10 Kohlenstoffatome, aufweisen, oder zyklische gesättigte aliphatische Gruppen, die 3 bis 8 Kohlenstoffatome, z.B. 3, 4, 5, 6, 7 oder 8 Kohlenstoffatome, aufweisen, oder bizyklische gesättigte aliphatische Gruppen, die 7, 8, 9 oder 10 Kohlenstoffatome aufweisen,
und der Begriff "Alkenylgruppe" geradkettige oder verzweigtkettige ungesättigte aliphatische Kohlenwasserstoffgruppen umfasst, die 2 bis 8 Kohlenstoffatome, z.B. 2, 3, 4, 5, 6, 7 oder 8 Kohlenstoffatome, aufweisen, oder zyklische ungesättigte aliphatische Kohlenwasserstoffgruppen, die 3 bis 8 Kohlenstoffatome, z.B. 3, 4, 5, 6, 7 oder 8 Kohlenstoffatome, aufweisen, und Kombinationen davon, die mindestens eine Doppelbindung mit ggf. entweder einer E oder einer Z-Stereochemie aufweisen;
-G NR₂R₃ oder CR₂R₃R₅ ist,
wobei die Bindung zwischen C und R₅ eine Einfachbindung, eine Doppelbindung oder eine Dreifachbindung sein kann; und wobei die Bindung zwischen C und R₅ eine Doppelbindung ist, entweder R₂ oder R₃ abwesend ist, und wobei sowohl R₂ als auch R₃ abwesend sind, wenn die Bindung zwischen C und R₅ eine Dreifachbindung ist;
R₂ und R₃ gleich oder verschieden sein können und individuell aus Folgendem ausgewählt werden: Wasserstoff, Halogen, optional substituiertes Alkyl, optional substituiertes Alkenyl, optional substituiertes Alkynyl, optional substituiertes Amino, eine optional substituierte heteroaromatische Gruppe, eine optional substituierte bizyklische Gruppe, und eine optional substituierte aromatische Gruppe; und
R₅ aus der Gruppe ausgewählt wird, die aus Folgendem besteht: Wasserstoff, Halogen, optional substituiertes Alkyl, optional substituiertes Alkenyl, optional substituiertes Alkynyl, optional substituiertes Amino, eine optional substituierte heteroaromatische Gruppe, eine optional substituierte bizyklische Gruppe, und eine optional substituierte aromatische Gruppe;
oder -G eine optional substituierte heteroaromatische Gruppe, eine optional substituierte bizyklische Gruppe, eine optional substituierte aromatische Gruppe, eine optional substituierte Alkylgruppe; eine optional substituierte Zykloalkylgruppe oder eine optional substituierte Heterozykloalkylgruppe ist;
unter den Bedingungen, dass:
(i) wenn E O ist, n 0 ist und p 0 ist, dann
ist -G nicht -NH_{2;} -CH₂Cl; 2-Furanyl; 2-Thiophenyl; Phenyl; 2-Halophenyl; 3-Halophenyl; oder 4-Halophenyl, wobei Halo F, Cl, Br oder I ist; 2-Aminophenyl; 4-Aminophenyl; 2-Hydroxyphenyl; 4-Hydroxyphenyl; 2-Pyridyl; 4-Pyridyl ist; 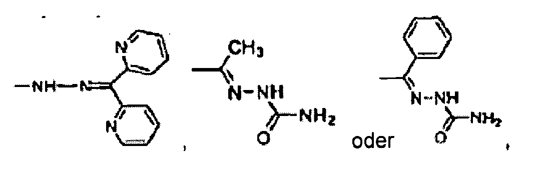 und
(ii) wenn E S ist, n 0 ist und p 0 ist, dann
ist -G nicht -NH₂; -NHCH₃; -NHPhenyl; N(Benzyl)₂; -NH(2-Pyridyl); N(Methyl)₂; N(Ethyl)₂; N(Propyl)₂; N(Butyl)₂; N(Pentyl)₂; N(Hexyl)₂; -N-Pyrrolidinyl; -N-Morpholinyl; 2-Thiophenyl; 2-Furanyl, 2-Hydroxyphenyl; 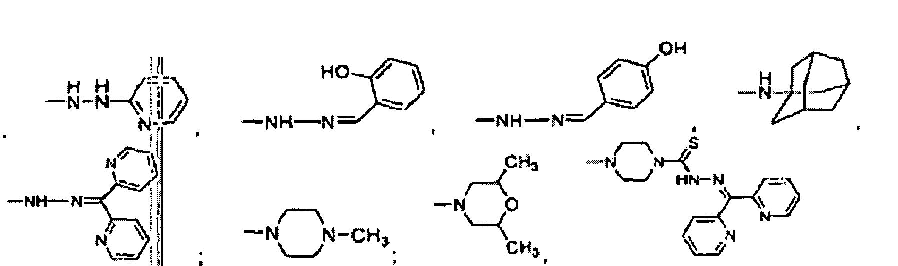 oder 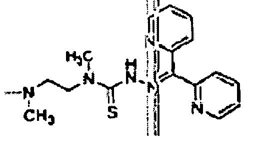 und
(iii) wenn E S ist, n 0 ist und p 1 ist, dann
a) wenn R₄ 6-Br ist, dann ist G nicht N(Methyl)₂;
b) wenn R₄ 6-Me ist, dann ist G nicht N(Methyl)₂; und
c) wenn R₄ 6-F ist, dann ist G nicht N-Pyrrolidinyl;

2. Verbindung nach Anspruch 1, wobei E S ist; und -G aus Folgendem ausgewählt wird: einer optional substituierten C₁₋₈ Alkylgruppe, C₂₋₈ Alkenyl, Aryl, Heteroaryl, -CH₂(Aryl)₂ und CH(Aryl)₂.

3. Verbindung nach Anspruch 1, wobei E O ist; und G aus Folgendem ausgewählt wird: einer optional substituierten C₁₋₈ Alkylgruppe, C₂₋₈ Alkenyl, Aryl, Heteroaryl, -CH₂(Aryl)₂ und CH(Aryl)₂.

4. Verbindung nach einem der Ansprüche 1 bis 3, wobei jeder der optionalen Substituenten unabhängig aus Folgendem ausgewählt wird: Alkyl, Alkenyl, Alkynyl, C₆₋₁₀ Aryl, Halo, Amino, Hydroxyl, O-Alkyl, S-Alkyl, Nitro, Cyano, C(O)-Alkyl, C(O)-O-(Alkyl), C(O)NH(Alkyl) und C(O)N(Alkyl)₂.

5. Verbindung nach Anspruch 1, wobei die Verbindung eine Verbindung nach Formel 4 ist: 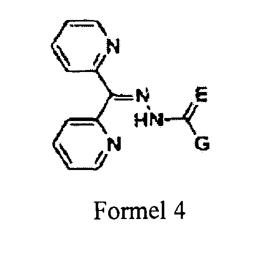 wobei
E O oder S ist; und
G der Definition aus Anspruch 1 entspricht, einschließlich der Bedingungen (i) und (ii).

6. Verbindung, die aus Folgendem ausgewählt wird:

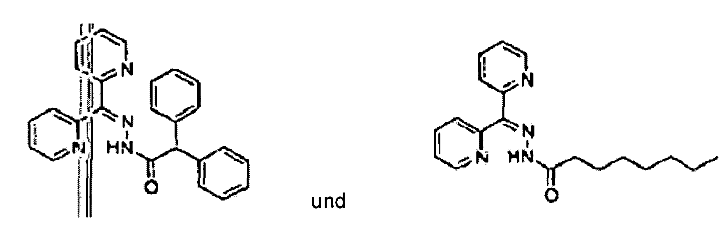

7. Verbindung, die aus Folgendem ausgewählt wird:

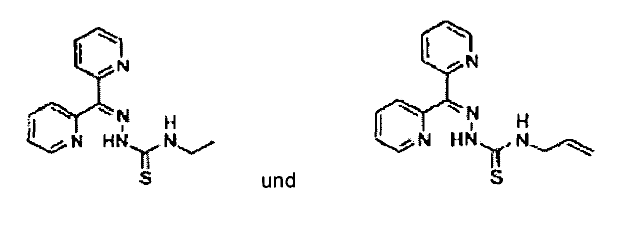

8. Metallionenkomplex einer Verbindung nach einem der Ansprüche 1 bis 7 mit den oder ohne die Bedingungen (i) und (ii), wobei das Metallion aus Folgendem ausgewählt wird: Eisen(II), Eisen(III), Kupfer, Zink, Palladium, Platin und Gallium.

9. Pharmazeutische Zusammensetzung, die ein pharmazeutisch akzeptables Verdünnungsmittel oder einen pharmazeutisch akzeptablenTräger und mindestens eine Verbindung nach einem der Ansprüche 1 bis 7 oder mindestens einen Metallionenkomplex nach Anspruch 8 umfasst.

10. Mindestens eine Verbindung nach einem der Ansprüche 1 bis 7 mit den oder ohne die Bedingungen (i) und (ii) oder eine pharmazeutische Zusammensetzung, die mindestens eine Verbindung nach einem der Ansprüche 1 bis 7 mit den oder ohne die Bedingungen (i) und (ii) umfasst, zusammen mit einem pharmazeutisch akzeptablen Verdünnungsmittel, Hilfsstoff oder Arzneiträger zur Verwendung bei der Eisenchelationstherapie.

11. Verbindung nach einem der Ansprüche 1 bis 7 mit den oder ohne die Bedingungen (i) und (ii) oder ein Metallionenkomplex nach Anspruch 8 oder eine pharmazeutische Zusammensetzung nach Anspruch 9 unter Ausschluss der Verbindung Bis(2-Pyridyl)ketonthiosemicarbazon zur Verwendung bei einer Therapie zur Hemmung von Zellproliferation oder zur Behandlung einer Proliferationskrankheit.

12. Verbindung, Komplex oder Zusammensetzung zur Verwendung nach Anspruch 11, wobei die Proliferationskrankheit aus Folgendem ausgewählt wird: Angiogenese-abhängige Krankheiten, Zellproliferationskrankheiten, Entzündungskrankheiten, Autoimmunkrankheiten, Blutgefäßkrankheiten, Thrombose, Krebs, ein Malignom, ein gutartiger Tumor, ein fester Tumor und ein weicher Tumor.

13. In-Vitro-Verwendung einer Verbindung nach einem der Ansprüche 1 bis 7 mit den oder ohne die Bedingungen (i) und (ii) oder ein Metallionenkomplex nach Anspruch 8 oder eine pharmazeutische Zusammensetzung nach Anspruch 9 zur Einleitung einer Apoptose in einer Zelle oder zur Hemmung von Zellproliferation und zur Einleitung einer Apoptose in einer Zelle.

14. Verbindung nach einem der Ansprüche 1 bis 7 mit den oder ohne die Bedingungen (i) und (ii) oder ein Metallionenkomplex nach Anspruch 8 oder eine pharmazeutische Zusammensetzung nach Anspruch 9 zur Verwendung bei einer Therapie zur Einleitung einer Apoptose in einem Säugetier oder zur Einleitung einer Apoptose und zur Hemmung von Zellproliferation in einem Säugetier.

15. Metallionenkomplex zur Verwendung nach einem der Ansprüche 11 bis 14, wobei der Metallionenkomplex ein Eisen(II)-Komplex oder ein Eisen(III)-Komplex ist.

16. Verbindung zur Verwendung nach einem der Ansprüche 11 bis 14, wobei die Verbindung aus Folgendem ausgewählt wird:
Di-2-Pyridylketon 4-Methyl-3-Thiosemicarbazon;
Di-2-Pyridylketon 4,4-Dimethyl-3-Thiosemicarbazon;
Di-2-Pyridylketon 4-Ethyl-3-Thiosemicarbazon;
Di-2-Pyridylketon 4-Allyl-3-Thiosemicarbazon;
Di-2-Pyridylketon 4-Phenyl-3-Thiosemicarbazon;
Di-2-Pyridylketonisonicotinoylhydrazon;
Di-2-Pyridylketonbenzoylhydrazon;
Di-2-Pyridylketon 4-Hydroxybenzoylhydrazon;
Di-2-Pyridylketon 3-Brombenzoylhydrazon;
Di-2-Pyridylketon 4-Aminobenzoylhydrazon;
Di-2-Pyridylketon 2-Thiophencarboxaldehydhydrazon;
Di-2-Pyridylketon 4,4-Diphenylcarboxaldehydsemicarbazon und
Di-2-Pyridylketonoctanhydrazon.

17. Verbindung zur Verwendung nach einem der Ansprüche 11 bis 14, wobei die Verbindung Di-2-Pyridylketon 4,4-Dimethyl-3-Thiosemicarbazon ist.

18. Verbindung zur Verwendung nach einem der Ansprüche 11 bis 14, wobei die Verbindung ein Metallionenkomplex ist, der aus Folgendem ausgewählt wird: Fe[PKIH]₂, Fe[PKBH]₂, Fe[PKHH]₂, Fe[PBBH]₂, Fe[PKAH]₂, Fe[PKTH]₂, Fe[PK44pH]₂ und Fe[PKoctH]₂, Fe[Dp4mT]₂, Fe[Dp44mT]₂, Fe[Dp4eT]₂, Fe[Dp4aT]₂ und Fe[Dp4pT]₂.

19. Verbindung zur Verwendung nach einem der Ansprüche 11 bis 14, wobei die Verbindung aus Folgendem ausgewählt wird: Di-2-Pyridylketonisonicotinoylthiohydrazon; Di-2-Pyridylketonbenzoylthiohydrazon; Di-2-Pyridylketon 4-Hydroxybenzoylthiohydrazon; Di-2-Pyridylketon 3-Brombenzoylthiohydrazon; Di-2-Pyridylketon 4-Aminobenzoylthiohydrazon; Di-2-Pyridylketon 2-Thiophencarboxaldehydthiohydrazon; Di-2-Pyridylketon 4,4-Diphenylcarboxaldehydthiosemicarbazon und Di-2-Pyridylketonoctanthiohydrazon.

## Revendications

1. Composé de Formule 3 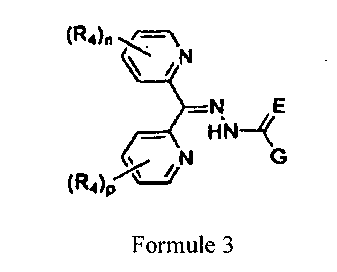 dans laquelle
E est O ou S ;
n est 0, 1, 2, 3 ou 4,
p est 0, 1, 2, 3 ou 4, et
chaque R₄ est indépendamment sélectionné parmi halogène, alkyle, alcényle, amino éventuellement substitué, hydroxyle, -O-alkyle, -S-alkyle, -C(O)-alkyle, -C(O)-alcényle, - C(O)-O-alkyle, nitro et cyano, dans lequel la signification du terme « groupe alkyle » inclut des groupes aliphatiques saturés à chaîne linéaire ou ramifiée ayant de 1 à 10 atomes de carbone, par ex., 1, 2, 3, 4, 5, 6, 7, 8, 9 ou 10 atomes de carbone, ou des groupes aliphatiques saturés cycliques ayant de 3 à 8 atomes de carbone, par ex., 3, 4, 5, 6, 7 ou 8 atomes de carbone, ou des groupes aliphatiques saturés bicycliques ayant 7, 8, 9 ou 10 atomes de carbone, et la signification du terme « groupe alcényle » inclut des groupes hydrocarbures aliphatiques non saturés à chaîne linéaire ou ramifiée ayant de 2 à 8 atomes de carbone, par ex., 2, 3, 4, 5, 6, 7 ou 8 atomes de carbone, ou des groupes hydrocarbures aliphatiques non saturés cycliques ayant de 3 à 8 atomes de carbone, par ex., 3, 4, 5, 6, 7 ou 8 atomes de carbone, et des combinaisons de ceux-ci, ayant au moins une double liaison, de stéréochimie E ou Z le cas échéant ;
-G est NR₂R₃ ou CR₂R₃R₅,
dans lequel la liaison entre C et R₅ peut être une simple liaison, une double liaison, ou une triple liaison ; et dans lequel quand la liaison entre C et R₅ est une double liaison, l'un parmi R₂ et R₃ est absent, et dans lequel quand la liaison entre C et R₅ est une triple liaison, R₂ et R₃ sont tous les deux absents ;
R₂ et R₃ peuvent être identiques ou différents et sont individuellement sélectionnés parmi hydrogène, halogène, alkyle éventuellement substitué, alcényle éventuellement substitué, alcynyle éventuellement substitué, amino éventuellement substitué, groupe hétéroaromatique éventuellement substitué, un groupe bicyclique éventuellement substitué, et un groupe aromatique éventuellement substitué ; et
R₅ est sélectionné parmi le groupe constitué d'hydrogène, halogène, alkyle éventuellement substitué, alcényle éventuellement substitué, alcynyle éventuellement substitué, amino éventuellement substitué, groupe hétéroaromatique éventuellement substitué, un groupe bicyclique éventuellement substitué, et un groupe aromatique éventuellement substitué ;
ou -G est un groupe hétéroaromatique éventuellement substitué, un groupe bicyclique éventuellement substitué, un groupe aromatique éventuellement substitué, un groupe alkyle éventuellement substitué, un groupe cycloalkyle éventuellement substitué, ou un groupe hétérocycloalkyle éventuellement substitué ;
à la condition que :
(i) quand E est O, n est 0 et p est 0, puis
-G n'est pas -NH₂ ; -CH₂Cl ; 2-furanyle ; 2-thiophényle ; phényle ; 2-halophényle ; 3-halophényle ou 4-halophényle, quand halo est F, Cl, Br ou 1 ; 2-aminophényle ; 4-aminophényle ; 2-hydroxyphényle ; 4-hydroxyphényle ; 2-pyridyle ; 4-pyridyle ; 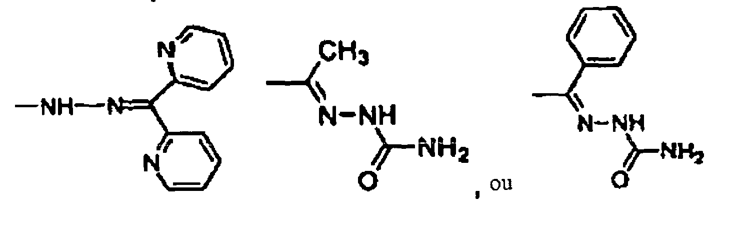 et
(ii) quand E est S, n est 0 et p est 0, puis
-G n'est pas NH₂, -NHCH₃, -NHPhényle, N(benzyle)₂ ; -NH(2-pyridyle) ; N(méthyle)₂ ; N(éthyle)₂ ; N(propyle)₂ ; N(butyle)₂ ; N(pentyle)₂; N(hexyle)₂ ; -N-pyrrolidinyle ; -N-morpholinyle ;
2-thiophényle ; 2-furanyle ; 2-hydroxyphényle ; 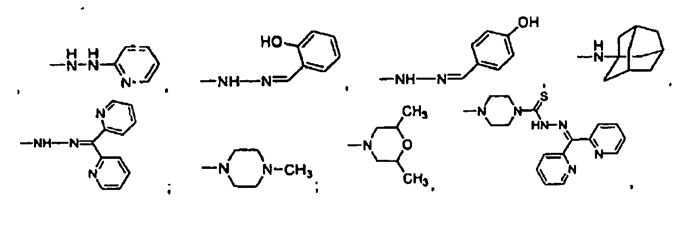 ou 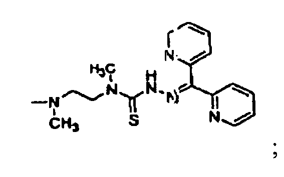 et
(iii) quand E est S, n est 0 et p est 1, puis
a) quand R₄ est 6-Br, G n'est pas N(méthyle)₂ ;
b) quand R₄ est 6-Me, G n'est pas N(méthyle)₂; et
c) quand R₄ est 6-F, G n'est pas N-pyrrolidinyle.

2. Composé selon la revendication 1, dans lequel E est S ; et -G est sélectionné parmi un groupe alkyle en C₁-C₆ éventuellement substitué, alcényle en C₂-C₈, aryle, hétéroaryle, - CH₂(aryle)₂ et CH(aryle)₂.

3. Composé selon la revendication 1, dans lequel E est O ; et G est sélectionné parmi un groupe alkyle en C₁-C₆ éve ntuellement substitué, alcényle en C₂-C₈, aryle, hétéroaryle, - CH₂(aryle)₂ et CH(aryle)₂.

4. Composé selon l'une quelconque des revendications 1 à 3, dans lequel chacun desdits substituants éventuels est indépendamment sélectionné parmi alkyle, alcényle, alcynyle, aryle en C₆-C₁₀, halo, amino, hydroxyle, O-alkyle, S-alkyle, nitro, cyano, C(O)-alkyle, C(O)-O-(alkyle), C(O)NH(alkyle), et C(O)N(alkyle)₂.

5. Composé selon la revendication 1, dans lequel ledit composé est un composé de Formule 4 : 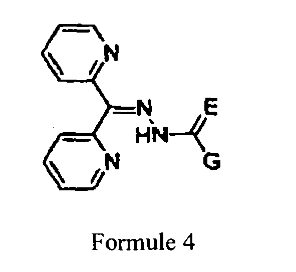 dans laquelle
E est O ou S ; et
G est tel que défini à la revendication 1, y compris aux conditions (i) et (ii).

6. Composé sélectionné parmi

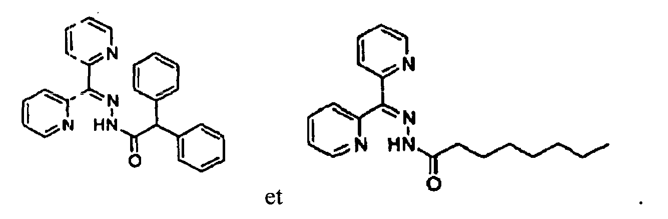

7. Composé sélectionné parmi

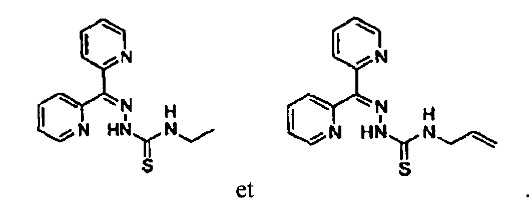

8. Complexe d'ion métallique d'un composé selon l'une quelconque des revendications 1 à 7, avec ou sans les conditions (i) et (ii), dans lequel l'ion métallique est sélectionné parmi le fer (II), le fer (III), le cuivre, le zinc, le palladium, le platine et le gallium.

9. Composition pharmaceutique comprenant un diluent ou un support pharmaceutiquement acceptable et au moins un composé selon l'une quelconque des revendications 1 à 7, ou au moins un complexe d'ion métallique selon la revendication 8.

10. Au moins un composé selon l'une quelconque des revendications 1 à 7 avec ou sans les conditions (i) et (ii), ou composition pharmaceutique comprenant au moins un composé selon l'une quelconque des revendications 1 à 7 avec ou sans les conditions (i) et (ii), avec un diluent, adjuvant, ou excipient pharmaceutiquement acceptable, à utiliser dans une thérapie par chélation du fer.

11. Composé selon l'une quelconque des revendications 1 à 7 avec ou sans les conditions (i) et (ii), ou un complexe d'ion métallique selon la revendication 8, ou une composition pharmaceutique selon la revendication 9, à l'exclusion du composé bis(2-pyridyl)cétone thiosemicarbazone , à utiliser dans une thérapie pour inhiber la prolifération cellulaire ou pour traiter un trouble proliférant.

12. Composé, complexe ou composition à utiliser selon la revendication 11, dans lequel le trouble proliférant est sélectionné parmi des maladies angiogéno-dépendantes, des maladies à prolifération cellulaire, des troubles inflammatoires, des maladies auto-immunes, des maladies des vaisseaux sanguins, une thrombose, un cancer, une tumeur maligne, une tumeur bénigne, une tumeur solide ou une tumeur non solide.

13. Utilisation in vitro d'un composé selon l'une quelconque des revendications 1 à 7 avec ou sans les conditions (i) et (ii), ou d'un complexe d'ion métallique selon la revendication 8, ou d'une composition pharmaceutique selon la revendication 9 pour induire une apoptose dans une cellule, ou pour inhiber une prolifération cellulaire et induire une apoptose dans une cellule.

14. Composé selon l'une quelconque des revendications 1 à 7 avec ou sans les conditions (i) et (ii), ou complexe d'ion métallique selon la revendication 8, ou composition pharmaceutique selon la revendication 9 à utiliser dans une thérapie pour induire une apoptose chez un mammifère ou pour induire une apoptose et inhiber une prolifération cellulaire chez un mammifère.

15. Complexe d'ion métallique à utiliser selon l'une quelconque des revendications 11 à 14, dans lequel le complexe d'ion métallique est un complexe de fer (II) ou un complexe de fer (III).

16. Composé à utiliser selon l'une quelconque des revendications 11 à 14, dans lequel le composé est sélectionné parmi :
di-2-pyridylcétone 4-méthyl-3-thiosemicarbazone ;
di-2-pyridylcétone 4,4-diméthyl-3-thiosemicarbazone ;
di-2-pyridylcétone 4-éthyl-3-thiosemicarbazone ;
di-2-pyridylcétone 4-allyl-3-thiosemicarbazone ;
di-2-pyridylcétone 4-phényl-3-thiosemicarbazone ;
di-2-pyridylcétone isonicotinoyl-hydrazone ;
di-2-pyridylcétone benzoyl-hydrazone ;
di-2-pyridylcétone 4-hydroxybenzoyl-hydrazone ;
di-2-pyridylcétone 3-bromobenzoyl-hydrazone ;
di-2-pyridylcétone 4-aminobenzoyl-hydrazone ;
di-2-pyridylcétone 2-thiophénécarboxaldéhyde hydrazone ;
di-2-pyridylcétone 4,4-diphénylcarboxaldéhyde semicarbazone ; et
di-2-pyridylcétone hydrazone octanoïque.

17. Composé à utiliser selon l'une quelconque des revendications 11 à 14, dans lequel le composé est le di-2-pyridylcétone 4,4-diméthyl-3-thiosemicarbazone .

18. Composé à utiliser selon l'une quelconque des revendications 11 à 14, dans lequel le composé est un complexe d'ion métallique sélectionné parmi Fe[PKIH]₂, Fe[PKBH]₂, Fe[PKHH]₂, Fe[PBBH]₂, F[PKAH]₂, Fe[PKTH]₂, Fe[PK44pH]₂ et Fe[PKoctH]₂, Fe[Dp4mT]₂, Fe[Dp44mT]₂, Fe[Dp4eT]₂, Fe[Dp4aT]₂, et Fe[Dp4pT]₂.

19. Composé à utiliser selon l'une quelconque des revendications 11 à 14, dans lequel le composé est sélectionné parmi di-2-pyridylcétone isonicotinoyl-thiohydrazone ; di-2-pyridylcétone benzoyl-thiohydrazone ; di-2-pyridylcétone 4-hydroxybenzoyl-thiohydrazone ; di-2-pyridylcétone 3-bromobenzoyl-thiohydrazone; di-2-pyridylcétone 4-aminobenzoyl-thiohydrazone ; di-2-pyridylcétone 2-thiophénécarboxaldéhyde thiohydrazone ; di-2-pyridylcétone 4,4-diphénylcarboxaldéhyde thiosemicarbazone ; et di-2-pyridylcétone thiohydrazone octanoïque.
